# EUROPEAN PATENT SPECIFICATION

(11) **EP 3 897 453 B1**
(45) Date of publication and mention of the grant of the patent: **08.04.2026**
(21) Application number: 19898876.8
(22) Date of filing: 20.12.2019
(51) Int. Cl.: A61F 2/06, A61B 17/00, A61B 17/064, A61B 17/068, A61B 90/00, A61F 2/24

(54) **SYSTEM FOR PROSTHETIC CARDIAC VALVE DEVICES**
SYSTEM FÜR HERZKLAPPENPROTHESE
SYSTÈMES POUR VALVULE CARDIAQUE PROTHÉTIQUE

(30) Priority: 21.12.2018 US 201862784280 P; 08.03.2019 US 201962815791 P; 22.05.2019 US 201962851245 P
(43) Date of publication of application: 27.10.2021
(73) Proprietor: Shifamed Holdings, LLC, Campbell, CA 95008 (US)
(72) Inventor: ARGENTO, Claudio, Campbell, California 95008 (US); BACKUS, Andrew, Campbell, California 95008 (US); YANG, Alice, Campbell, California 95008 (US)
(74) Representative: J A Kemp LLP
(86) International application number: PCT/US2019/068088
(87) International publication number: WO 2020/132590

(56) References cited:
- US-A1- 2002 173 841
- US-A1- 2005 165 344
- US-A1- 2009 093 826
- US-A1- 2010 049 239
- US-A1- 2012 053 680
- US-A1- 2012 203 333
- US-A1- 2015 250 480
- US-A1- 2015 374 493
- US-A1- 2016 199 177
- US-A1- 2017 216 025
- US-A1- 2017 311 937
- US-A1- 2018 055 628
- US-A1- 2018 206 993
- US-A1- 2018 344 303
- US-B2- 8 657 872

## Description

### BACKGROUND

Blood flow between and out of heart chambers is regulated by native valves - the mitral valve, the aortic valve, the pulmonary valve, and the tricuspid valve. Each of these valves are passive one-way valves which open and close in response to differential pressures. Patients with valvular disease have abnormal anatomy and/or function of at least one valve. For example, a valve may suffer from insufficiency, also referred to as regurgitation, when the valve does not fully close and allows blood to flow retrograde. Valve stenosis can cause a valve to fail to open properly. Other diseases may also lead to dysfunction of the valves. While medications may be used to treat the disease, in many cases the defective valve may need to be repaired or replaced at some point during the patient's lifetime. Existing valves and surgical repair and/or replacement procedures may have increased risks, limited lifespans, and/or are highly invasive. Some less-invasive transcatheter options are available, however these generally are limited to aortic valve procedures, are limited in their patient-to-patient flexibility, and often take longer than desired to implant.

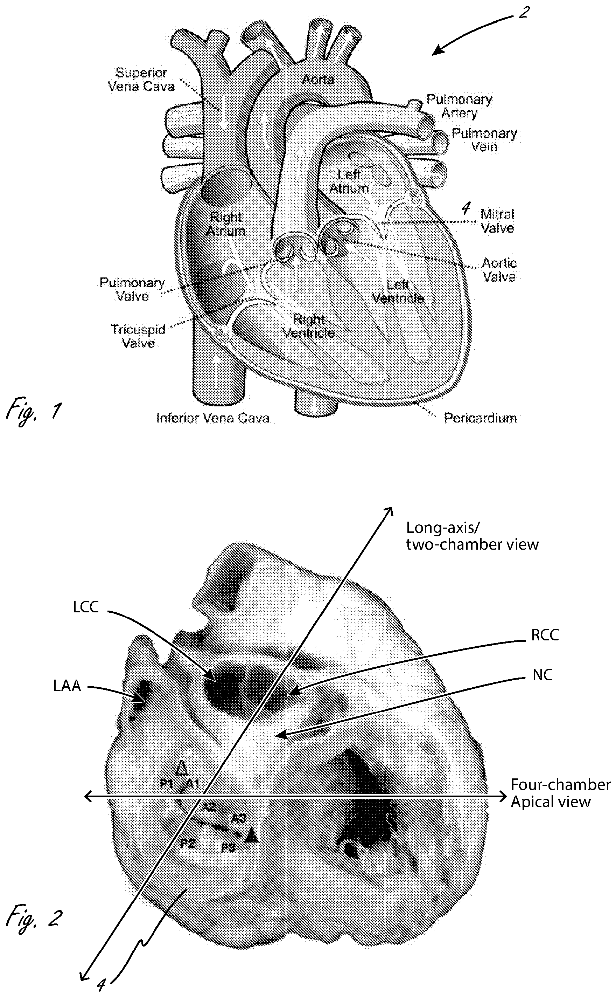

Referring to FIGS. 1 and 2, the heart **2** includes four chambers connected by four valves. The upper part of the heart **2** includes the left atrium **25** and right atrium **5.** The lower part includes the left ventricle **26** and right ventricle **6.** The heart **2** and cardiovascular system operates like a closed circuit. The right side of the heart **2** receives de-oxygenated blood from the body and delivers the blood through the pulmonary artery **7** to the lungs where it becomes re-oxygenated. The oxygenated blood is returned to the left side of the heart **2,** referred to as the systemic side, which delivers the oxygenated blood throughout the body.

Blood flow between the heart chambers is regulated by the valves. On the left side of the heart, the mitral valve **4** is located between the left atrium **25** and the left ventricle **26** and the aortic valve **9** is located between the left ventricle **26** and the aorta **1.** On the right side of the heart **2,** the pulmonary valve **3** is located between the right ventricle **6** and the pulmonary artery **7** and the tricuspid valve **8** is located between the right ventricle **6** and the right atrium **5.**

All four of heart valves are passive one-way valves with "leaflets" which open and close in response to differential pressures. For example, in a healthy heart during systole the left ventricle **26** contracts and pushes blood out the aortic valve **9.** In turn, the pressure in the left ventricle **26** causes the mitral valve **4** to close thereby preventing blood from going back into the left atrium **25** during systole.

A significant population will acquire valve disease in their lifetime. Congenital heart disease is also a significant problem. Patients with valvular disease have abnormal anatomy and/or function of at least one valve. Congenital valve abnormalities may be tolerated and/or treated palliatively for some years before developing into a life-threatening problem in later years. However, congenital heart disease may present life-threatening risk without notice. Patients may acquire valvular disease from rheumatic fever, heart failure, degenerative leaflet tissue, bacterial infection, and more.

Valvular disease may be caused by several factors as shown in FIGS. 3 to 5. FIG. 3 shows a healthy mitral valve **4.** Referring to FIGS. 4 to 5 show a diseased mitral valve **4.** The valve **4** in FIG. 4 suffers from insufficiency, also referred to as regurgitation. Such a valve **4** does not fully close and allows blood to flow retrograde. In this case, blood will flow back into the left atrium **25** during systole. FIG. 5 shows a mitral valve **4** with stenosis. Such a valve **4** does not open properly. Some valves **4** can have concomitant insufficiency and stenosis. Other diseases may also be present, such as Barlow's disease, which prevent the valve **4** from functioning properly. These diseases reduce cardiac output and force the heart **2** to work harder, thus increasing the risk of heart failure and chordae failures.

While medications may be used to treat the disease, in many cases the defective valve may need to be repaired or replaced at some point during the patient's lifetime. The native valve can be replaced with a mechanical valve or tissue valve. Mechanical valves have a disc or other member which opens and closes. Although mechanical valves are formed of biocompatible materials, they carry an increased risk of clotting. Thus, patients usually need to take anticoagulants for the remainder of their lives, which presents additional complications. Tissue valves can be formed of human or animal tissue, as well as polymeric materials. Tissue valves, unlike mechanical valves, do not typically require long-term use of anti-coagulants, but because they are formed of a living tissue they are not as widely available nor do they last as long as mechanical valves. Common tissue valves include porcine aortic valves mounted within a stent-like structure.

More recently there has been increased interest in less invasive procedures for implantation of prosthetic valves. One type of percutaneous procedure involves using a catheter to place a prosthetic valve inside of a diseased or injured heart valve.

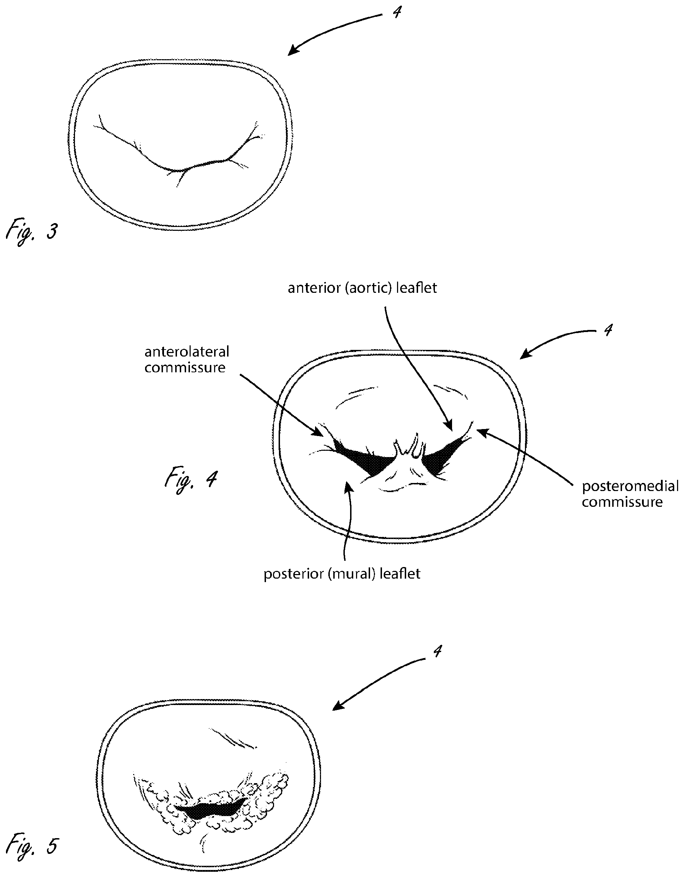

Existing percutaneous procedures for valve repair still face many challenges. These challenges have limited the adoption of transcatheter procedures to certain patient populations and anatomies. Thus far, transcatheter devices are largely focused on aortic valve procedures and the sickest patient populations who may not be able to tolerate surgery. There is a continuing need for improved transcatheter devices which meet or exceed the performance and safety of surgical valves. Percutaneous valve replacement has also been limited to aortic valve procedures. While a large segment of the population suffers from tricuspid and mitral valve disease, the anatomy and function of these valves present challenges to transcatheter replacement. The aortic valve can be accessed via the femoral artery whereas the mitral valve, for example, typically requires a transseptal approach. The mitral valve anatomy presents more complexities to transcatheter procedures than the aortic valve. For example, as shown in FIG. 4, the mitral valve **4** includes two asymmetrical leaflets **4a, 4b** and an irregularly-shaped annulus **4c.** The mitral valve **4** also varies far more considerably patient-to-patient than the aortic valve. For these and other reasons, surgical replacement and percutaneous repair thus far are the only widely-available commercial treatments for mitral valve disease.

There is a continuing need to provide improved less invasive procedures for repair and replacement of heart valves. There is a continuing need to provide less invasive procedures for replacement of diseased valves, including the mitral valve.

There is a continuing need to be able to be able to provide a variety of different valve assemblies to accommodate the requirements of different patients, such as by providing prosthetic valves that can accommodate a variety of individual patients.

Furthermore, existing valve repair/replacement procedures are often complicated and time-consuming. Currently available procedures often require the placement of more than one component - for example, a prosthetic valve and a mechanism to anchor it to the native anatomy. Such procedures generally utilize multiple delivery catheters to carry the various components and delivery of each component separately to the valve, which can be time-consuming (particularly if components are delivered sequential), complicated, and/or dangerous. For example, some devices provide rotational anchoring elements to capture the native anatomy such as the chordae tendineae in order to reduce delivery time. However, such anchoring elements, often by design, capture and pull the chordae along during their rotation, which can torque or otherwise stress and damage the chordae during deployment of the anchor elements, resulting in the need for additional medical interventions for the patient. It would therefore be desirable to provide quicker, less-complicated, and less dangerous valve assemblies and procedures for valvular replacement and repair.

US 2016/199177 A1 discloses systems and methods for docking a heart valve prosthesis. The system includes a helical anchor formed as multiple coils adapted to support a heart valve prosthesis with coil portions positioned above and below the heart valve annulus.

US 8 657 872 B2 discloses methods and apparatus for repairing or replacing a defective cardiac valve including an anchor having a double helix configured to engage the cardiac valve leaflets of a diseased or defective cardiac valve.

US 2017/311937 A1 discloses a fistula treatment system comprising a guide coil which is adapted to extend partially around a tissue tract and an implant element. The implant element is activated to draw tissue surrounding the tract inwardly.

US 2018/206993 A1 discloses devices and methods for the repair of heart valves. A device may comprise a first section having a generally spiral shape and a second section connected to the first section. A method involves positioning the device such that chords associated with the heart valve are positioned within the path of the generally spiral shape of the first section and positioning the second section on an opposite side of the heart valve.

### SUMMARY

It would be desirable to provide a less invasive procedure for repair and replacement of heart valves, including the mitral valve, quicker surgical methods, and/or prosthetic valves that can accommodate a variety of individual patients. Not necessarily all such aspects or advantages are achieved by any particular embodiment. Thus, various embodiments may be carried out in a manner that achieves or optimizes one advantage or group of advantages taught herein without necessarily achieving other aspects or advantages as may also be taught or suggested herein.

The invention is defined in claim 1. Further aspects and preferred embodiments of the invention are defined in the dependent claims. Any aspects, embodiments and examples of the present disclosure which do not fall under the scope of the appended claims do not form part of the invention but represent background that may be useful for understanding the invention.

### BRIEF DESCRIPTION OF THE DRAWINGS

The novel features of the present disclosure are set forth with particularity in the appended claims. A better understanding of the features and advantages of the present disclosure will be obtained by reference to the following detailed description that sets forth illustrative embodiments, in which the principles of the present disclosure are utilized, and the accompanying drawings of which:
FIG. 1 is a schematic of a human heart illustrating the path of blood flow through the heart, in accordance with embodiments.
FIG. 2 is a cross-sectional view of a heart looking down through the mitral valve, aortic valve, and aorta, in accordance with embodiments.
FIG. 3 is a schematic of a healthy mitral valve, in accordance with embodiments.
FIGS. 4 and 5 are schematics of diseased mitral valves, in accordance with embodiments.

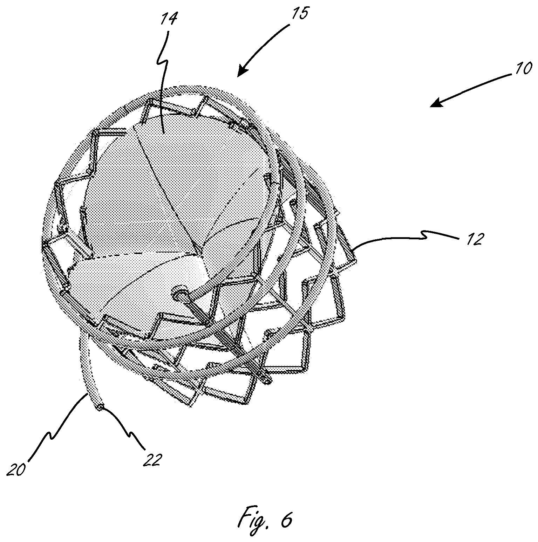

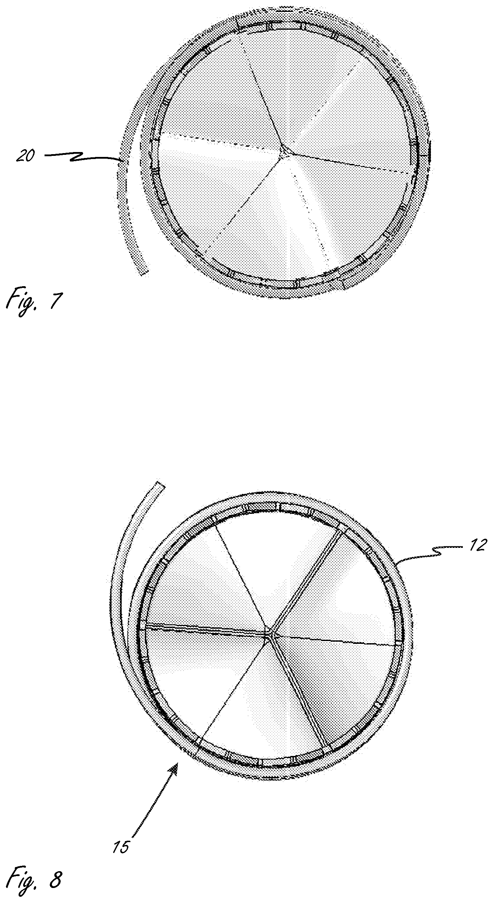

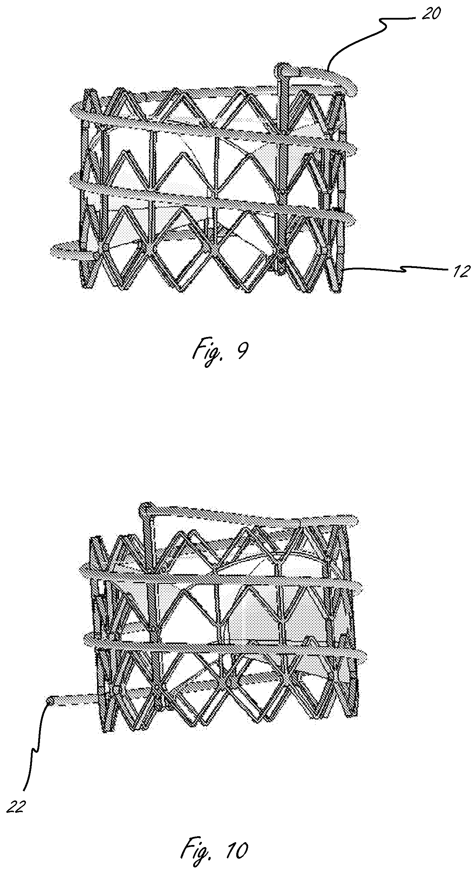

FIGS. 6-10 are several views of a percutaneous valve for replacement of a diseased native valve, in accordance with embodiments.

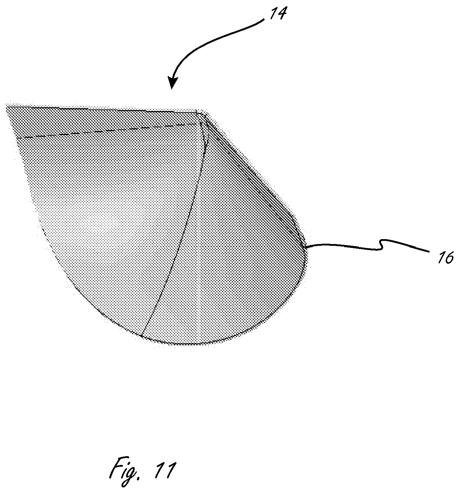

FIG. 11 is a perspective view of the prosthetic valve leaflet of the valve of FIG. 6, in accordance with embodiments.

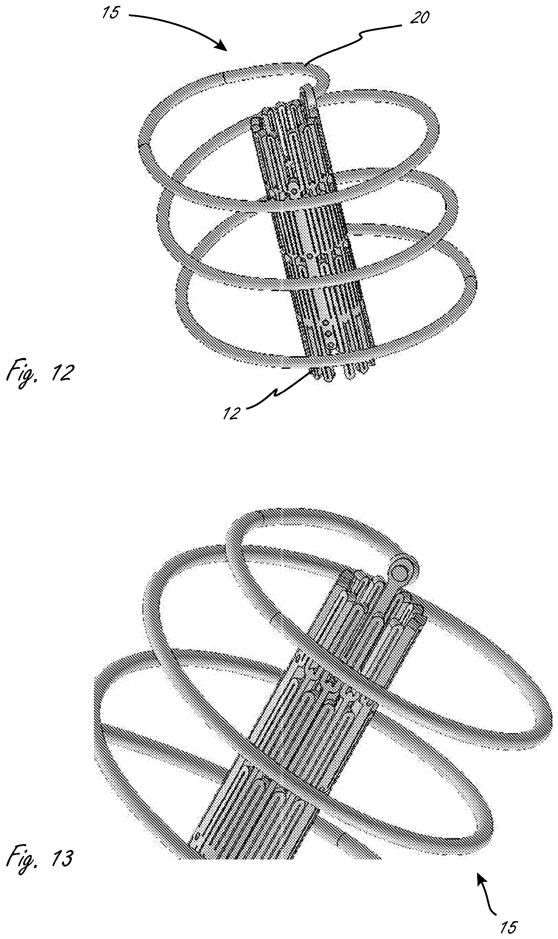

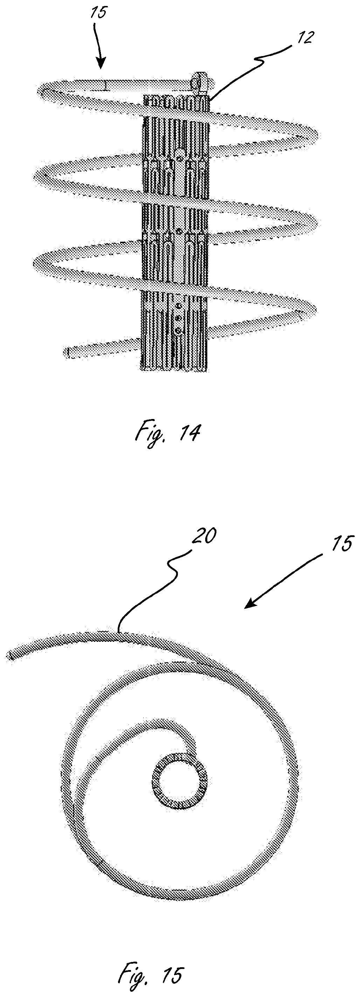

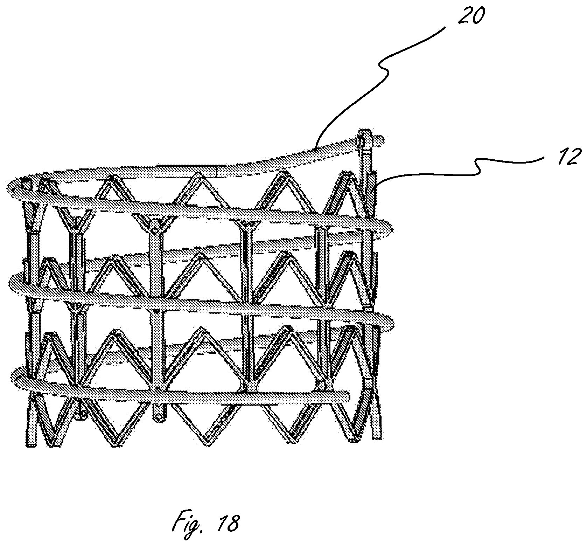

FIGS. 12-18 are several views of the frame structure of the valve of FIG. 6, in accordance with embodiments.

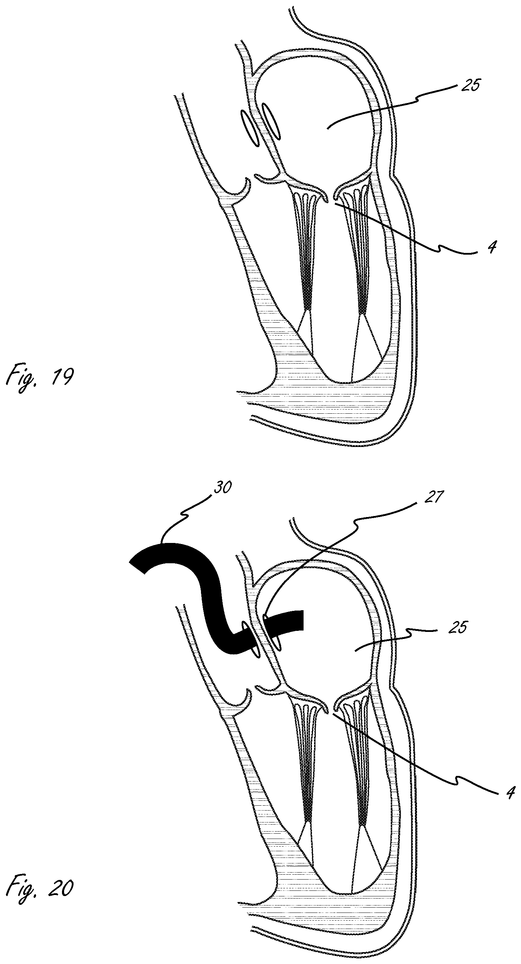

FIGS. 19-26 are several views of the method of implanting the valve of FIG. 6, in accordance with embodiments.

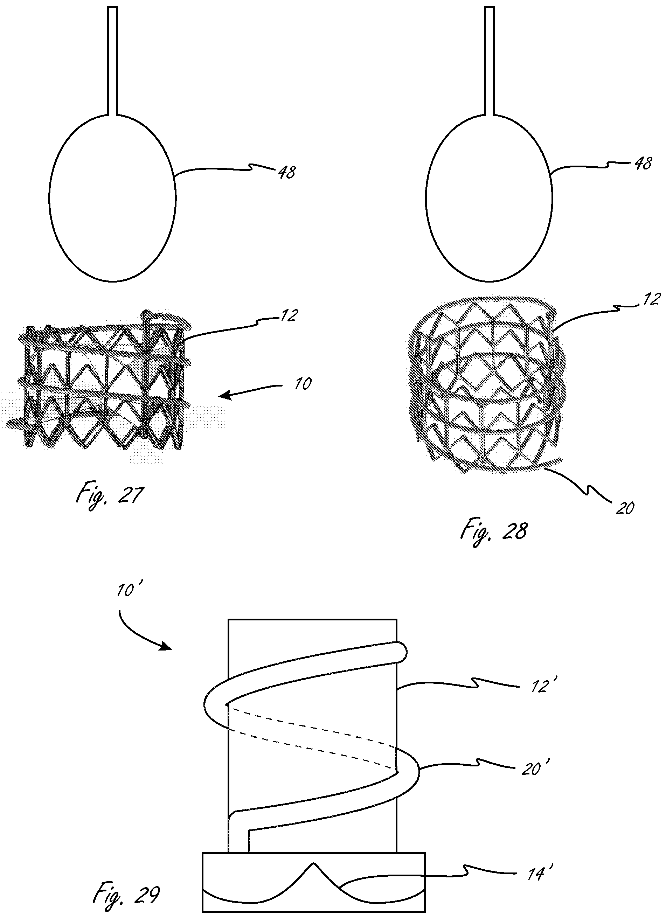

FIGS. 27 and 28 illustrate expanding of the frame structure using a balloon, in accordance with embodiments.
FIG. 29 is a front view of another percutaneous valve similar to the one of FIG. 6, in accordance with embodiments.

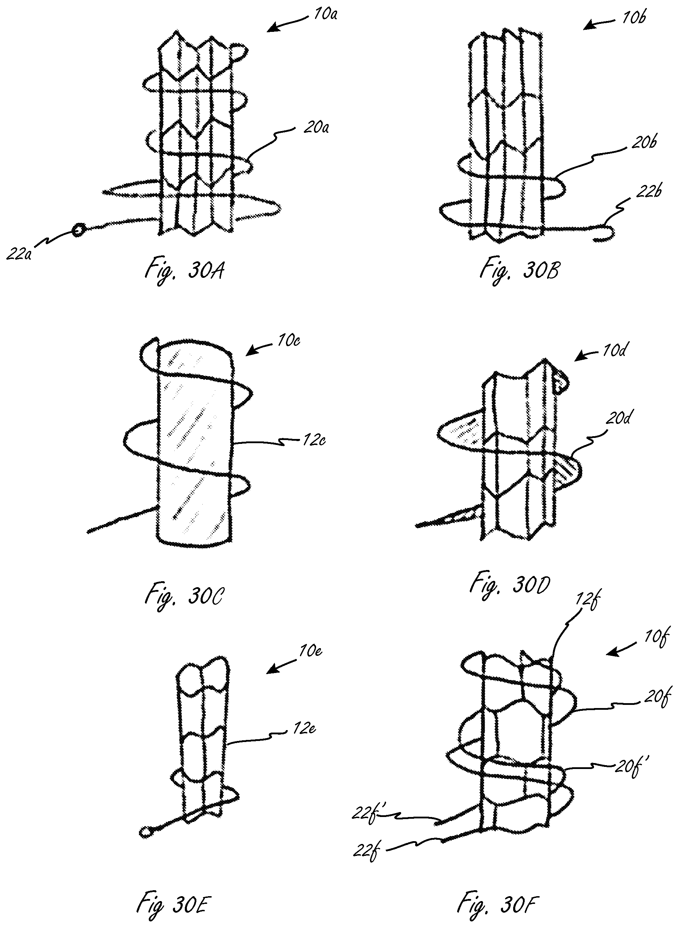

FIGS. 30A-30F are front views of other percutaneous valves similar to the one of FIG. 6, in accordance with embodiments.

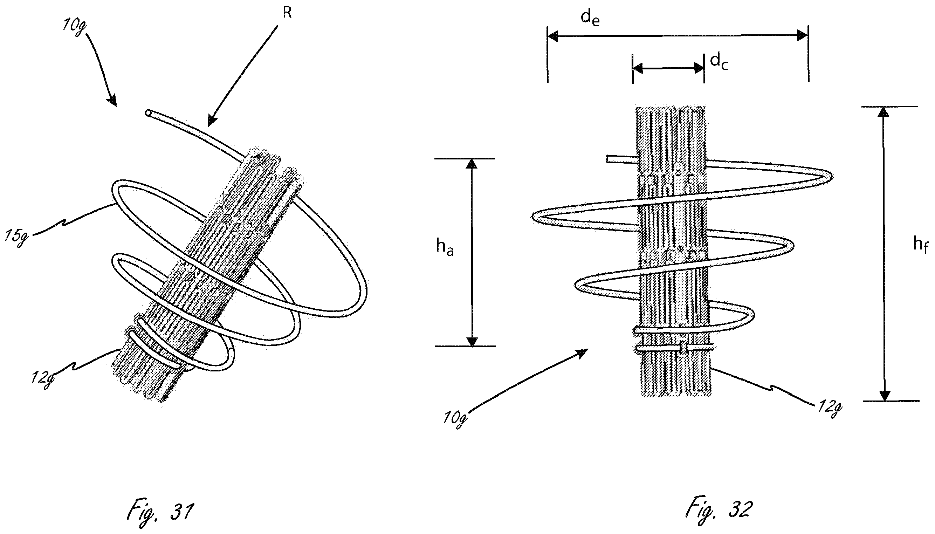

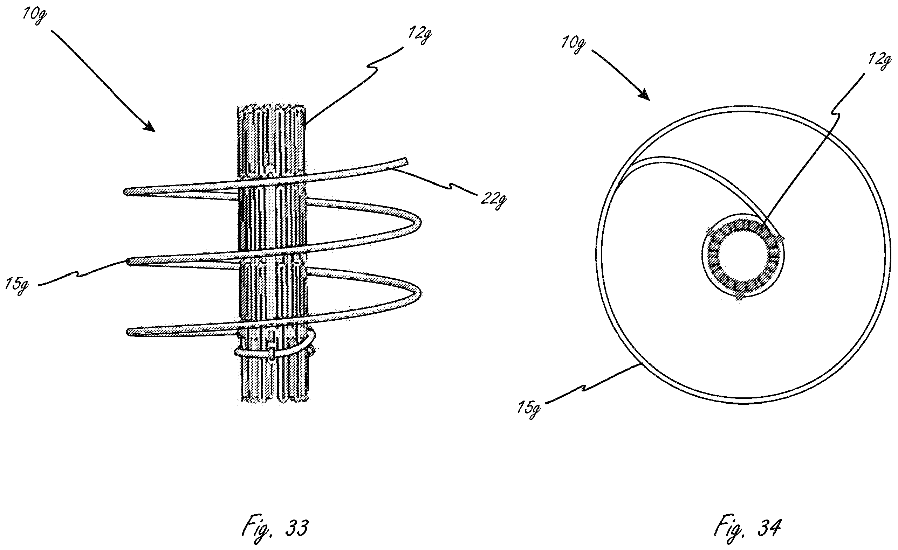

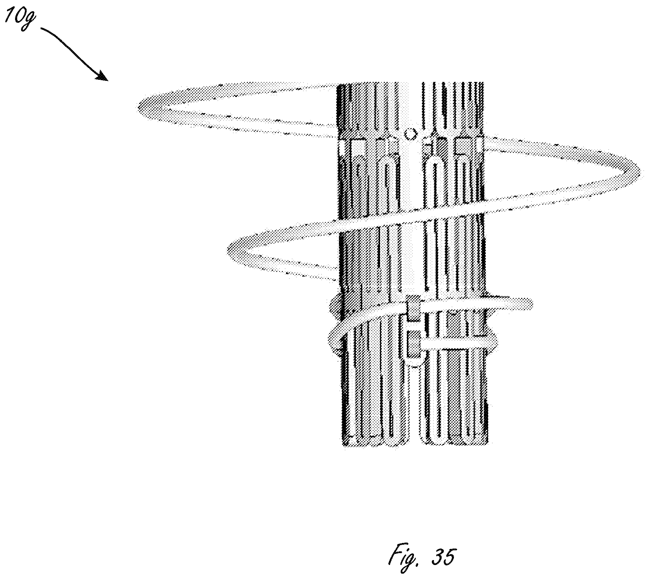

FIGS. 31-35 illustrate another valve device similar to the one of FIG. 6, in accordance with embodiments. FIG. 31 is a perspective view. FIG. 32 is a front view. FIG. 33 is a back view. FIG. 34 is a top view. FIG. 35 is an enlarged view of a bottom portion of the device, showing attachment of the anchor to the frame structure.

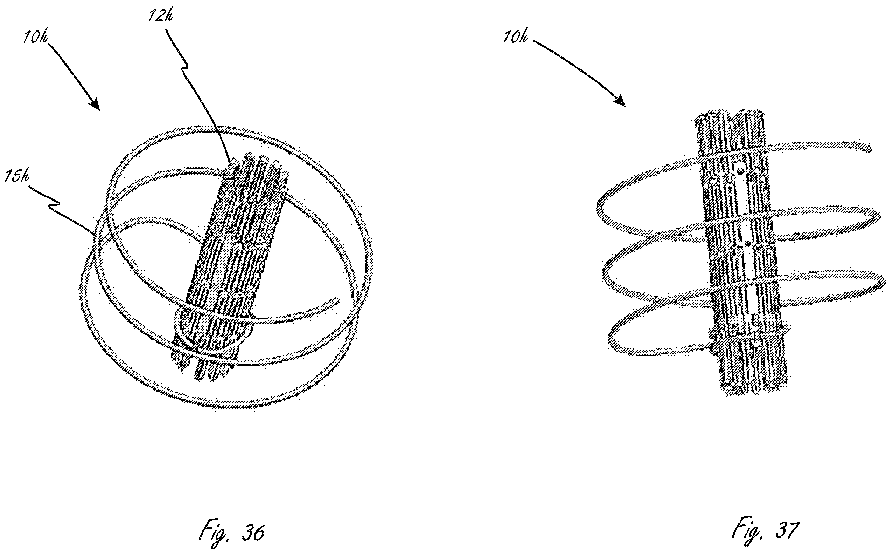

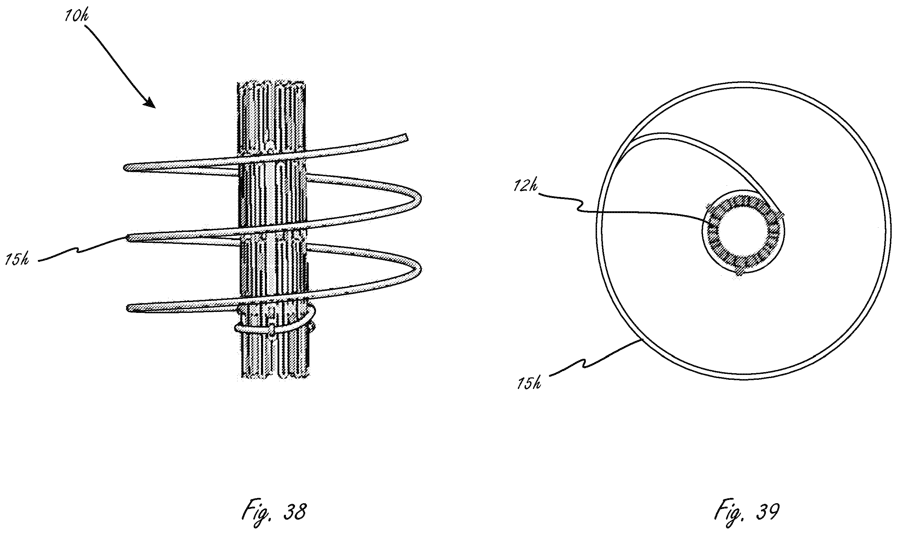

FIGS. 36-39 illustrate another valve device similar to the one of FIG. 6, in accordance with embodiments. FIGS. 36 and 37 are perspective views. FIG. 38 is a front view. FIG. 39 is a top view.

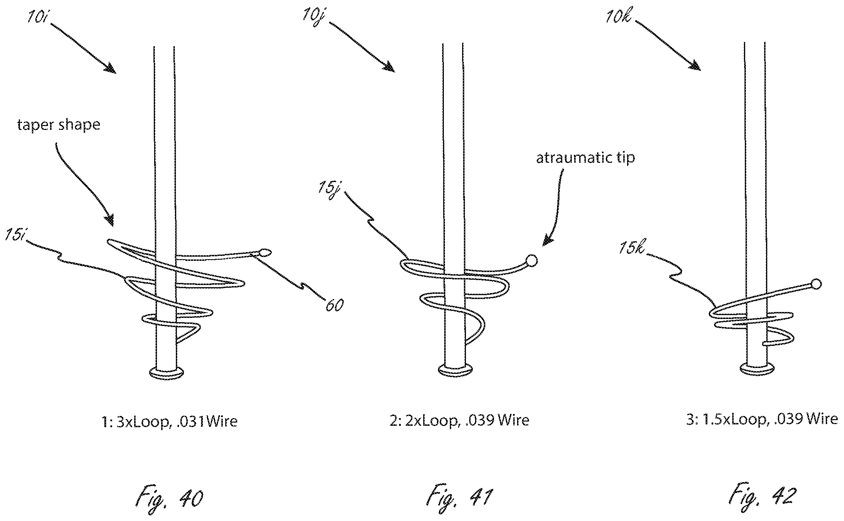

FIGS. 40-42 are front views of valve devices similar to the one of FIG. 6, illustrating different anchors, in accordance with embodiments.

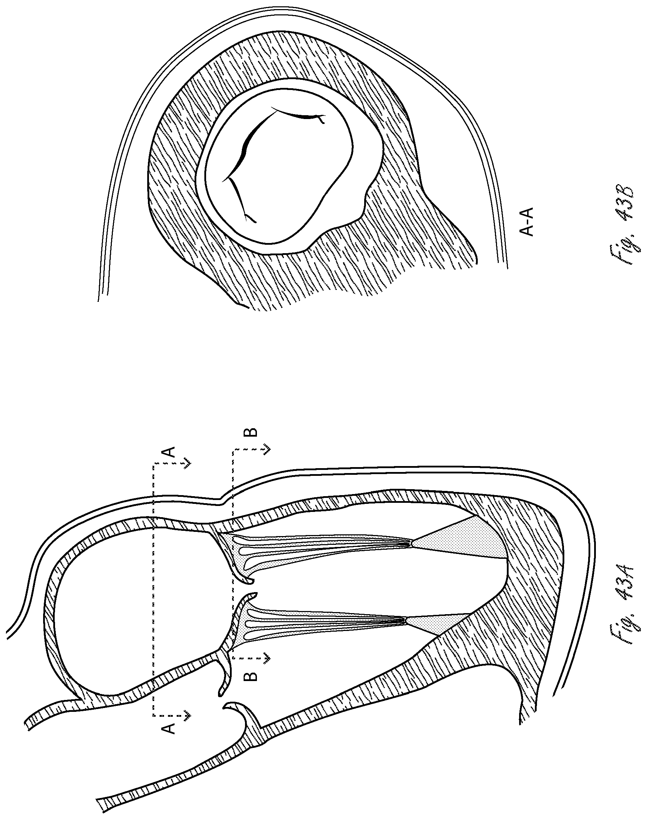

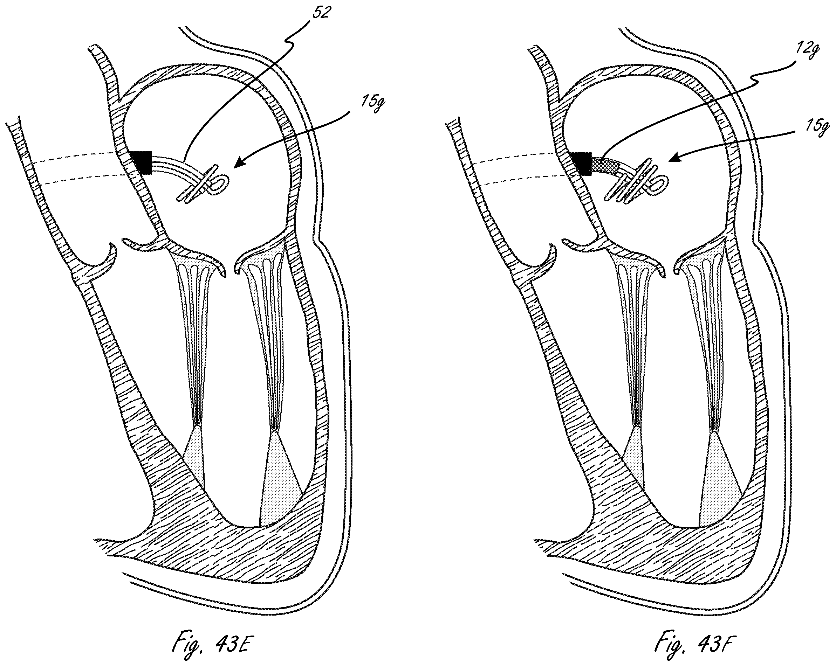

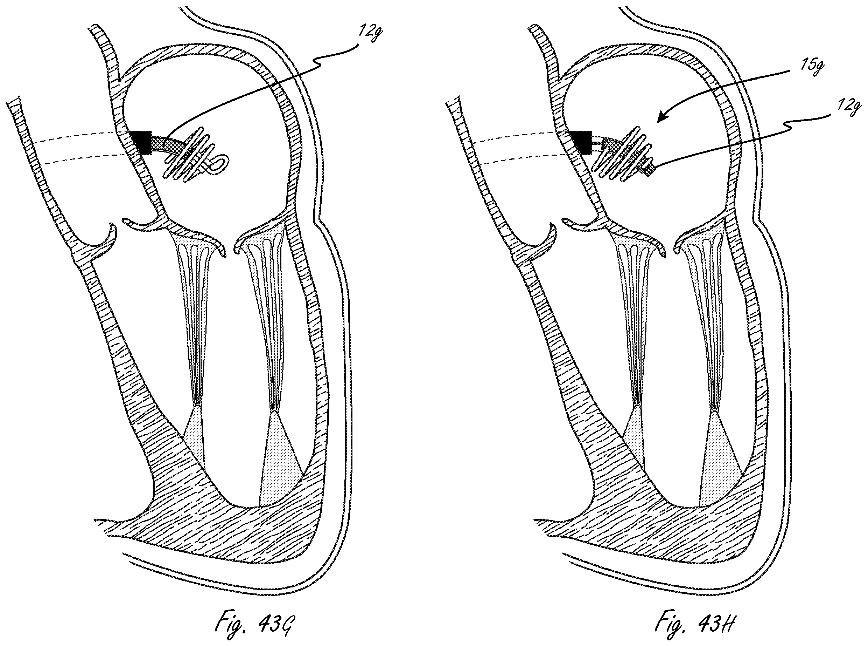

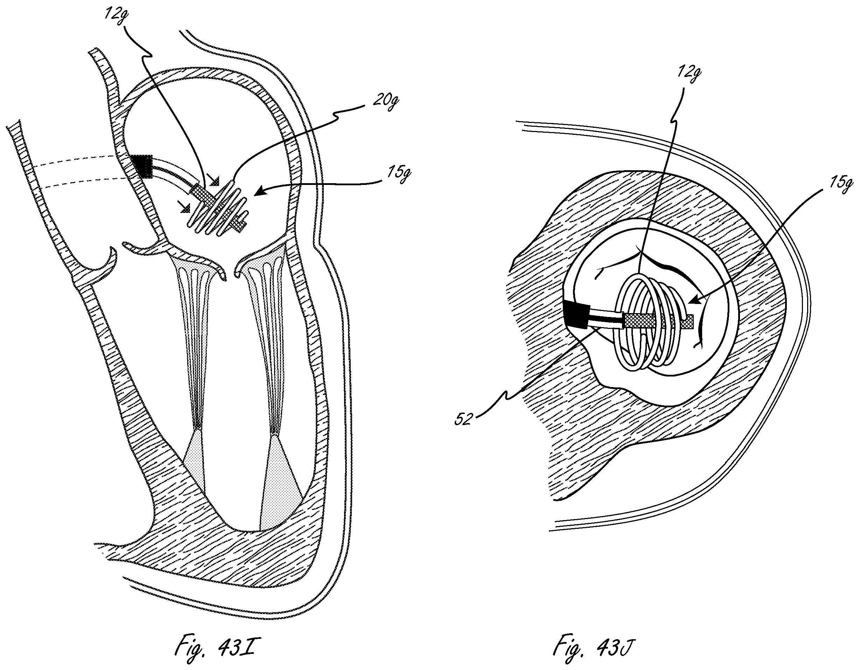

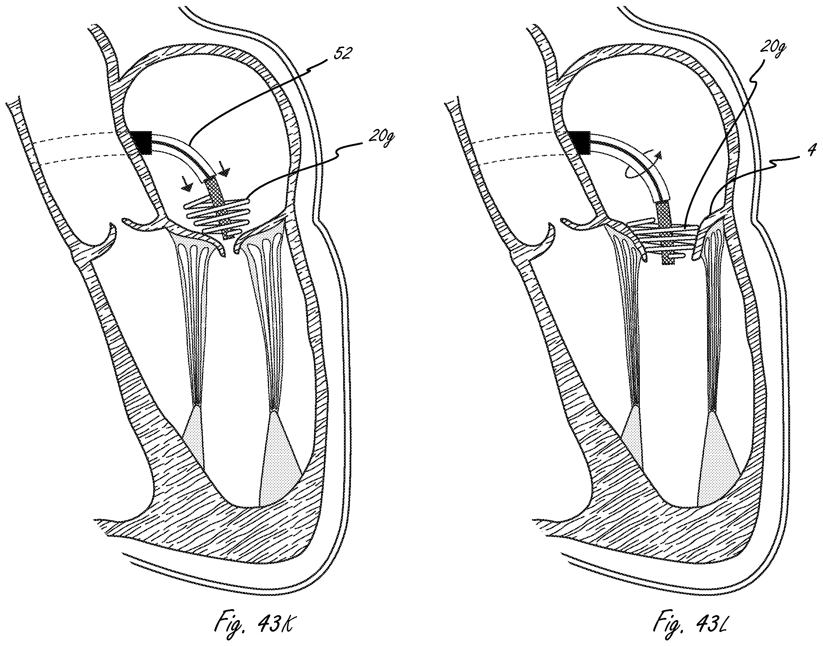

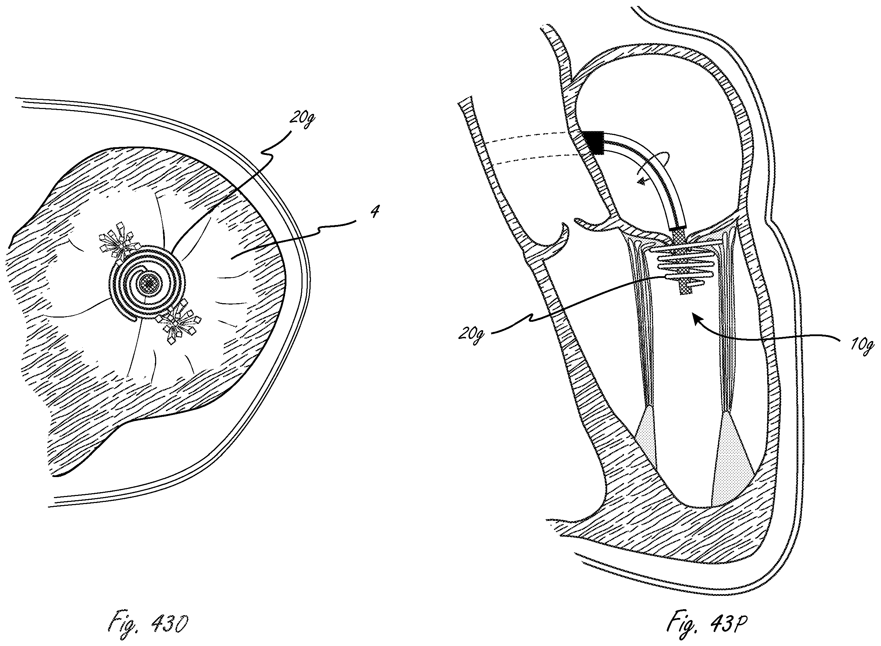

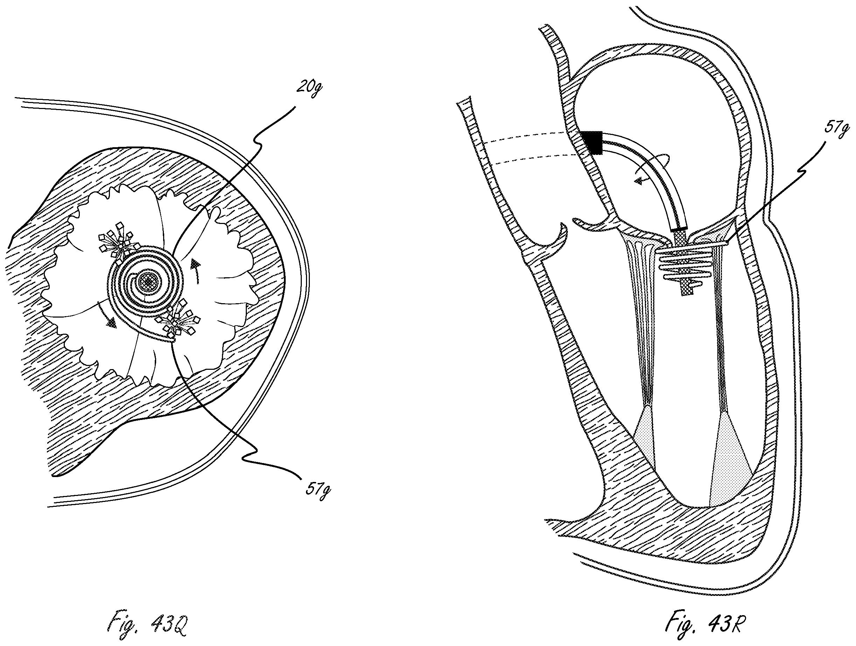

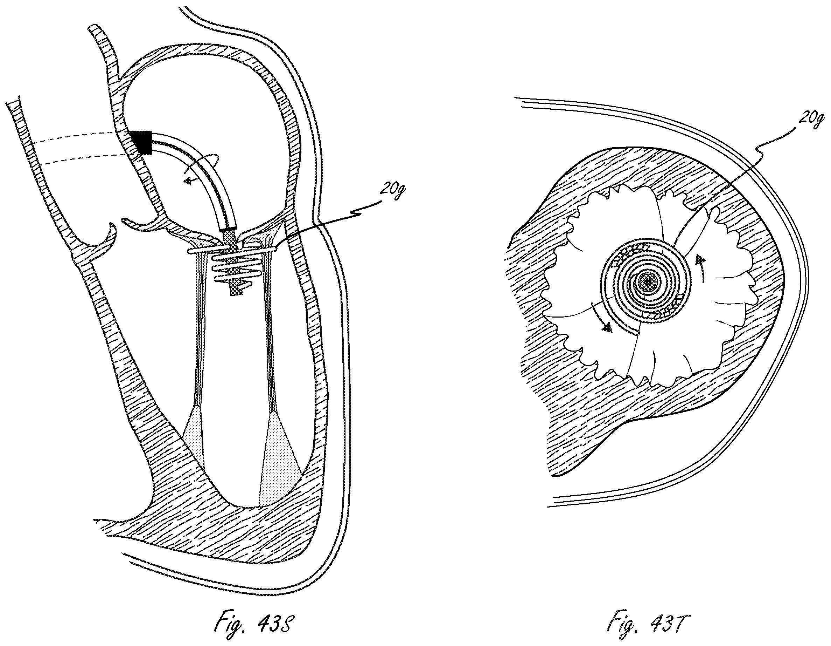

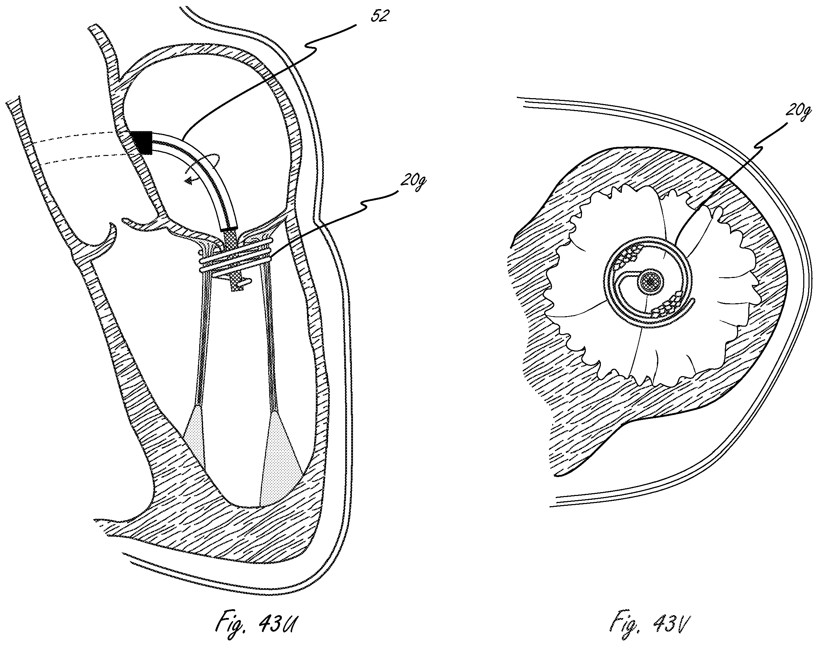

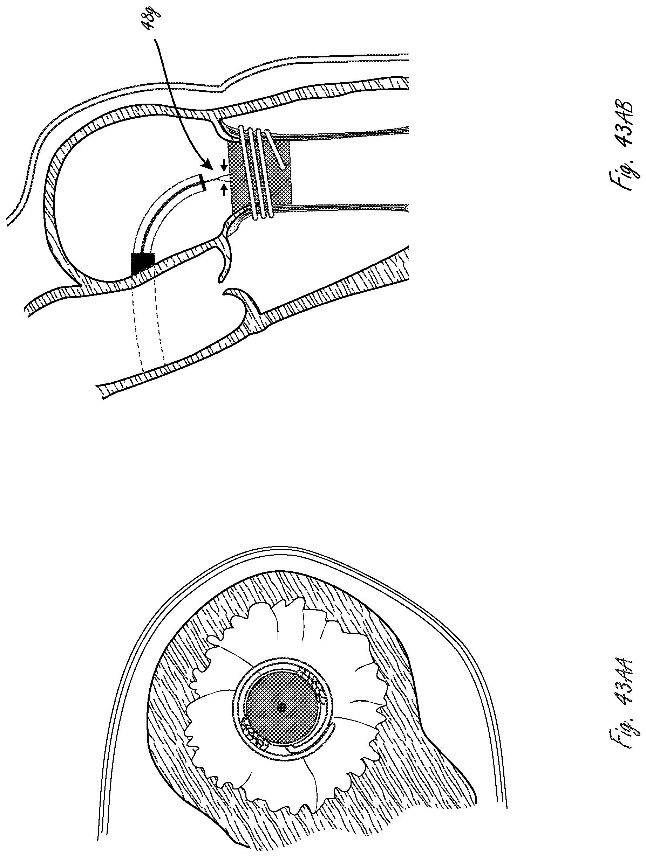

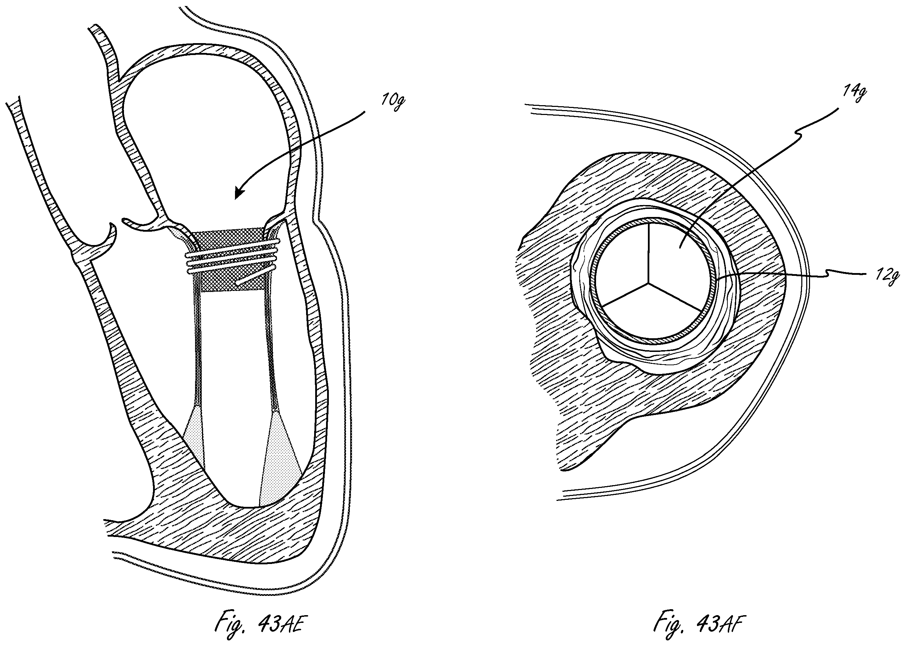

FIGS. 43A-43AF are sequential views of a method of implanting a device similar to the one of FIGS. 6 and 31, in accordance with embodiments.

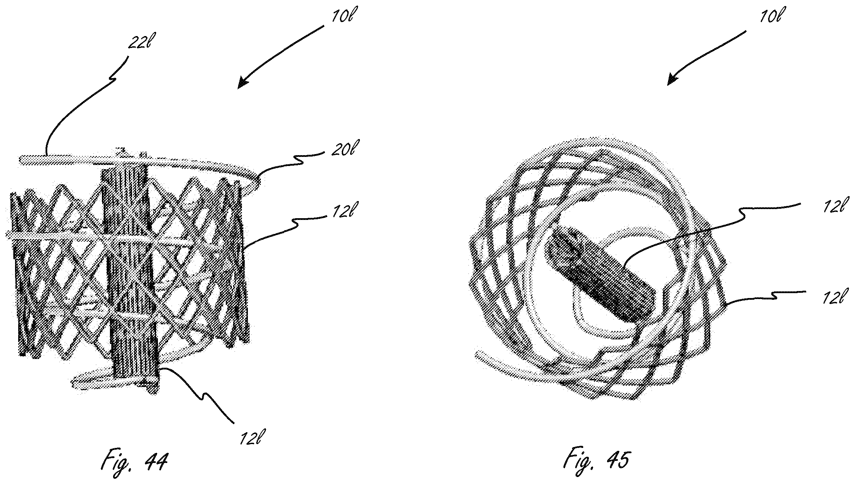

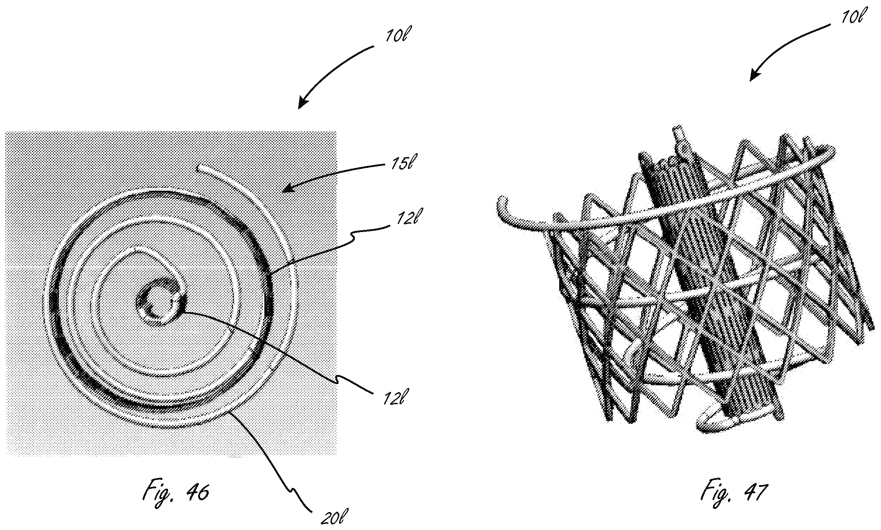

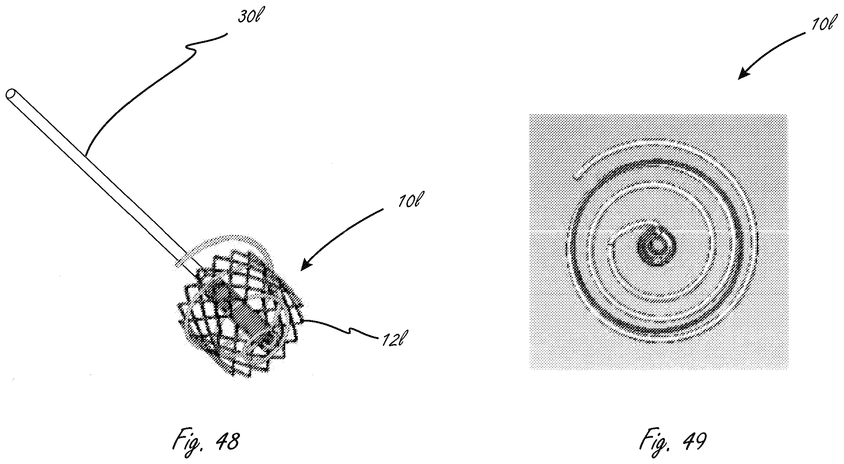

FIGS. 44-49 show a valve device similar to the one of FIG. 6, except that the frame structure is self-expanding, in accordance with embodiments. FIG. 44 is a front view of the device. FIG. 45 is a perspective top view. FIG. 46 is a bottom view. FIG. 47 is a perspective view. FIG. 48 is a perspective view of the valve device loaded on a delivery catheter. FIG. 49 is a bottom view of the device loaded on a delivery catheter.

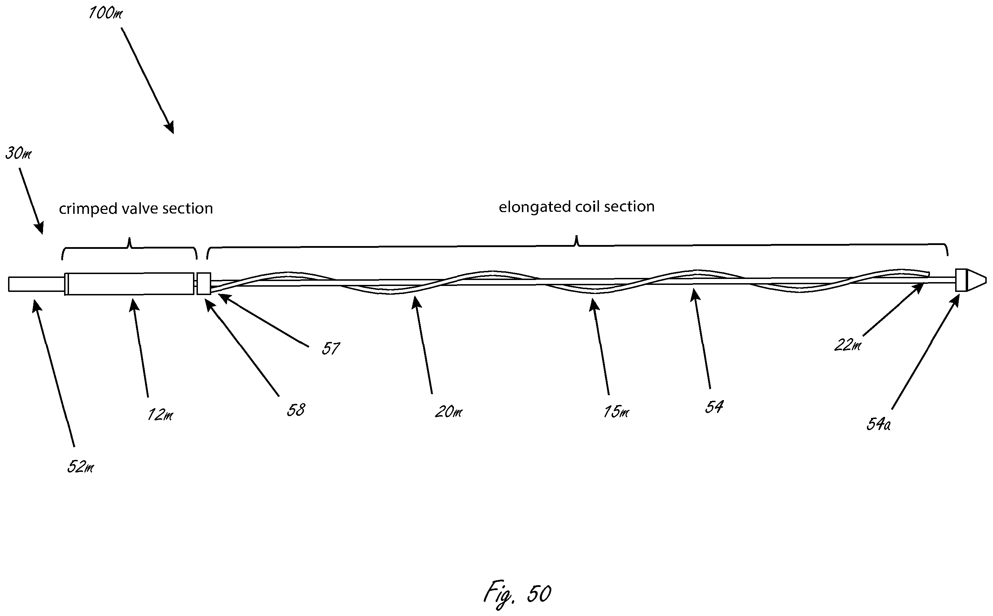

FIG. 50 shows a side view of a valve prosthesis system comprising a frame structure and an anchor loaded on a delivery device with the anchor in a delivery (e.g., elongated) configuration, in accordance with embodiments.

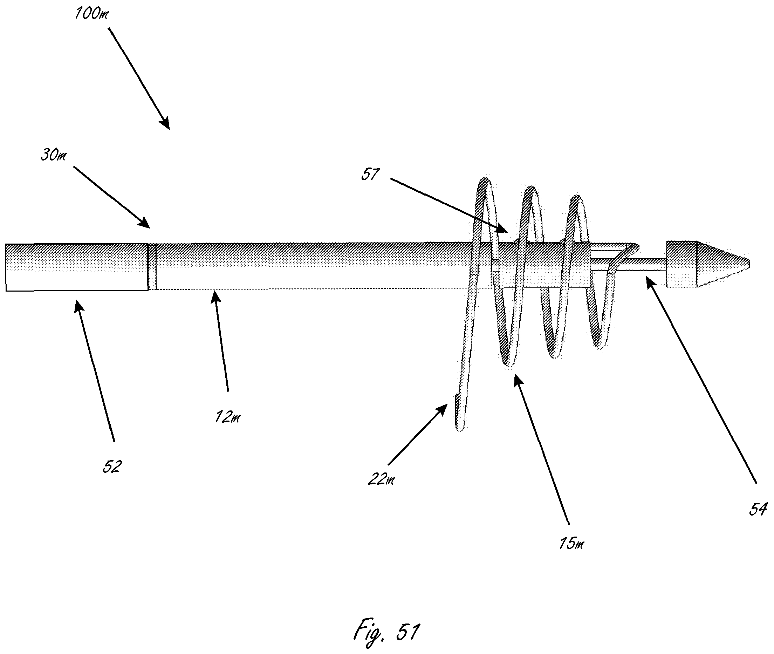

FIG. 51 shows a side view of the valve prosthesis system of FIG. 50 with the anchor in a deployed configuration, in accordance with embodiments.

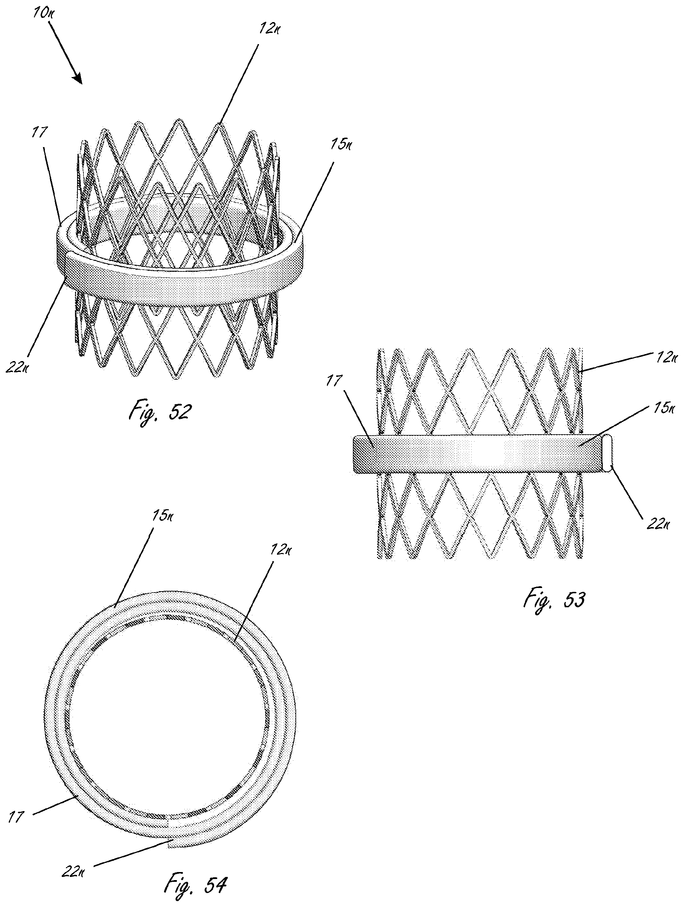

FIGS. 52-54 show several views of a valve prosthesis with an anchor comprising a spiral band, in accordance with embodiments.

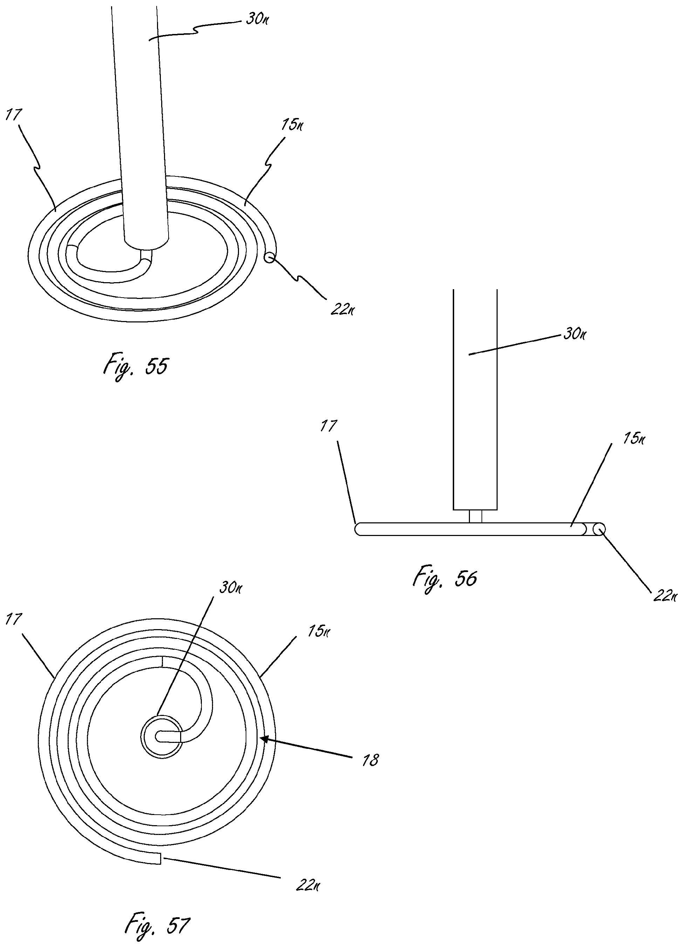

FIGS. 55-57 show several views of the anchor of FIGS. 52-54 loaded on a delivery device, in accordance with embodiments.

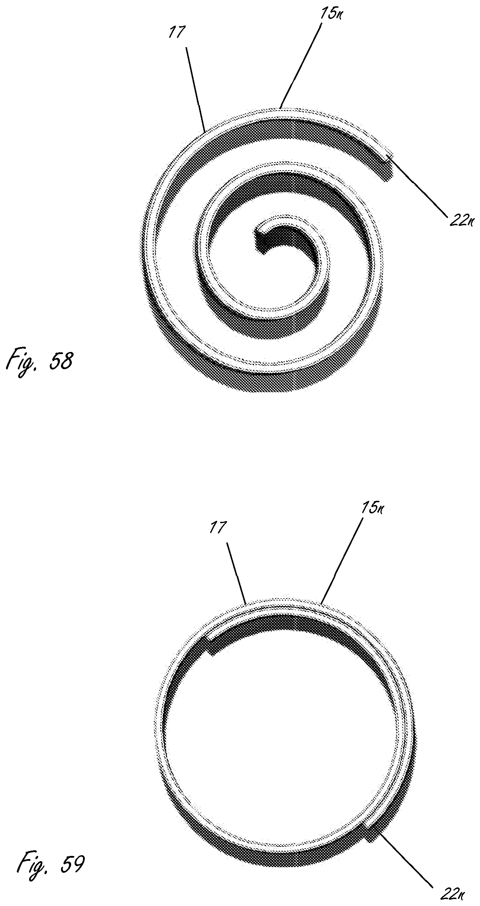

FIGS. 58-59 show deployment of the anchor of FIGS. 52-54 from an unexpanded, undeployed configuration (FIG. 58) to an expanded, deployed configuration (FIG. 59), in accordance with embodiments.

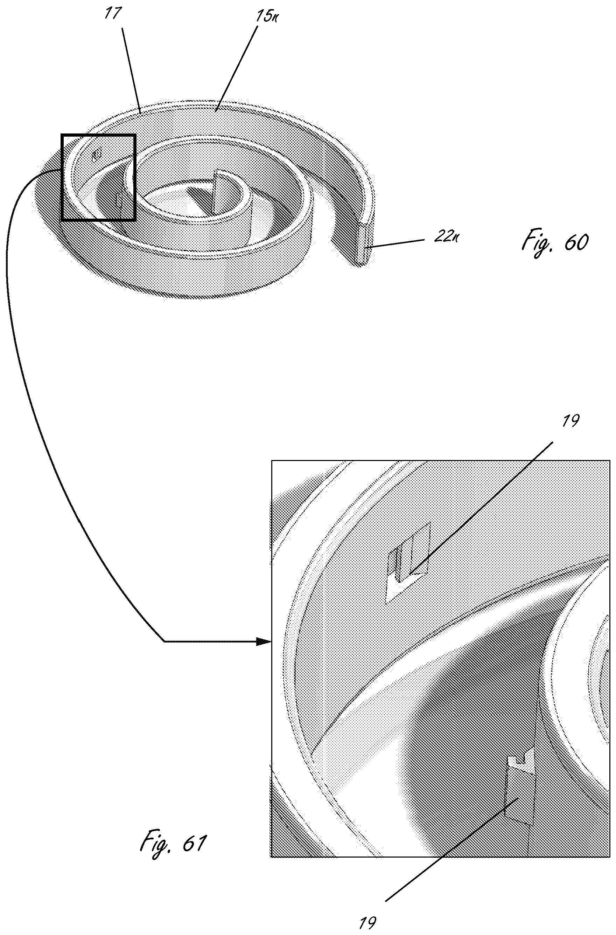

FIGS. 60-61 show an optional locking mechanism for a spiral band anchor, in accordance with embodiments.

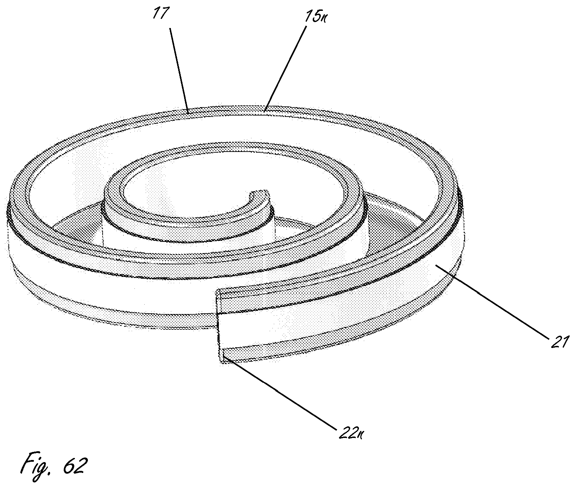

FIG. 62 shows another optional locking mechanism for a spiral band anchor, in accordance with embodiments.

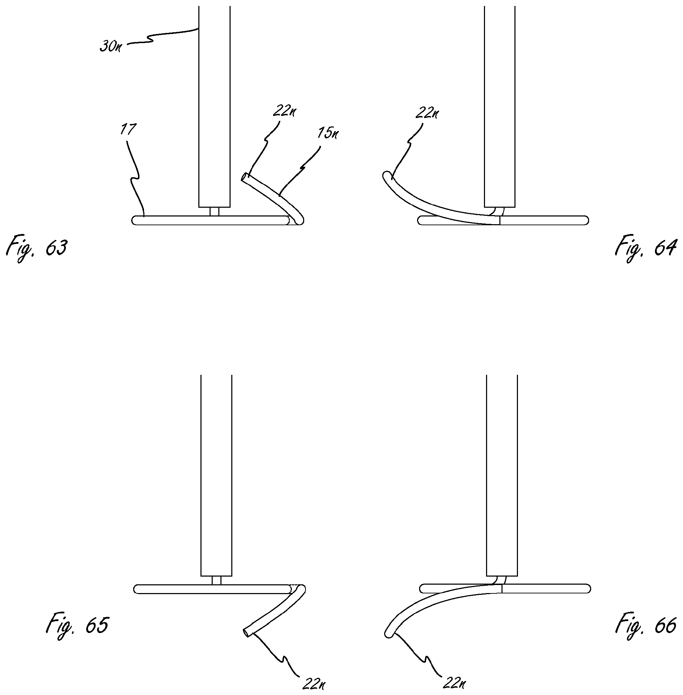

FIGS. 63-64 show an optional tip orientation for a spiral band anchor, in accordance with embodiments.
FIGS. 65-66 show another optional tip orientation for a spiral band anchor, in accordance with embodiments.

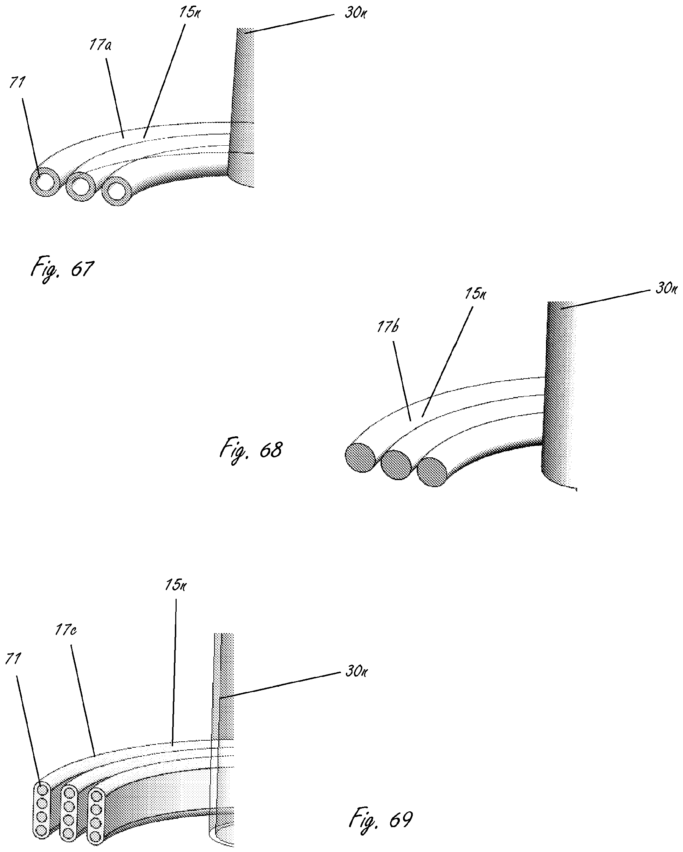

FIGS. 67-69 show various optional configurations for a spiral band anchor, in accordance with embodiments.

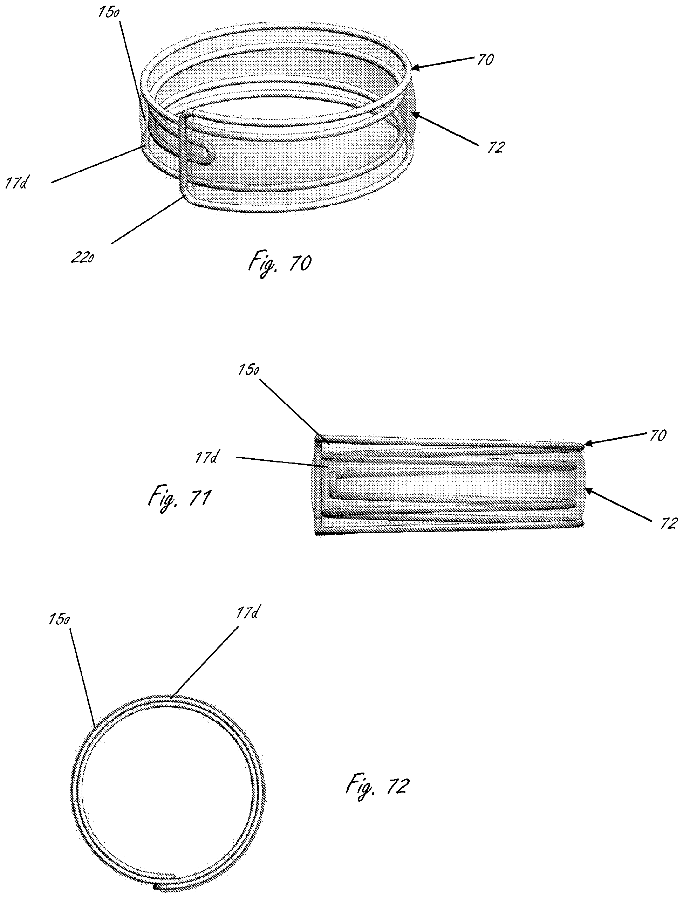

FIGS. 70-72 show several views of an anchor comprising a tapered spiral band, in accordance with embodiments.

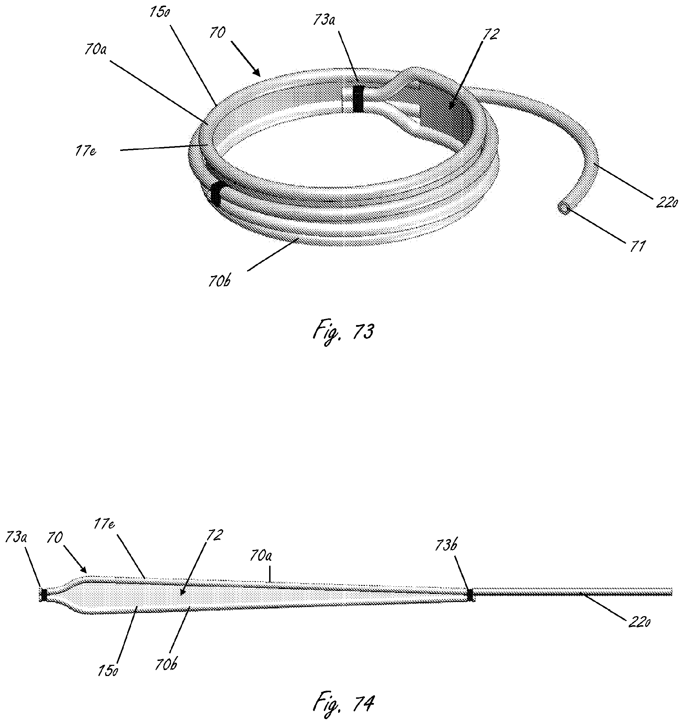

FIGS. 73-76 show several views of another anchor comprising a tapered spiral band, in accordance with embodiments.

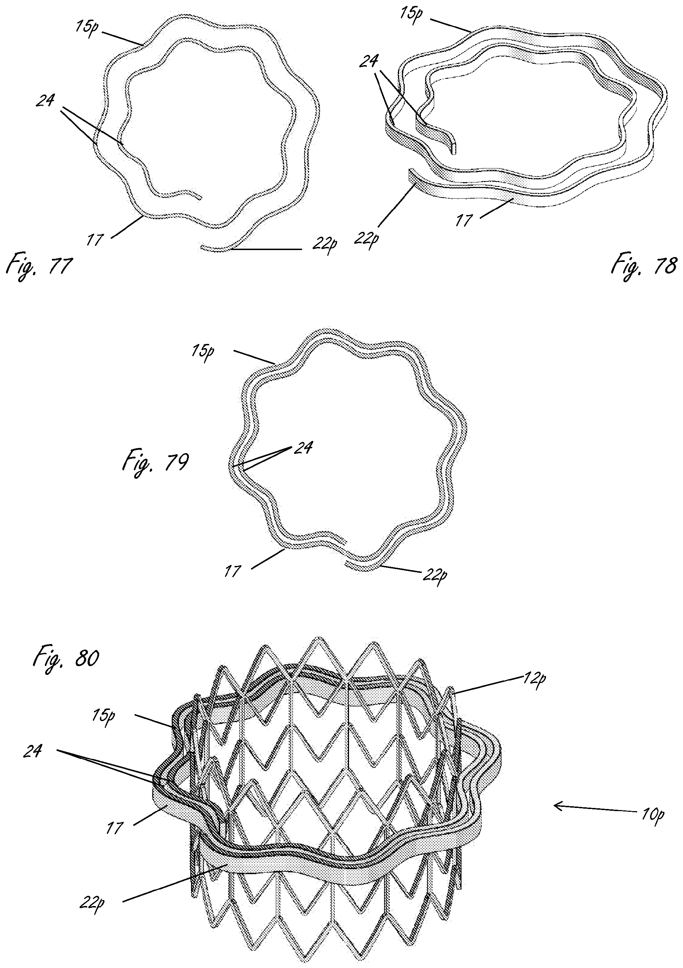

FIGS. 77-80 show several views of an optional locking mechanism for a spiral band anchor, in accordance with embodiments.

The invention relates to a system for treating a diseased native valve in a patient as shown in figs. 52-69.

### DETAILED DESCRIPTION

In the following detailed description, reference is made to the accompanying figures, which form a part hereof. In the figures, similar symbols typically identify similar components, unless context dictates otherwise. The illustrative embodiments described in the detailed description and figures are not meant to be limiting. Other embodiments may be utilized, and other changes may be made, without departing from the scope of the subject matter as defined by the appendend claims. It will be readily understood that the aspects of the present disclosure, as generally described herein, and illustrated in the figures, can be arranged, substituted, combined, separated, and designed in a wide variety of different configurations, all of which are explicitly contemplated herein.

Although certain embodiments and examples are disclosed below, inventive subject matter extends beyond the specifically disclosed embodiments to other alternative embodiments and/or uses, and to modifications thereof. Thus, the scope of the claims appended hereto is not limited by any of the particular embodiments described below. For example, in any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain embodiments, however, the order of description should not be construed to imply that these operations are order dependent. Additionally, the structures, systems, and/or devices described herein may be embodied as integrated components or as separate components.

For purposes of comparing various embodiments, certain aspects and advantages of these embodiments are described. Not necessarily all such aspects or advantages are achieved by any particular embodiment. Thus, for example, various embodiments may be carried out in a manner that achieves or optimizes one advantage or group of advantages as taught herein without necessarily achieving other aspects or advantages as may also be taught or suggested herein.

The present disclosure is described in relation to deployment of systems, devices, or methods for treatment of a diseased native valve of the heart, for example a mitral valve, aortic valve, or tricuspid. However, one of skill in the art will appreciate that this is not intended to be limiting and the devices and methods disclosed herein may be used in other anatomical areas and in other surgical procedures.

For convenience in explanation and accurate definition in the appended claims, the terms "up" or "upper", "down" or "lower", "inside" and "outside" are used to describe features of the present disclosure with reference to the positions of such features as displayed in the figures.

In many respects the modifications of the various figures resemble those of preceding modifications and the same reference numerals followed by subscripts "a", "b", "c", and "d" designate corresponding parts. It will be understood by one of ordinary skill in the art that modifications of corresponding parts of the various figures are interchangeable with one another between embodiments to arrive at multiple combinations with multiple modified parts.

Turning now to the drawings, wherein like components are designated by like reference numerals throughout the various figures, attention is directed to FIGS. 1-5. FIG. 1 shows a human heart **2** and the blood flow pathways through the four chambers of the heart. FIG. 2 is a human heart **2** showing the mitral valve **4,** aortic valve **9,** and aorta **1.** The mitral valve **4** includes two leaflets **4a, 4b.** The anterior (aortic) leaflet **4a** is adjacent the aorta **1.** The posterior (mural) leaflet **4b** is remote from the aorta **1.** The aortic valve **9** includes three leaflets. In the current view, the heart **2** is in systole with the aortic valve **9** open and the mitral valve **4** closed. Whereas FIG. 1 illustrates a healthy heart **2,** FIGS. 2-5 illustrate exemplary mitral valve **4** disease states which may be addressed by the prosthetic valve in accordance with the present disclosure. The prosthetic valve may also be used to treat functional regurgitation such as functional mitral regurgitation (FMR).

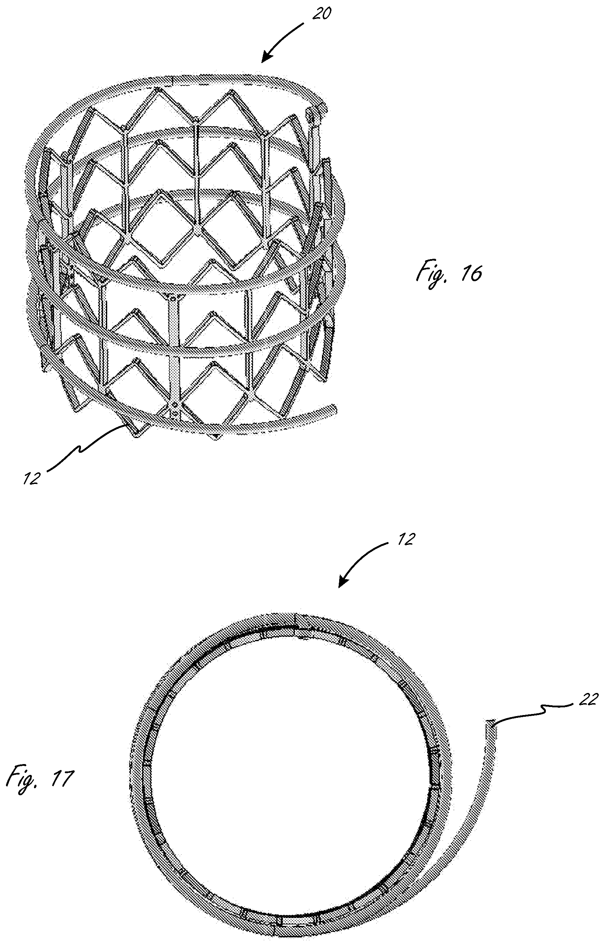

FIGS. 6-18 show an exemplary valve prosthesis **10** (also referred to herein as "valve device") for replacement of a diseased mitral valve in accordance with the present disclosure. The illustrated valve prosthesis **10** comprises a frame structure **12,** a valve segment **14,** and an anchor **15.** FIGS. 6-10 show the valve prosthesis **10** in an expanded, deployed state. FIGS. 12-18 show the frame structure **12** without the valve segment **14.** The frame structure **12** is in a collapsed state in FIGS. 12-15 and an expanded state in FIGS. 16-18. The anchor **15** is shown in a deployed state.

The exemplary valve prosthesis **10** will now be described with reference to FIGS. 6-11. In the illustrated embodiment, valve prosthesis **10** is configured for replacement of a native mitral valve. Valve **10** includes a frame structure **12,** valve segment **14,** and anchor **15.** In the illustrated embodiment, the anchor **15** includes a wire **20** formed in a helical or spiral shape around the frame structure.

The exemplary frame structure **12** is configured like a stent. The frame structure **12** has an expanded state and an unexpanded (e.g., collapsed or compressed) state. The compressed state is sized and dimensioned for percutaneous insertion and the expanded state sized and dimensioned for implantation in a native valve of a patient. In various embodiments, the frame structure **12** comprises an expanded outer periphery and a compressed outer periphery when subject to an external radial force, the compressed outer periphery being slightly smaller in diameter than the expanded outer periphery. The frame structure **12** is shown in the expanded, deployed state in FIG. 6. The frame structure **12** is shown in the collapsed, delivery state in FIG. 12.

The exemplary frame structure **12** is a scaffold in a diamond pattern formed from a shape memory material (e.g. NiTi). One of ordinary skill in the art will appreciate from the description herein that many other structures, materials, and configurations may be employed for the frame structure **12.** For example, the frame structure **12** may be formed of a polymer of sufficient elasticity. The frame structure **12** may be formed of a combination of a metal and polymer, such as a metal (e.g., shape memory material) covered in polymer. The frame structure **12** may include a variety of patterns besides diamond shapes.

Valve prosthesis **10** includes a valve segment **14** within the frame structure **12.** The exemplary valve segment **14** is expandable and collapsible. In the illustrated embodiment, the valve segment **14** is affixed within the frame structure **12** and expands and collapses with the frame structure **12.** Valve segment is used somewhat interchangeably with prosthetic valve leaflet and generally refers to the prosthetic leaflets and frame. As used herein, "prosthetic valve" may refer to all manner of prosthetic and artificial replacement valves including tissue (biological) valves, tissue-engineered valves, polymer valves (e.g. biodegradable polymer valves), and even certain mechanical valves.

In the illustrated embodiment, frame structure **12** is a closed frame such that blood flow is forced through valve segment **14** therein. One or more skirts and/or seals may help force blood through valve segment **14.**

Valve segment **14** can be configured as would be understood by one of skill from the description herein. The valve segment **14** can be similar to existing transcatheter valves. The valve segment **14** can be similar to existing surgical tissue valves, and mechanical valves. In various embodiments, the valve segment **14** includes leaflets **16** formed of multi-layered materials for preferential function. At least one leaflet **16** may have an inner layer and an outer layer. In various embodiments, the leaflet **16** is connected to a valve structure which in turn is connected to the frame structure **12.** The valve structure may be connected to the frame structure **12** before or after the frame structure **12** has been deployed adjacent a native valve. In various embodiments, the leaflet **16** is attached to the frame structure **12** directly. The leaflet **16** may have an inner layer and an outer layer, with the outer layer attached to the frame structure **12.** The leaflet **16** may be attached to an end of the frame structure **12.** Alternatively, or in combination, the leaflet **16** may be attached to an intermediate portion of the frame structure **12.** In various embodiments, the valve segment **14** includes a plurality of leaflets **16,** such as two, three, or more leaflets. In the illustrated embodiment, the valve segment **14** includes three leaflets **16** which are attached to frame structure **12.** An exemplary leaflet **16** is shown in FIG. 11. The leaflet **16** is concave to permit flow in one direction. In particular, flow in one direction causes the leaflet(s) **16** to deflect open and flow in the opposite direction causes the leaflet(s) **16** to close.

Turning back to FIGS. 6-18, and more particularly FIGS. 12-18, an exemplary anchor **15** comprises a helical member, such as wire **20,** having a free end **22.** The other end of the wire **20** is attached to a top end of frame structure **12.** In the illustrated embodiment, one end of the wire **20** is fixed to a strut of the frame structure **12.** This end can be attached by suitable means as would be understood by one of skill in the art from the description herein including, but not limited to, a weld, an adhesive, and a mechanical fastener. In various embodiments, the helical wire **20** is attached to the frame structure only at the location of the second end.

Although referred to as an anchor, one will appreciate that anchor **15** does not require performing an anchor function in the traditional sense. As will be described in more detail below, the anchor guides valve prosthesis **10** into a desired position within a native valve. The anchor **15** may also mitigate against undesired entanglement and disturbances to the chordae tendineae and valve leaflets of the mitral valve.

Wire **20** is formed of a material having sufficient rigidity to hold a predetermined shape. In the exemplary embodiment, the wire **20** is formed of a shape memory material (e.g. NiTi). It may be desirable for at least an end portion to be relatively rigid such that it can exert a force to move chordae tendineae, while still retaining flexibility to be collapsed within a catheter. In various embodiments, the end portion (including free end **22)** only needs sufficient rigidity to hold its shape and will deform under a load. For example, the end portion may be configured with similar rigidity to a guidewire, or slightly stiffer.

In various embodiments, the anchor **15** comprises a helical member. The helical member may comprise a helical wire or flat ribbon. The helical member may comprise a three-dimensional surface as described herein.

In various embodiments, the anchor **15** may comprise a first portion comprising the helical wire **20** and another portion. Alternatively, or in combination, the anchor **15** may comprise a plurality of helical wires **20.** For example, the anchor **15** may comprise at least two helical wires **20** having the same or different diameters. Alternatively, or in combination, the anchor **15** may comprise at least two helical wires **20** having the same or different winding pitches.

In various embodiments, the anchor **15** may comprise a plurality of anchors, for example a plurality of helical wires **20** as described herein.

In various embodiments, the anchor **15** may comprise a flat spiral shape. Loops of the flat spiral shaped anchor may be generally positioned within the same plane (the plane being perpendicular to a longitudinal axis of a delivery device) as described herein.

In the illustrated embodiment, valve prosthesis **10** is configured for replacing a mitral valve and free end **22** is configured for insertion through a commissure. FIG. 1 is a schematic of a human heart **2** having a mitral valve **4.** FIGS. 2 and 4 show an exemplary mitral valve **4.** As can be seen in the figures, several commissure points (anterolateral commissure **4d** and posteromedial commissure **4e)** are presented at the ends of the valve leaflets **4a, 4b.**

With continued reference to FIGS. 6-18, the exemplary free end **22** is sized and dimensioned for insertion through one of the commissures. In the various embodiments, the free end **22** is configured to be atraumatic to avoid risk of injury to the valve tissue and leaflets. The free end **22** may be in the form of a blunt end, a ball tip, a curved tip (e.g., J-tip or pigtail), and other atraumatic shapes. In various embodiments, the free end **22** is configured with a sharp end to pierce tissue. In various embodiments, the free end **22** is separate from and extends outward from the main coils of the anchor **15.** In various embodiments, the main body coils of the anchor **15** circumscribe an area (in the case of a spiral coil) or a volume (in the case of a helical coil) having a diameter, and the free end **22** extends to a radius greater than the diameter of the anchor **15.** In various embodiments, the free end **22** extends to a radius substantially greater than the diameter of the anchor **15.** In various embodiments, the free end **22** is configured to circumscribe a larger diameter than the anchor **15.** In various embodiments, the free end **22** is configured to circumscribe all of the chordae tendineae of the native valve to be treated.

In various embodiments, wire **20** has varying stiffness along its length. The wire **20** may have two or more segments of differing stiffness and/or the stiffness may transition over its length. In various embodiments, wire **20** is attached to frame **12** at multiple points such that free end **22** is relatively flexible and the wire **20** is more rigid along portions where it is attached to the frame structure **12.**

In various embodiments, free end **22** extends radially outward from frame structure **12,** and in particular the remainder of wire **20.** As will be described below, the free end **22** is configured to encircle a larger radius than the main coils of the wire **20.** When the main coils of wire **20** have a generally curved shape (e.g., spiral, helical, tubular, frustoconoical, etc.), the free end **22** may extend radially outward from the curved shape. For example, when the main coils of wire **20** have a generally spiral shape, the free end **22** may extend radially outward from the spiral shape. When the main coils of wire **20** have a generally tubular shape, the free end **22** may extend radially outward from the tubular shape. When the main coils of wire **20** have a generally helical shape, the free end **22** may extend radially outward from the helical shape. When the main coils of wire **20** have a generally frustoconical shape, the free end **22** may extend radially outward from the frustoconical shape. The larger diameter facilitates capturing of the valve leaflets and/or chordae tendineae within the sweep of the free end **22** during rotation as will be described in more detail below.

A method of implanting valve prosthesis **10** in accordance with the present disclosure will now be described with reference to FIGS. 19-28. Although shown and described with respect to a mitral valve, one of ordinary skill in the art will understand that the principles described herein may be applied equally to other atrioventricular valves. Aspects of the procedure, delivery tool, and implanted valve prosthesis are similar to those described in U.S. Pat. Nos. 9,034,032; 9,005,273; 8,323,336; 8,075,615; 7,621,948; and 7,175,656 and U.S. Pub. No. 2011/0288637.

Prior to implantation, valve prosthesis **10** may be collapsed and loaded into a delivery device **30,** for example, a delivery catheter. The valve system may optionally be primed before or after loading into the delivery catheter **30.** FIG. 19 shows a cross-sectional side view of a heart **2** with a transseptal puncture **27** in the atrial septum thereof. The leaflets **42** of valve **4** do not fully prolapse and the patient is experiencing regurgitation.

Next, the delivery catheter **30** is inserted through an introducer into a vessel. The delivery catheter **30** can be guided over a guidewire to a target location using the Seldinger technique. In the illustrated embodiment, the delivery catheter **30** is guided to the left atrium **25** through a transseptal puncture **27** in conventional fashion as shown in FIG. 20.

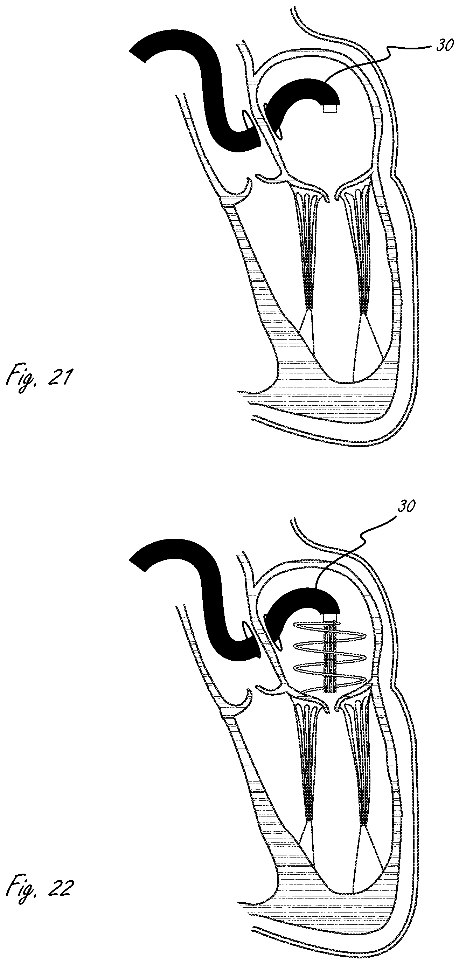

Turning to FIGS. 21-22, at this point, the end of the delivery catheter **30** is pointed towards the mitral valve **4.** Valve prosthesis **10** is then pushed out of the distal end of delivery catheter **30.** The delivery device **30** may comprise an outer catheter **50** and an inner catheter or shaft **52.** Delivery device **30** may refer to various components in certain embodiments. For example, delivery device **30** may refer to a steerable catheter for transseptal delivery. Delivery device **30** may refer to a catheter for delivery of the replacement valve, frame structure, and/or anchor. In some embodiments, delivery device 30 may refer to a first delivery device component detachably coupled to the anchor and a second delivery device component detachably coupled to the frame structure. In some embodiments, one or more valve prosthesis components may be different delivery devices or different components of a delivery device system. In some embodiments, once the delivery device **30** is in position, the delivery tube **52** extends out of the outer catheter **50** to move valve device **10** distally towards the native valve **4.** As the valve prosthesis **10** comes out from the delivery catheter **30,** an anchor **15,** such as wire **20,** is deployed (e.g., from a straightened shape within the delivery device **30**) to its pre-formed deployed shape and wraps around frame **12,** which remains in its collapsed state as shown in FIG. 22. The valve prosthesis **10** is then aligned with the target native valve **4** so the axis of the prosthetic valve **10** is aligned with a central axis of the native valve **4.**

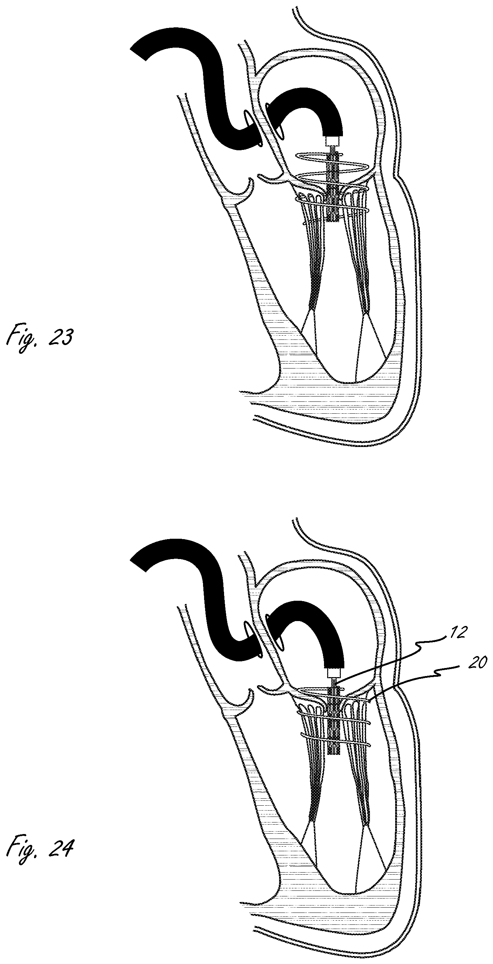

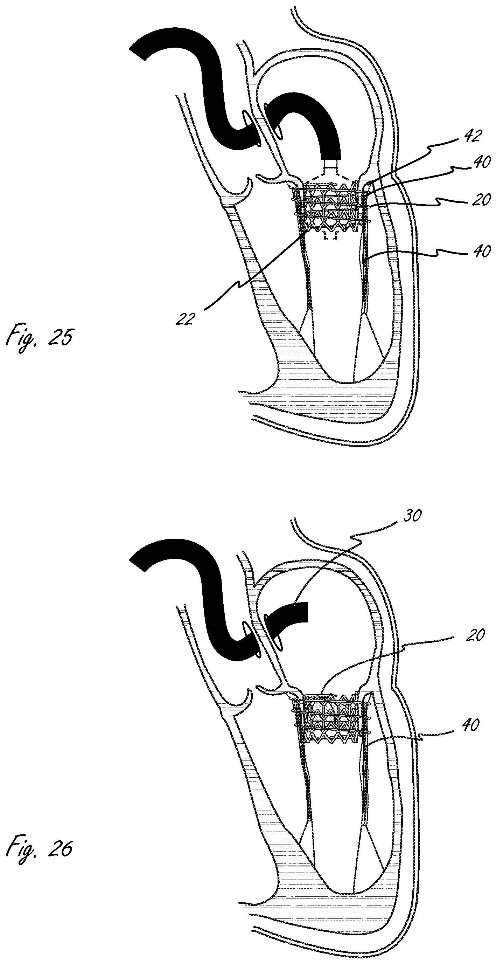

Turning to FIGS. 23-24, valve **10** is anchored to the native valve **4** using exemplary helical wire **20.** The valve prosthesis **10**-frame structure **12,** wire **20,** and valve segment **14-**are slowly rotated into the native mitral valve **4.** In the illustrated embodiment, a torquer is provided in the delivery catheter **30** for rotating valve **10.** Free end **22** of wire **20** is rotated through a commissure and extends below the native valve **4** annulus. The valve prosthesis **4** is further rotated so the free end **22** captures the chordae tendineae (also referred to as "papillary muscles") **40** and/or native valve leaflets **42.** As the wire **20** is continually rotated, the chordae tendineae 40 are gathered and pulled radially inward. Free end **22** has a larger radius than the main body of the helical coil (e.g., is disposed radially outward of the main body of the helical coil) in order to facilitate capture of the chordae tendineae **40** during rotation of the valve prosthesis **10.** Frame structure **12** also moves into the native valve **4** as the wire **20** is rotated. Valve prosthesis **10** is in the correct position when the chordae tendineae **40** have been captured to a sufficient degree and/or frame structure **12** is in the desired location in the native valve **40.** Insertion of the device through the native valve may be facilitated by the natural opening and closing of the native valve during the cardiac cycle. In the illustrated embodiment, the chordae tendineae **40** are pulled inwardly into a bunches (best seen in FIG. 25). The native valve leaflets **42** are also in communication with the helical coil **20.** At this stage valve device **10** is rigidly anchored adjacent the native valve **40** annulus.

If the clinician desires to remove or reposition the valve, the helical wire **20** can be counter-rotated to back out the device **10** from the native valve **4.** The implant rotation procedure can then be repeated.

Frame structure **12** is expanded once valve **10** is in the desired location as shown in FIG. 25. The frame structure **12** may comprise a first and second opposite ends, the first end extending above a native valve and the second end extending below the native valve when the frame structure **12** is anchored to the native valve **4.** In the illustrated embodiment, the frame structure **12** is expanded with a balloon **48** as shown in FIGS. 27-28. In various embodiments, the frame structure **12** is self-expanding. The self-expanding exemplary frame structure **12** is formed of a shape memory material or any material having superelastic properties. The self-expanding frame structure **12** is configured and expands in a similar manner to a self-expanding stent or scaffold. Expanding the frame structure **12** comprises removing a sheath (for example, outer sheath **50**) of the delivery device **30** from the frame structure **12.**

Once the frame structure **12** is expanded the entire valve assembly **10** is released from the delivery catheter **30** and the delivery catheter **30** is removed as shown in FIG. 26. In some embodiments, expansion of the frame structure **12** may occur simultaneously with release of the frame structure **12** from the delivery catheter **30.**

In the illustrated embodiment, the valve structure **14** and frame structure **12** are deployed together. One of ordinary skill in the art will appreciate, however, that the frame structure **12** can be deployed first and then receive the prosthetic valve segment **14.**

In various embodiments, valve prosthesis **10** does not include a valve segment **14.** Instead, the frame structure **12** and anchor **15** are positioned within the native valve **4.** The frame structure **12** is configured to receive a valve segment **14** delivered separately. In certain embodiments, the frame structure **12** can be configured to receive one of several valve sizes and types. In this manner, a clinician can choose the proper valve for the individual patient.

In the illustrated embodiment, the helical wire **20** of anchor **15** guides the valve system **10** along a desired axis into position adjacent the native valve **4.** The wire **20** also provides an initial anchoring. The valve prosthesis **10** is finally anchored when the frame structure **12** is expanded within the native valve **4.** The frame structure **12** dilates the valve leaflets **14** and the compressive force fixes the valve prosthesis **10** into position. Thereafter tissue ingrowth ensures the valve prosthesis **10** remains seated and does not migrate.

The valve devices described herein in accordance with the present disclosure provides several advantages over conventional valve systems. Embodiments described herein provide an easy-to-use, repositionable device. Unlike conventional valve systems, the valve prosthesis described herein reduces the risk of injuring or tearing chordae. Typical mitral valve replacement systems involve implanting a prosthetic annulus or ring around the valve. The ring increases the circumference of the valve and risks occluding the entry to the aortic valve. The valve device described herein overcomes these and other problems.

FIG. 29 illustrates another embodiment in accordance with the present disclosure. A valve prosthesis **10'** includes a helical wire **20'** and frame structure **12'.** Valve structure **10'** is similar to valve **10** except that valve segment **14'** is fixed within a separate end of frame structure **12'.** Wire **20'** is wrapped around a lower portion of the frame structure **12** having a smaller diameter than the upper portion of the frame structure **12** to which the valve segment **14'** is fixed.

FIGS. 30A to 30F illustrate several other embodiments in accordance with the present disclosure. Each of valves **10**a to **10**f includes a helical wire and frame. Each can optionally include a valve segment within the frame.

FIG. 30A shows a valve prosthesis **10**a which is similar to valve prosthesis **10** except that free end **22a** includes an atraumatic ball tip. Also, wire **20**a has a tubular shape at one end and a frustoconical shape at another end. Frame structure **12a** is substantially similar to frame structure **12.**

FIG. 30B shows a valve prosthesis **10**b which is similar to valve prosthesis **10** except that free end **22** has a pigtail tip. Also, wire **20**b is attached to an intermediate portion of frame structure **12b** instead of an end of the frame structure **12b.** Frame structure **12b** is substantially similar to frame structure **12.**

FIG. 30C shows a valve prosthesis **10c** which is similar to valve prosthesis **10** except that frame structure **12c** is a tubular structure instead of a scaffold or stent-like structure. The frame structure **12c** can be formed of expandable materials such as polyurethane or polycarbonate urethane. The wire **20c** is substantially similar to wire **20.** The free end **22c** is substantially similar to free end **22.**

FIG. 30D shows a valve prosthesis **10d** which is similar to valve prosthesis **10** except that the anchor **15** is formed of a three-dimensional surface **20d** instead of a wire **20.** Three-dimensional surface **20d** comprises a free end **22d,** which may be substantially similar to any of the free ends described herein. Frame structure **12d** is substantially similar to frame structure **12.**

FIG. 30E shows a valve prosthesis **10e** which is similar to valve prosthesis **10** except that frame structure **12e** has a conical shape instead of a tubular shape. One will appreciate from the description herein that the frame structure **12** may take a variety of shapes in accordance with the present disclosure. The wire **20e** is substantially similar to wire **20.** The free end **22e** is substantially similar to free end **22.**

FIG. 30F shows a valve prosthesis **10f** which is similar to valve prosthesis **10** except that the valve device **10f** includes a plurality of wires **20f** and **20f'**. The use of a plurality of wires **20f** and **20f'** provides increased anchoring security. Because it may be difficult to insert both free ends **22f** and **22f'**, one or both free ends **22f** and **22f'** may include a sharp point for piercing tissue. In this manner, the sharp end can pierce the valve annulus or leaflets. Barbs or other mechanisms may be employed to increase anchoring of the wire. For example, one or both of the wires **20f** and **20f'** may include a braided surface or barbs to prevent axial dislocation once it is screwed into place.

FIGS. 31-35 illustrate another valve prosthesis **10**g embodiment which is similar to valve prosthesis **10** except that the valve prosthesis **10**g is configured for anchoring from the bottom (i.e., ventricular side) of the native mitral valve annulus. By comparison to valve prosthesis **10,** valve prosthesis **10**g includes a helical anchor **15**g in a frustoconical (or conical) shape with a narrow section at the bottom of the frame structure **12**g and a wider section along a central portion of the device **10**g. In the illustrated embodiment, the widest section of the anchor **15**g is near a top of the coil **15**g. In the illustrated embodiment, the coiled anchor **15**g extends from a bottom of the frame structure **12**g to a top section of the frame structure **12**g in the collapsed (e.g., unexpanded) configuration. The frame structure **12**g, anchor **15**g, and valve segment (not shown) are otherwise generally similar to those of valve **10** or any of the frame structures, anchors, or valve segments described herein.

The anchor **15**g is inverted compared to anchor **15** in FIG. 6. A free end **22**g is positioned along a central section of the valve prosthesis **10**g. As will be described below in more detail with reference to FIGS. 43A-43AF, the valve prosthesis device **10**g may be configured to be inserted through the native valve (e.g. mitral valve) and anchored from below. In particular, the anchor **15**g may be configured to capture the chordae and/or native valve leaflets from below the valve annulus. This inverted design provides a simple transcatheter approach to the diseased native valve while providing a long-term and clinically useful anchoring mechanism.

The exemplary anchor **15**g is attached to the frame structure **12**g at one end **57.** In the illustrated embodiment, as best shown in FIG. 35, the end is fixed to a strut of the frame structure **12**g. This attached end can be attached by suitable means as would be understood by one of skill in the art from the description herein including, but not limited to, a weld, an adhesive, and a mechanical fastener. In the illustrated embodiment, the valve prosthesis **10**g includes optional eyelets **55** for supplemental securement of the coil anchor **15**g. The exemplary anchor **15**g is formed in the shape of a coil wire similar to the other anchor wires described herein and with a section extending through the eyelets **55.** The coil **15**g is slidably held within the eyelets **55.** The coil **15**g may be configured to retain its shape after deployment. As the frame structure **12**g is subsequently expanded, the coil wire anchor **15**g slides through the eyelets **55** to allow the frame structure **12**g to expand within the larger diameter of the anchor **15**g. The eyelets **55** hold the coil wire anchor **15**g in a radial position relative to the frame structure **12**g. This may mitigate against the risk of the coil **15**g bending away from the frame structure **12**g and/or fracture failure.

With continued references to FIGS. 31-32, in an exemplary embodiment, the anchor **15**g has a radius of curvature (**R**) is between about 15mm and about 50m. In various embodiments, **R** is about 35mm. In various embodiments, the height of the anchor (**Hₐ**) is between about 10mm and about 60mm. In various embodiments, the height of the anchor (**Hₐ**) is between about 3mm and about 60mm. In various embodiments, the height of the anchor (**Hₐ**) is between about 3mm and about 6mm. In various embodiments, the height of the anchor (**Hₐ**) is between about 3mm and about 50mm. In various embodiments, **Hₐ** is about 30mm. In various embodiments, the height of the frame structure (**H_{f}**) is between about 20mm and about 55mm. In various embodiments, **H_{f}** is about 30mm. In various embodiments, the width of the frame structure in a collapsed configuration (**W_{c}**) is between about 4mm and about 8mm. In various embodiments, **W_{c}** is about 6mm. In various embodiments, the width of the frame structure **12**g in an expanded configuration (**Wₑ**) is between about 25mm and about 35mm. In various embodiments, **W_{c}** is about 30mm. In various embodiments, the anchor **15**g is formed as a coil with varying widths and the minimum width (or radius) is about 10mm and the maximum radius is about 30mm.

It will be understood by one of ordinary skill in the art that any of the anchor embodiments described herein may be formed with similar dimensions as those described herein with reference to anchor **15**g, or any of the other anchors described herein.

It will be understood by one of ordinary skill in the art that any of the frame structure embodiments described herein may be formed with similar dimensions as those described herein with reference to frame structure **12**g, or any of the other frame structures described herein.

FIGS. 36-39 illustrate another valve prosthesis **10**h embodiment which is similar to valve proshtesis **10**g except that the anchor **15**h has a generally symmetrical tubular profile. A proximal end **57**h of anchor **15**h extends inwardly from the coil body to an attachment point on the frame structure **12**h. The end **57**h wraps around the frame structure **12**h and is fixed to the frame structure **12**h as described herein. The coil **20**h can have a portion of a turn around the collapsed frame structure **12**h. The coil **20**h can have one or more turns around the collapsed frame structure **12**h. A portion of the coil **20**h may extend through eyelets **55**h as described herein. Similar to valve **10**g, the frame structure **12**g may be allowed to expand by the coil anchor **15**g sliding through the eyelets **55**h. At the same time, the eyelets **55**h may maintain a relative radial position of the respective portion of the coil anchor **15**g. Although the embodiment shows eyelets **55**g, one of ordinary skill in the art will appreciate from the description herein that other mechanisms may be suitable for guiding the anchor **15**g including, but not limited to, hooks and guiderails or the like, or any combination thereof.

FIGS. 40-42 illustrate exemplary valve prosthesis embodiments **10**i, **10**j, and **10**k which are similar to valve prosthesis **10**g and having anchors **15**i, **15**j, and **15**k of varying configurations. In the illustrated embodiments, the exemplary anchors include an atraumatic tip **60** as described herein.

FIG. 40 illustrates a valve prosthesis **10**i which is similar to valve prosthesis **10**g except that the anchor **15**i does not have a linear or symmetrical shape. At least a portion of anchor **15**i has a tapered shape. In the illustrated embodiment, the anchor **15**i is formed as a coil and at least one turn of the windings has a curvilinear shape. Whereas at least some of the turns in the windings of anchor **10**g are generally flat in a plane, the respective turns in the valve prosthesis **10i** are configured to extend out of the plane in a three-dimensional, degenerate surface. In the illustrated embodiment, the curvilinear shape is more pronounced, and the radius of curvature is greater, on the turn at the top than the turns at the bottom. The overall shape of anchor **15i** is generally conical. This shape may promote insertion of the anchor through the native valve leaflets as will be described in more detail below.

FIG. 41 illustrates a valve prosthesis **10**j which is similar to valve prostheses **10**g, **10**h, **10i,** and **10**j. Anchor **15**j is formed as a coil with a complex, tapered shape. FIG. 41 illustrates a valve prosthesis **10**k which is similar to valve prostheses **10**g, **10**h, **10**i, and **10**j except that the anchor **15**k is configured for facilitating easier insertion through the native valve. In the illustrated embodiment, the anchor **15**k is formed as a coil with a relatively smaller form factor than the other anchors. The smaller radial width of the coil may promote insertion through the native valve while still having sufficient width to capture a necessary number of chordae and/or the native valve leaflets.

Any of the anchor embodiments described herein may have windings with varying shapes and curvatures. In various embodiments, a lower portion of the anchor has windings which curve in a first direction and an upper portion has windings which curve in a second direction (in planes generally perpendicular to a major axis of the frame structure and/or anchor). In various embodiments, the second direction is opposite the first direction. In various embodiments, the anchor includes a first portion having a first radius of curvature (in a plane generally perpendicular the major axis of the frame structure and/or anchor), and a second portion having a second radius of curvature. In various embodiments, the anchor includes a third portion having a third radius of curvature. In various embodiments, the anchor includes a fourth portion having a fourth radius of curvature. In various embodiments, the anchor includes a plurality of portions each having a unique radius of curvature. In various embodiments, the respective radii of curvature are all different. In various embodiments, the second radius of curvature is greater than the first, and the third radius of curvature is greater than the second. In various embodiments, the radius of curvature of the upper windings is greater than 30mm. In various embodiments, the radius of curvature of the lower windings is greater than 10mm.

FIG. 42 illustrates an exemplary modality for attachment of the anchor **15**j to a frame structure **12**j. In various embodiments, the anchor **15**j is welded to the frame structure **12**j, for example, to one of the struts of the frame structure **12**j. In various embodiments, one end of the anchor **15**j is configured to extend inside the frame structure **12**j where it is mechanically fastened. In the embodiment illustrated in FIG. 42, the anchor **15**j is fixed to a bottom-most (inferior) end of the frame structure **12**j.

A method of using a valve device similar to valve devices **10**g, **10**h, **10**i, **10**j, **10**k, **10**l, **10**m, **10**n, **10**o, **10**p, etc. will now be described with reference to FIGS. 43A to 43AF.

FIGS. 43A-43AF show sequential views of a method of implanting a valve prosthesis **10** using a delivery device **30'.** The valve prosthesis **10** may be similar to any of the valve prostheses described herein or understood by one of ordinary skill in the art from the description herein. For example, valve prosthesis **10** may be substantially similar to the valve prosthesis **10**g shown in FIGS. 31-35 and may comprise a frame structure **12**g and anchor **15**g as described herein. The delivery device **30'** may be substantially similar to any of the delivery devices described herein or understood by one of ordinary skill in the art from the description herein. The delivery device **30'** may comprise an inner shaft or delivery tube **52** as described herein. The delivery device **30'** may optionally comprise an outer shaft or outer catheter **50,** a guidewire **54,** and/or an inflatable balloon **48,** in any combination thereof as desired by one of ordinary skill in the art. Not all elements are labeled in each of FIGS. 43A-43AF in order to make the illustrations less cluttered and easier to see.

While the method shown in FIGS. 43A-43AF is described in relation to a mitral valve replacement procedure, it will be understood by one of ordinary skill in the art that the methods described herein may be applied to a variety of procedures or anatomical areas, for example other atrioventricular valves of the heart or the like. For example, the methods described herein may be applied to replacement of a diseased aortic valve or tricuspid valve.

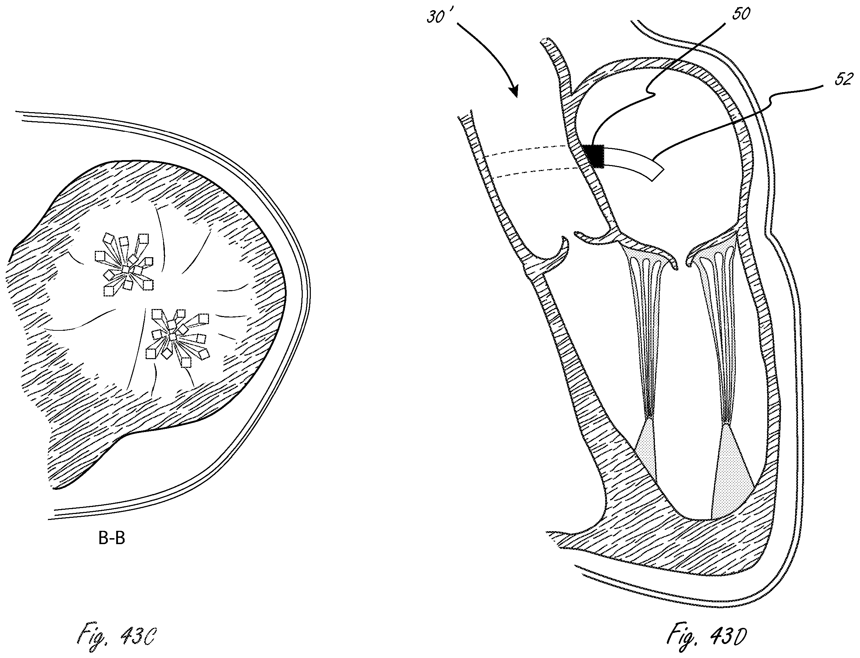

FIGS. 43A-43C shows various cross-sectional views of a heart **2** having a diseased mitral valve **4** which may be treated using the devices, systems, and methods described herein. The mitral valve **4** sits between the left atrium **25** and the left ventricle **26** and, when functioning properly, allows blood to flow from the left atrium **25** to the left ventricle **26** while preventing backflow or regurgitation in the reverse direction. As shown in FIG. 43A, the native valve leaflets **42** of the diseased mitral valve **4** do not fully prolapse and the patient experiences regurgitation. FIG. 43B shows a cross-sectional view of the heart **2** taken along line A-A, shown in FIG. 43A, which shows the native valve leaflets **42** of the mitral valve **4** from the viewpoint of the left atrium 25. FIG. 43C shows a cross-sectional view of the heart **2** taken along line B-B, shown in FIG. 43A, which shows the chordae tendineae **40** of the left ventricle **26.**

As shown in FIG. 43D, a distal end of the delivery device **30'** may be inserted into the left atrium **25** of the heart **2** via a transseptal puncture as described herein. For example, the distal ends of inner shaft **52** and/or outer sheath **50** may be advanced into the left atrium **25** of the heart **2.** The inner shaft **52** may optionally be advanced distally into the left atrium **25** away from the distal end of the outer sheath **50.** In some embodiments, advancing the inner shaft **52** relative to the outer sheath **50** may aid in deployment and/or placement of the valve prosthesis **10**g as described herein. Alternatively, both the inner shaft **52** and the outer sheath **50** may be advanced distally into the left atrium **25** through the transseptal puncture.

FIGS. 43E-43H show deployment of the anchor **15**g from the distal end of the delivery device **30'.** As described herein, at least a portion of the valve prosthesis **10**g may be deployed from an undeployed (for example, compressed or unexpanded) configuration to an expanded configuration within the left atrium **25.** At least a portion of the anchor **15**g may be deployed from a delivery and/or elongated configuration to a deployed configuration within the heart. For example, anchor **15**g may be actuated from an elongated configuration (e.g., from a straightened shape) to a deployed configuration (e.g., a pre-formed shape for implantation, such as a spiral, helical, or conical shape) within the left atrium **25** as described herein. In some embodiments, the anchor **15**g may be deployed from the inner shaft **52** by pushing the anchor **15**g out of the inner shaft **52,** releasing the anchor **15**g from radial constraint by retracting the outer sheath **50,** or the like as described herein. In some embodiments, the anchor **15**g may be pushed out the inner shaft **52** using a pusher on a proximal handle (not shown) located outside the body. After the anchor **15**g has been deployed from the delivery device **30',** the frame structure **12**g may be at least partially deployed from the delivery device **30'** as shown in FIG. 43H so as to place the frame structure **12**g within the anchor **15**g. The frame structure **12**g may be deployed from the delivery device **30'** in either the unexpanded configuration or the expanded configuration, depending on the location of deployment, as will be understood by one of ordinary skill in the art based on the teachings herein.

FIGS. 43I-43K show advancement of the valve prosthesis **10**g, with anchor **15**g deployed around the unexpanded frame structure **12**g, towards the native valve **4** requiring treatment. The distal end of the delivery device **30'** (for example, the distal end of the inner shaft **52** and/or the outer sheath **50**) may be steered such that the distal end of the delivery device **30'** points toward the atrial side of the native valve **4.** Such steering may occur prior to, during, or after deployment of at least a portion (for example deployment of an anchor **15**g) of the valve prosthesis **10**g. In some embodiments, the distal end of the outer sheath **50** may be steerable. Alternatively, or in combination, the inner shaft **52** may comprise a joint configured to change an angle of the distal portion of the inner shaft **52** relative to a proximal portion of the inner shaft **52.** The inner shaft **52** may be steered by changing the angle of the distal portion of the inner shaft **52** relative to the proximal portion of the inner shaft **52.** The angle of the joint may be changed passively or actively. In various embodiments, the angle may be selectively controlled by a proximal handle. For example, pull wires or other mechanisms may connect to the joint to controls on the handle.

FIGS. 43L-43P show the valve prosthesis **10**g being advanced through the native valve **4** by the delivery device **30'** from the left atrium **25** to the left ventricle **26.** Advancement of the valve prosthesis **10**g and optionally delivery device **30'** through the mitral valve **4** may be facilitated by the natural opening and closing of the valve **4** during the cardiac cycle. The distal end of the delivery device **30'** and/or valve prosthesis **10**g may be configured to be advanced from a first side of a native valve to a second side of the native valve. For example, the distal end of the delivery device **30'** and/or valve prosthesis **10**g may be advanced from a left atrial side of a mitral valve **4** to a left ventricular side of a mitral valve **4.** Advancing the anchor **15**g may comprise pushing the anchor **15**g through the native valve **4.** Alternatively, or in combination, advancing the anchor **15**g may comprise rotating the anchor **15**g through the native valve **4.** In some instance, the combination of rotational motion and pushing may facilitate advancement of the device from the first side of the native valve **4** to the second side of the native valve **4.** Rotation of the valve prosthesis **10**g, for example rotation of the anchor **15**g and/or frame structure **12**g, may be facilitated by the inner shaft **52** described herein. For example, the inner shaft **52** may transmit rotational motion to the valve prosthesis **10**g in order to rotate the valve prosthesis **10**g during advancement through the native valve **4.**

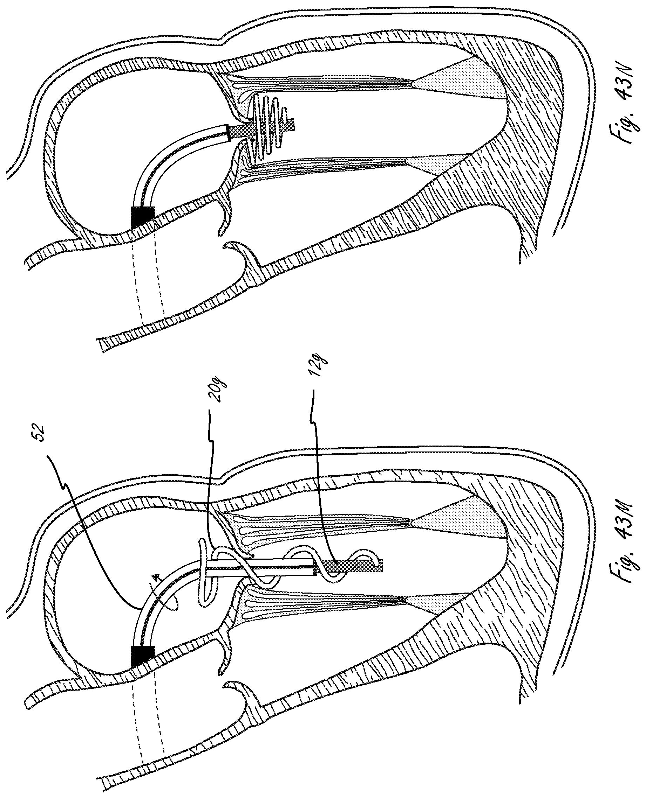

In some instances, advancing the anchor **15**g through the native valve **4** may cause the anchor **15**g to be stretched or elongated as shown in FIG. 43M. Rotation of the anchor **15**g during advancement may assist with the stretching process by aiding in unwinding the anchor **15**g. Additionally, the rotational motion may reduce the risk of the free end **22**g of the anchor **15**g undesirably engaging other anatomy during insertion through the native leaflets **43.** The anchor **15**g may be sufficiently elastic so as to enable relatively easy insertion through the native valve **4** and/or reduce the risk of injury to the native leaflets **43.** At the same time, the anchor may be sufficiently rigid for guiding through and anchoring to the structures in the heart. After the anchor **15**g has stretched through the native valve **4** it may return to the deployed configuration as shown in FIG. 43N. FIGS. 43O-43P show the position of the valve prosthesis **10**g within the ventricle **26** and, in particular, the position of the anchor **15**g relative to the native chordae tendineae **40** and native valve annulus.

In some embodiments, the anchor **15**g may be advanced into the ventricle after being fully deployed from the delivery (e.g., elongated) configuration to the deployed configuration.

In some embodiments, the anchor **15**g may be advanced into the ventricle before being deployed from the delivery (e.g., elongated) configuration to the deployed configuration.

FIGS. 43Q-43T show rotation of the valve prosthesis **10**g around one or more structures on the ventricular side of the mitral valve **4.** The one or more structures may comprise one or more valve leaflets **43** and/or one or more chordae tendineae **40.** After the anchor **15**g has been at least partially deployed within the left ventricle **26** adjacent one or more chordae tendineae **40,** the valve prosthesis **10**g may be rotated to capture and anchor the native chordae **40** and/or native leaflets **43.** The free end **22**g of the anchor **15**g may extend radially outward from the rest of the anchor **15**g to facilitate capture of the native structures. The free end **22**g of the coil**15**g may be rotated around one or more of the chordae tendineae **40** as shown in FIGS. 43Q-43R. Additional rotation of the valve coil **15**g may gradually capture additional chordae tendineae **40** as shown in FIGS. 43S-43T.

Rotation of the valve prosthesis **10**g, for example, rotation of the anchor **15**g and/or frame structure **12,** may be facilitated by the delivery device **30'** described herein. For example, the inner shaft **52** may be rotated and rotational motion may be transmitted from the inner shaft **52** to the valve prosthesis **10**g in order to rotate the valve prosthesis **10**g around one or more of the structures on the ventricle side of the mitral valve 4 as described herein.

In some embodiments, the valve prosthesis **10**g, for example anchor **15**g, may be counter-rotated in order to reposition the anchor **15**g with respect to the chordae tendineae **40** before continuing the rotation in the first direction. For example, counter-rotation may be applied if the chordae tendineae **40** are caught by the free end of the anchor **15**g (or another part of the valve prosthesis **10**g or delivery device **30**) during the initial rotation. In such instances, counter-rotation may enable to the clinician to disengage some or all of the chordae tendineae **40** to reduce the stress or torque on the chordae tendineae **40** (e.g., by adjusting the position of the valve prosthesis **10**g) before resuming rotation. As another example, the anchor **15**g may encounter friction or other resistance to rotation. In this case the clinician may counter-rotate the anchor **15**g to return to the original position and then begin rotating the anchor **15**g to re-start and/or continue encircling chordae tendineae **40.** Rotation and counter-rotation may be applied as many times as desired by the clinician in order to properly position the anchor **15**g around the valve structures.

FIGS. 43U-43V show the valve prosthesis **10**g wrapped around the captured chordae tendineae **40.** The valve prosthesis **10**g may be rotated around the chordae tendineae **40** such that the chordae tendineae **40** are pulled inwardly into bunches. As shown in FIG. 43U, the native valve leaflets **43** may also be in communication with the valve prosthesis **10**g. The valve prosthesis **10**g may be rotated to capture enough chordae tendineae **40** and/or valve leaflets **43** to rigidly anchor the anchor **15**g adjacent the native valve annulus. The valve prosthesis **10**g may be anchored by wrapping around only a portion of the chordae **40.** Although it may be possible to capture all or substantially all the chordae **40,** this may not be necessary to provide sufficient anchoring of the prosthesis **10**g. As described further herein, the prosthesis may be further anchored by expansion of the frame structure **12**g within the native valve and against the anchor **15**g.

In some embodiments, the anchor **15**g may be deployed such that at least a portion of the anchor **15**g resides within a subvalvular plane. For example, at least 50 %, 60%, 70%, 80%, 90%, 100% of the anchor **15**g may reside within the subvalvular plane after being deployed. The subvalvular plane may be located at the posterior valve annulus, below the valve annulus and around the native valve leaflets **43,** and/or parallel to a plane within at least three points of the plane in which the valve annulus resides. In some instances, the anchor **15**g may be rotated in the subvalvular plane around the chordae tendineae **40.** In some instances, the anchor **15**g may be rotated in a plane below to the subvalvular plane in order to encircle the chordae tendineae **40** before the anchor **15**g is moved into the subvalvular plane (e.g., by pulling the anchor **15**g into the sub-annular space).

Once the anchor **15**g has been anchored adjacent to the native valve 4, the frame structure **12**g and prosthetic valve segment **14** may be expanded at least partially within the anchor **15**g as described herein. The frame structure **12**g and the valve segment **14**g may be deployed (e.g., expanded) simultaneously. Alternatively, or in combination, the frame structure **12**g and the valve segment **14**g may be deployed sequentially, for example by first expanding the frame structure **12**g and then receiving the prosthetic valve segment **14**g therein.

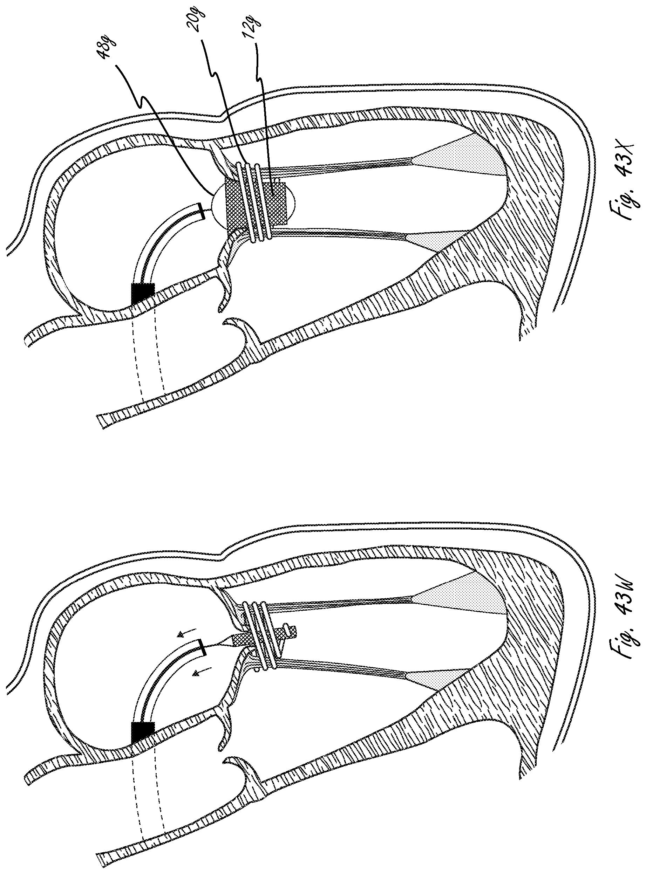

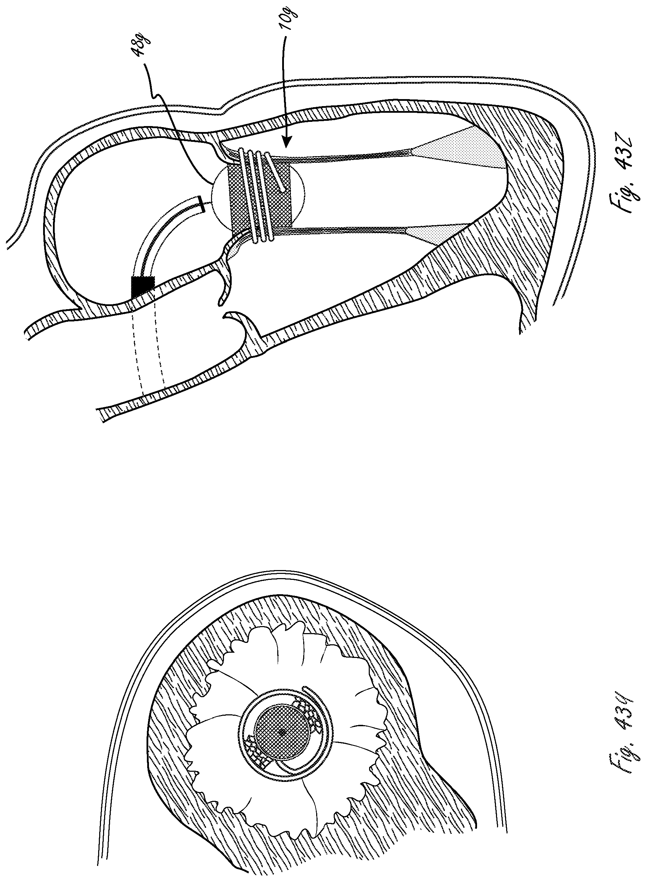

FIGS. 43W-43AA show expansion of the frame structure **12**g within the native valve **4.** The frame structure **12**g may be expanded within the native valve **4** from an unexpanded configuration to an expanded configuration. In some embodiments, at least a portion the frame structure **12**g may be expanded within at least a portion of the deployed anchor **15**g to anchor the frame structure **12**g to the native valve **4.** In some embodiments, the frame structure **12**g may comprise an expandable stent. In some embodiments, the frame structure **12** of valve prosthesis **10**g may be self-expandable. In some embodiments, the frame structure **12** of valve prosthesis **10**g may be balloon-expandable. The delivery device **30'** may comprise a balloon **48**g which may be disposed within the valve prosthesis 10g in order to expand the valve prosthesis **10**g. The balloon **48**g may be positioned within at least a portion of the valve prosthesis **10**g, for example within at least a portion of frame structure **12** in an uninflated configuration, as shown in FIG. 43W, prior to being inflated. The inflatable balloon **48**g may, for example, be disposed within the inner shaft **52** or outer sheath **50** while the anchor **15**g is being positioned adjacent the native valve **4** and then advanced therefrom (or the inner shaft **52** or outer sheath **50** is retracted therefrom) to be positioned within the frame structure **12**g. Alternatively, the inflatable balloon **48**g may be disposed within the frame structure **12**g during placement of the valve prosthesis **10**g. FIGS. 43X-43Y show the frame structure **12**g partially expanded by partially-inflated balloon **48**g. As shown in FIG. 43Y, the frame structure **12**g may be partially expanded towards the anchor **15**g in order to capture the chordae tendineae **40** therebetween. As the frame structure **12**g continues to be expanded to a fully expanded state, as shown in FIGS. 43Z-43AA, the chordae tendineae **40** may be sandwiched between the anchor **15**g and the frame structure **12**g. The frame structure **12**g and anchor **15**g may thus be anchored to the chordae tendineae **40.**

The valve prosthesis **10**g may then be released from the delivery device **30'.** In some embodiments, releasing the valve prosthesis **10**g may comprise releasing the anchor **15**g and/or the frame structure **12**g. Releasing the valve prosthesis **10**g from the delivery device **30'** may comprise expanding the valve prosthesis **10**g from the unexpanded configuration to the expanded configuration. For example, expanding the frame structure **12**g and releasing the frame structure **12**g may occur simultaneously as described herein. Alternatively, the frame structure **12**g may be released prior to or after being expanded.

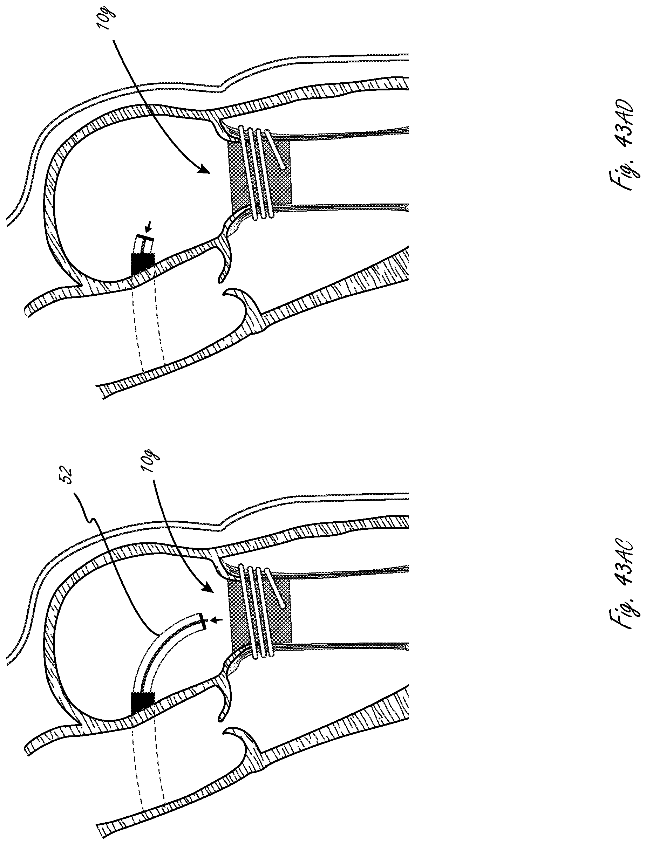

FIGS. 43AB-43AD show deflation of the balloon **48**g (FIG. 43AB), retraction of the balloon 48g into inner shaft 52 (FIG. 43AC), and removal of the delivery device **30'** from the heart **2** (FIG. 43AD). After the frame structure **12**g has been expanded and anchored to the native valve **4** as described herein, the inflatable balloon **48**g may be deflated. The balloon **48**g may be retracted back into the delivery device **30',** for example into inner shaft **52.** The delivery device **30'** may then be removed from the heart **2.**

FIGS. 43AE-43AF show the valve prosthesis **10**g fully expanded with the native valve leaflets **42** and chordae tendineae **40** captured between the frame structure **12**g and the anchor **15**g. As described herein, the valve prosthesis **10**g may comprise one or more valve segments **14**g disposed therein to replace the native valve leaflets **42.**

Although the steps above show a method of deploying a valve prosthesis **10** within a native valve **4** in accordance with embodiments, a person of ordinary skill in the art will recognize many variations based on the teaching described herein. The steps may be completed in a different order. Steps may be added or deleted. Some of the steps may comprise sub-steps. Many of the steps may be repeated as often as necessary to assemble at least a part of an article.

For example, in some embodiments deploying the valve prosthesis **10** may occur in multiple steps such that a portion of the valve prosthesis **10** (e.g., anchor **15**) may be deployed before another portion the valve prosthesis **10** (e.g., frame structure **12**). Alternatively, or in combination, in some embodiments, deploying the anchor **15** may occur in multiple steps such that a portion of the anchor **15** may be deployed before being advanced through the native valve **4** and another portion of the anchor **15** may be deployed after being advanced through the native valve **4.** Alternatively, or in combination, the delivery device **30** may be advanced from the left atrium **25** to the left ventricle **26** with the valve prosthesis **10** undeployed. In many embodiments, the frame structure may **12** be self-expanding and the balloon **48** may not be necessary for expansion of the frame structure **12.** Alternatively, or in combination, the anchor **15** may be released after the frame structure **12** has been expanded within it.

In some embodiments, any of the valve prostheses described herein may be deployed to replace a diseased mitral valve. The first side of the native valve may comprise a left atrium and the second side of the native valve may comprise a left ventricle.

In some embodiments, any of the valve prostheses described herein may be deployed to replace a diseased tricuspid valve. The first side of the native valve may comprise a right atrium and the second side of the native valve may comprise a right ventricle.

In some embodiments, any of the valve prostheses described herein may be deployed to replace a diseased aortic valve. The first side of the native valve may comprise a left ventricle and the second side of the native valve may comprise an aorta.

It will be understood by one of ordinary skill in the art that, while FIGS. 43A-43AF refer to delivery of valve prosthesis **10**g, similar steps may be used for delivery of any of the valve prosthesis described herein.

FIGS. 44-49 show another exemplary valve prosthesis device **10**l which is similar to valve prosthesis devices **10** and **10**g-**10**j except that the frame structure **12**l is self-expanding. As shown in FIGS. 44-47, the valve prosthesis **10**l includes a helical coil **20**l similar to helical coils **20** and **20**g. Frame structure **12**l is similar frame structures **12** and **12**g-**12**j except that it is configured to expand to the deployed shape upon removal of the sheath of the delivery device. The frame structure **12**l is shown in the drawings in both the collapsed and deployed shapes for ease of understanding. The exemplary frame structure **12**l may be formed of a shape memory material or any material having superelastic properties. The exemplary frame structure **12**l is formed with a diamond pattern, but one of ordinary skill in the art will appreciate from the description herein that many other patterns and configurations will be suitable. The exemplary frame structure **12**l is configured to expand in a similar manner to a self-expanding stent or scaffold.

FIGS. 48 and 49 show the exemplary valve prosthesis device **10**l loaded on an exemplary delivery tool or catheter **30**l. The manner of implanting the valve prosthesis device **10**l is similar to the method described herein with respect to valve device **10**g except that the frame structure **12**l is configured to expand on its own rather than under the force of a balloon. In this case, the frame structure **12**l and prosthetic valve segment can be easily deployed in one shot after the coil **15**l has been anchored to one or more structures adjacent the native valve. This may reduce the time of the implantation procedure and may eliminate complications like a system for deploying, inflating, and deflating the balloon used in a balloon-expandable embodiment.

The valve prosthesis device and implant method described herein in accordance with the present disclosure may provide many advantages as will be understood by one of ordinary skill in the art. The overall device and method may provide a simpler way to approach the native valve compared to existing devices. The system may enable a transcatheter approach through the septal wall compared to more invasive transapical approaches. The device may provide a consistent and relatively easy mechanism for anchoring to the native valve. Clinicians need only use the common technique of inserting the device through the valve and then rotating the anchor. The coil may provide preliminary anchoring in the native valve. If desired, the clinician can readjust the anchor and/or retrieve the anchor (e.g. by counterrotation). The device is then easily set by expanding within the native valve leaflets. The device and methods in accordance with the present disclosure may also address unmet clinical needs with atrioventricular repair and replacement. Existing devices face challenges with the complex anatomy of the mitral and tricuspid valves, for example. The present disclosures address these complications by reshaping the native valve annulus to a conventional round shape and providing a robust, yet simple, anchoring mechanism.

FIG. 50 shows a side view of a valve prosthesis system **100**m comprising a frame structure 12m and an anchor **15**m loaded on a distal end of a delivery device **30**m with the anchor **15**m in an elongated delivery configuration. FIG. 51 shows a side view of the valve prosthesis system **100**m of FIG. 50 with the anchor **15**m in a deployed configuration. The valve prosthesis may be substantially similar to any of the valve prostheses described herein except that the anchor may be detachably coupled to the delivery device during delivery as described herein instead of the frame structure. By coupling the anchor to the delivery device instead of the frame structure, the frame structure may be expanded inside the anchor without altering the shape or position of the deployed anchor during expansion, which may lead to a better connection between the anchor and the frame structure adjacent the native valve than may have been possible if expansion of the frame structure caused deflection or movement of the deployed anchor.

The frame structure **12**m may have an unexpanded (for example, a compressed configuration) and an expanded configuration (not shown). The frame structure 12m is shown in the unexpanded configuration. The anchor **15**m may comprise a wire **20**m having a free end **22**m. The anchor **15**m may be configured to be fully advanced from a first side of a native valve in a patient (e.g. an atrial side) to a second side of the native valve (e.g., into a ventricle of the heart) and anchor the frame structure **12**m to the native valve when the frame structure **12**m is in the expanded configuration adjacent the native valve. The delivery device **30**m may comprise an outer sheath (e.g. an outer catheter, not shown), an inner shaft **52** (e.g., a delivery tube) disposed within a lumen of the outer sheath, and a guidewire **54** disposed within a lumen of the inner shaft **52.** A proximal end **57** of the anchor **15**m may be detachably coupled to the inner shaft **52** during delivery to the native valve. The outer sheath may be steerable.

The anchor **15**m may comprise an elongated delivery configuration (shown in FIG. 50) and a deployed configuration (shown in FIG. 51). The anchor **15**m may be configured to be actuated from the delivery configuration to the deployed configuration adjacent the native valve. In various embodiments, the anchor **15**m may be self-expanding and may move to the deployed configuration as it is removed from the delivery sheath. In various embodiments, the anchor may be configured to self-assemble when it is deployed in the heart cavity (e.g. in a left atrium). Retraction of the guidewire **54** into the lumen of the inner shaft **52** may actuate the anchor 15m into the deployed configuration. Alternatively, or in combination, the anchor **15**m may be maintained in the elongated configuration by radial constriction from the outer sheath. Advancement of the inner shaft **52** out of the lumen of the outer sheath may actuate the anchor **15**m into the deployed configuration.

A proximal end **57** of the anchor **15**m may be detachably coupled to the inner shaft **52** of the delivery device **30**m. The proximal end **57** may be configured to remain engaged with the inner shaft 52 after being actuated from the elongated configuration to the deployed configuration adjacent the native valve. The frame structure **12**m may be configured to remain in its unexpanded configuration while the anchor **15**m is in the deployed configuration.

The proximal end **57** of the anchor **15**m may be detachably coupled to the inner shaft **52** of the delivery device **30**m by radial constriction from the outer sheath. Retraction of the outer sheath away from the proximal end **57** of the anchor **15**m may detach the anchor **15**m from the delivery device 30. Alternatively, or in combination, the proximal end **57** of the anchor may be detachably coupled to the inner shaft **52** of the delivery device **30**m by an attachment element **58.** Alternatively, or in combination, the proximal end **57** of the anchor **15**m may be detachably coupled to the inner shaft **52** of the delivery device **30**m by a weak adhesive.

The anchor **15**m may be configured to rotate when the inner shaft **52** is rotated. Rotation of the anchor may aid in advancement of the anchor to the second side of the native valve. Alternatively, or in combination, rotation of the anchor, for example a wire **20**m comprising a free end **22**m, may aid in capture of one or more structures on the second side of the native valve by the free end **22**m as described herein. By capturing one or more structures on the second side of the native valve, the anchor **15**m may maintain its position relative to the native valve and provide an anchor point for the frame structure **12**m when in the expanded configuration.

The frame structure **12**m may comprise an unexpanded configuration and an expanded configuration as described herein. The expanded configuration may have a generally tubular expanded shape. The frame structure **12**m may be configured for expanding within the native valve of the patient. In some embodiments, the unexpanded configuration may be sized and dimensioned for percutaneous insertion and the expanded configuration may be sized and dimensioned for implantation in the native valve of the patient.

Similar to the other frame structures described herein, the frame structure **12**m may comprise a first and second opposite ends, the first end extending above a native valve and the second end extending below the native valve when the frame structure **12**m is anchored to the native valve. Alternatively, the frame structure **12**m may be configured to sit entirely below the native valve when the frame structure **12**m is anchored to the native valve.

In some embodiments, similar to other frame structures described herein, the frame structure may comprise an expanded outer periphery in the expanded configuration and a compressed outer periphery when subject to an external radial force in the unexpanded configuration. The compressed outer periphery may be smaller in diameter than the expanded outer periphery.

The frame structure **12**m may be balloon-expandable, self-expanding, or otherwise expansible as will be understood by one of ordinary skill in the art from the description herein.

For example, the delivery system **30**m may comprise an inflatable balloon (not shown) disposed within the frame structure **12**m. Inflation of the balloon may cause expansion of the frame structure **12**m as described herein.

Alternatively, or in combination, the frame structure **12**m may be maintained in the unexpanded configuration by radial constriction from the outer sheath of the delivery device **30**m. Advancement of the inner shaft out of the lumen of the outer sheath may actuate the frame structure into the expanded configuration.

The frame structure **12**m may be detachably coupled to the delivery device **30**m in the unexpanded configuration during delivery to the native valve. Expansion of the frame structure **12**m to the expanded configuration may detach the frame structure from the delivery device.

Similar to other frame structure and anchor embodiments described herein, at least a portion the frame structure **12**m may be expanded within at least a portion of the deployed anchor **15**m to anchor the frame structure **12**m to the native valve. For example, the anchor **15**m may be deployed such that it captures one or more structures therein, for example one or more chordae tendineae and/or one or more valve leaflets. Expansion of the frame structure **12**m, or a portion thereof, within the anchor **15**m may compress the capture structures between the frame structure **12**m and the anchor **15**m to anchor the frame structure **12**m in place.

The guidewire **54** may comprise a nosecone **54a** configured to facilitate guidance of the guidewire to the native valve.

Similar to other wires described herein, the wire **20**m may comprise a helical wire in the deployed configuration. The free end **22**m of the helical wire **20**m may extend radially outward from the frame structure **12**m, and in particular from the remainder of the wire **20**m. In some embodiments, the helical wire **20**m may have a generally tubular shape. The free end **22**m of the helical wire **20**m may extend radially outward from the tubular shape. In some embodiments, the helical wire **20**m may have a generally frustoconical shape. The free end **22**m of the helical wire **20**m may extend radially outward from the frustoconical shape. In some embodiments, the helical wire **20**m may have a generally cylindrical shape. The free end **22**m of the helical wire **2**0m may extend radially outward from the cylindrical shape. The free end **22**m may be configured to encircle a larger radius than the main coils of the helical wire **20**m. The larger diameter may facilitate capturing of one or more structures, for example the valve leaflets of the chordal tendineae within the sweep of the free end **22**m when rotated as described herein.

Optionally, the anchor **15**m, or any of the anchors described herein, may comprise a first portion comprising the helical wire **20**m and another portion. Alternatively, or in combination, the anchor **15**m may comprise a plurality of helical wires **20**m. For example, the anchor 15m may comprise at least two helical wires **20**m having the same or different diameters. Alternatively, or in combination, the anchor **15**m may comprise at least two helical wires **20**m having the same or different winding pitches.

As with other anchors described herein, the free end **22**m of the wire **20**m may be sized and dimensioned for insertion through the native valve, for example through tissue at or near a commissure of the native valve or through the valve opening itself. In some embodiments, the free end **22**m of the wire **20**m may comprise an atraumatic tip to avoid reduce risk of injury to the native valve tissue and leaflets. For example, the free end may comprise a blunt end, a ball tip, a curved tip (e.g. J-tip or pigtail), or other atraumatic shapes. Alternatively, the free end **22**m of the wire **20**m may be configured for piercing tissue. In various embodiments, the free end **22**m is separate from and extends outward from the main coils of the anchor **15**m. In various embodiments, the main body coils of the anchor **15**m circumscribe an area (in the case of a spiral coil) or a volume (in the case of a helical coil) having a diameter, and the free end **22**m extends to a radius greater than the diameter of the anchor **15**m. In various embodiments, the free end **22**m extends to a radius substantially greater than the diameter of the anchor **15**m. In various embodiments, the free end **22**m is configured to circumscribe a larger diameter than the anchor **15**m. In various embodiments, the free end **22**m is configured to circumscribe all of the chordae tendineae of the native valve to be treated.

Wire **20**m, or any of the wires described herein, may be formed of a material having sufficient rigidity to hold a predetermined shape. The wire may, for example, be formed of a shape memory material (e.g. NiTi). It may be desirable for at least an end portion (e.g. free end **22**m) to be relatively rigid such that it can exert a force to move chordal tendineae, while still retaining flexibility to be collapsed within a delivery device. In various embodiments, the end portion only needs sufficient rigidity to hold its shape and will deform under a load. For example, the end portion may be configured with a similar rigidity to a guidewire, or slightly stiffer.

The frame structure **12**m, or any of the frame structures described herein, may be configured like a stent. The frame structure **12**m may, for example, comprise a scaffold in a diamond pattern formed from a shape memory material (e.g. NiTi). One of ordinary skill in the art will appreciate that many other structures, materials, and configurations may be employed for the frame structure **12**m, or any of the other frame structures described herein. For example, the frame structure **12**m may be formed of a polymer of sufficient elasticity. The frame structure **12**m may be formed of a combination of metal and polymer, such as metal (e.g. shape memory material) covered in polymer. The frame structure **12**m may include a variety of patterns besides diamond shapes.

The frame structure **12**m, or any of the frame structures described herein, may comprise a valve segment (not shown) disposed therein. As described above, valve segment is used somewhat interchangeably with prosthetic valve leaftlet and generally refers to the prosthetic leaflets and frame. As used herein, "prosthetic valve" may refer to all manner of prosthetic and artificial replacement valves including tissue (biological valves), tissue-engineered valves, polymer valves (e.g. biodegradable polymer valves), and even certain mechanical valves. The valve segment can be similar to existing transcatheter valves. The valve segment can be similar to existing surgical tissue valves, and mechanical valves. At least a portion of the valve segment may be positioned within at least a portion of the frame structure. The valve segment may include leaflets formed of multi-layered materials for preferential function. The valve segment may comprise at least one leaflet having an inner layer and an outer layer. The valve segment may be attached to a valve structure which is in turn connected to the frame structure **12**m. The valve structure may be connected to the frame structure **12**m before or after the frame structure 12m has been deployed adjacent a native valve. The valve segment may be attached directly to the frame structure **12**m. The frame structure **12**m may be attached to a leaflet, for example an outer layer of a leaflet, at one or more ends of the frame structure **12**m. The frame structure **12**m may be attached to a leaflet, for example an outer layer of a leaflet, at one or more intermediate portions of the frame structure **12**m. The valve segment may comprise a plurality of leaflets. The valve segment may comprise a biocompatible one-way valve. Flow in one direction may cause the leaflet(s) to deflect open and flow in the opposite direction may cause the leaflet(s) to close.

One of ordinary skill in the art will recognize based on the description herein that any of the valve prostheses described herein may comprise any of the frame structure shapes, frame structure designs, frame structure materials, anchor shapes, anchor windings, anchor materials, free end tips, leaflet(s) configurations, or any other of the variable features described herein in any combination thereof as desired.

### Method of use

The distal end of the delivery device **30**m may be configured to be advanced from a first side of a native valve to a second side of the native valve. For example, the distal end of the delivery device **30**m may be advanced from a left atrial side of a mitral valve to a left ventricular side of a mitral valve. In some instances, the distal end of the delivery device **30**m may be transseptally inserted into the left atrium of the heart prior to advancement into the left ventricle. Alternatively, or in combination, the distal end of the delivery device **30**m may be steerable such that it is positionable to point towards the first side of the native valve before being advanced to the second side of the native valve.

After advancing to the second side of the native valve, the anchor **15**m may be fully deployed on the second side of the native valve. Fully deploying the anchor **15**m may comprise actuating the anchor **15**m from an elongated configuration to a deployed configuration as shown in FIG. 51.

In some embodiments, fully deploying the anchor **15**m may comprise actuating the anchor **15**m from an elongated configuration to a deployed configuration on the first side of the native valve (e.g., in the left atrium) and advancing the anchor **15**m, in the deployed configuration, through the native valve to the second side of the native valve (e.g., into the left ventricle). Advancing the anchor **15**m may comprise pushing the anchor **15**m through the native valve as described herein. Advancing the anchor **15**m may further comprise rotating the anchor **15**m through the native valve.

In some embodiments, fully deploying the anchor **15**m may comprise positioning the anchor **15**m such that it is located only on the second side of the native valve.

In some embodiments, the anchor **15**m may be actuated from the delivery configuration to the deployed configuration on a first side of the native valve prior to being advanced to a second side of the native valve. For example, the anchor **15**m may be fully deployed in a left atrium of a heart prior to being advanced to a left ventricle of the heart as described herein.

Alternatively, the anchor **15**m may be actuated from the delivery configuration to the deployed configuration on a second side of the native valve after being advanced to the second side from a first side of the native valve. For example, anchor **15**m may be advanced from a left atrium of a heart prior to being deployed in a left ventricle of the heart.

The free end **22**m of the deployed anchor **15**m may optionally be rotated around one or more structures on the second side of the native valve. The one or more structures may comprise one or more valve leaflets of the native valve. Alternatively, or in combination, the one or more structures may comprise one or more chordae of the left ventricle.

The free end **22**m of the deployed anchor **15**m may optionally rotated around one or more structures on the second side of the native valve such that the one or more structures (e.g., chordae, leaflets, or annulus) are pulled radially inwards towards the longitudinal axis of the anchor **15**m and/or towards the longitudinal axis of the delivery device **30**m. The anchor **15**m and/or free end **22**m may be configured such that minimal torque is applied to the one or more structures. Alternatively, or in combination, the anchor **15**m and/or free end **22**m may be configured such that the one or more structures are not rotated, or are minimally rotated, during rotation of the anchor 15m.

In some embodiments, the valve prosthesis **10**m, for example anchor **15**m, may be counter-rotated in order to reposition the anchor **15**m with respect to the one or more structures of the native valve before continuing the rotation in the first direction. For example, counter-rotation may be applied if the one or more structures are caught by the free end of the anchor **15**m (or another part of the valve prosthesis **10**m or delivery device **30**m) during the initial rotation. In such instances, counter-rotation may enable to the clinician to disengage some or all of the one or more structures to reduce the stress or torque on the one or more structures (e.g., by adjusting the position of the valve prosthesis **10**m) before resuming rotation. Rotation and counter-rotation may be applied as many times as desired by the clinician in order to properly position the anchor **15**g around the one or more structures of the native valve.

The anchor **15**m may then be released from the distal end of the delivery device **30**m. The anchor **15**m may be released from the distal end of the delivery device **30**m on the second side of the native valve.

The frame structure **12**m may be expanded within the native valve from an unexpanded configuration to an expanded configuration.

The frame structure **12**m may be released from the distal end of the delivery device **30**m. In some embodiments, at least a portion the frame structure **12**m may be expanded within at least a portion of the deployed anchor to anchor **15**m the frame structure **12**m to the native valve.

In some embodiments, expanding the frame structure and releasing the frame structure may occur simultaneously.

Finally, the delivery device **30**m may be retracted from the native valve.

Additional information about the frame structure may be found in U.S. Provisional Applications No. 62/720,853, 62/742,043, 62/748,162, 62/755,996, 62/784,280, 62/813,963, 62/815,791, 62/820,570, 62/828,835, 62/833,425, 62/833,430, 62/851,245, 62/872,016, 62/873,454, 62/879,979, 62/894,565, 62/925,505, 62/927,922, 62/933,122, 62/951,260, U.S. Patent Applications No. 16/546,901, 16/594,946, and International Patent Applications No. PCT/US2019/047542, PCT/US2019/055049, and PCT/US2019/057082.

Additional information about the anchor may be found in U.S. Provisional Applications No. 62/720,853, 62/742,043, 62/748,162, 62/755,996, 62/784,280, 62/813,963, 62/815,791, 62/820,570, 62/828,835, 62/833,425, 62/833,430, 62/851,245, 62/872,016, 62/873,454, 62/879,979, 62/894,565, 62/925,505, 62/927,922, 62/933,122, 62/951,260, U.S. Patent Applications No. 16/546,901, 16/594,946, and International Patent Applications No. PCT/US2019/047542, PCT/US2019/055049, and PCT/US2019/057082.

Additional information about the delivery device may be found in U.S. Provisional Applications No. 62/720,853, 62/742,043, 62/748,162, 62/755,996, 62/784,280, 62/813,963, 62/815,791, 62/820,570, 62/828,835, 62/833,425, 62/833,430, 62/851,245, 62/872,016, 62/873,454, 62/879,979, 62/894,565, 62/925,505, 62/927,922, 62/933,122, 62/951,260, U.S. Patent Applications No. 16/546,901, 16/594,946, and International Patent Applications No. PCT/US2019/047542, PCT/US2019/055049, and PCT/US2019/057082.

The valve prosthesis may be substantially similar to any of the valve prostheses described in U.S. Provisional Applications No. 62/720,853, 62/742,043, 62/748,162, 62/755,996, 62/784,280, 62/813,963, 62/815,791, 62/820,570, 62/828,835, 62/833,425, 62/833,430, 62/851,245, 62/872,016, 62/873,454, 62/879,979, 62/894,565, 62/925,505, 62/927,922, 62/933,122, 62/951,260, U.S. Patent Applications No. 16/546,901, 16/594,946, and International Patent Applications No. PCT/US2019/047542, PCT/US2019/055049, and PCT/ US2019/057082.

FIGS. 52-54 show several views of a valve prosthesis **10**n with an anchor comprising a flat spiral shaped anchor. The valve prosthesis **10**n may be substantially similar to any of the valve prostheses described herein except that the anchor is configured to have a flat spiral shape. For example, the anchor may comprise a spiral band. The frame structure **12**n has an unexpanded configuration (for example, a compressed configuration as described herein) and an expanded configuration as described herein. The frame structure **12**n may be substantially similar to any of the frame structures described herein or understood by one of ordinary skill in the art from the description herein. The anchor **15**n may comprise a spiral band **17**. The anchor **15**n is configured to anchor the frame structure **12**n to the native valve when the frame structure **12**n is in the expanded configuration adjacent the native valve. The frame structure **12**n is configured to be actuated from the unexpanded configuration to the expanded configuration adjacent a native valve in a patient. The anchor **15**n may be configured to be fully advanced from a first side of a native valve in a patient (e.g. an atrial side) to a second side of the native valve (e.g. into a ventricle of the heart) and anchor the frame structure **12**n to the native valve when the frame structure **12**n is in the expanded configuration adjacent the native valve as described herein.

The flat spiral anchor **15**n comprises a free end **22**n. The other end of the anchor **15**n may be coupled to the top (proximal end) or bottom (distal end) of the frame structure **12**n as described herein. Alternatively, or in combination, the other end of the anchor **15**n may not be attached to the frame structure **12**n as described herein. The free end **22**n of the anchor **15**n may facilitate capturing of the valve leaflets and/or chordae tendineae within the sweep of the free end during rotation as described herein. During rotation of the anchor **15**n, the leaflets and/or chordae tendineae may be captured by the free end **22**n and trapped between the valve frame structure **12**n and an interior surface of the anchor **15**n.

The free end **22**n of the flat spiral anchor **15**n may be sized and dimensioned for insertion through the native valve, for example through tissue at or near a commissure of the native valve or through the valve opening itself. In some embodiments, the free end **22**n may comprise an atraumatic tip to avoid reduce risk of injury to the native valve tissue and leaflets. For example, the free end **22**n may comprise a blunt end, a ball tip, a curved tip (e.g. J-tip or pigtail), or other atraumatic shapes. Alternatively, the free end **22**n may be configured for piercing tissue.

The free end **22**n of the flat spiral anchor **15**n may optionally rotated around one or more structures on the second side of the native valve such that the one or more structures (e.g., chordae, leaflets, or annulus) are pulled radially inwards towards the longitudinal axis of the anchor **15**n and/or towards the longitudinal axis of the delivery device **30**n. The anchor **15**n and/or free end **22**n may be configured such that minimal torque is applied to the one or more structures. Alternatively, or in combination, the anchor **15**n and/or free end **22**n may be configured such that the one or more structures are not rotated, or are minimally rotated, during rotation of the anchor **15**n. For example, the anchor **15**n may comprise one or more spaces between loops of the spiral band (for example, spaces **18** shown in FIG. 57) which facilitate movement of the captured tissue (e.g. chordae and/or leaflets) from the free end **22**n to the center of the spiral structure with little or no torque and/or rotation of the structures during rotation of the anchor **15**n as described herein. Alternatively or in combination, the anchor **15**n may be configured such that, when fully deployed (for example, as shown in FIG. 59), none of the structures reside between the loops of the spiral. Instead, the one or more structures may sit radially inward of the loops in order to facilitate capture of the one or more structures between the anchor **15**n and the expanded frame structure **12**n. The one or more structures may retain or nearly retain their normal anatomical position when the anchor **15**n is fully deployed.

The anchor **15**n may comprise a delivery (e.g. an elongated) configuration and a deployed configuration. The anchor **15**n may be configured to be actuated from the delivery configuration to the deployed configuration adjacent a native valve in a patient. In various embodiments, the anchor **15**n may have a generally spiral shape in the deployed configuration. In various embodiments, the anchor **15**n may be elongated-rather than spiral-shaped-in the delivery configuration. For example, the anchor **15**n may be elongated into a straight shape within the delivery device. In various embodiments, a portion of the anchor **15**n may have a spiral shape. In various embodiments, a substantial portion of the anchor **15**n may have a spiral shape. In embodiments of the invention, the spiral anchor **15**n is formed as a flat spiral (in the deployed configuration) whereby the loops generally are positioned within the same plane (the plane being perpendicular to a longitudinal axis).

Anchor **15**n may be formed of a material having sufficient rigidity to hold a predetermined shape. The wire may, for example, be formed of a shape memory material (e.g. NiTi). It may be desirable for at least an end portion (e.g. free end **22**n) to be relatively rigid such that it can exert a force to move chordae tendineae, while still retaining flexibility to be collapsed within a delivery device. In various embodiments, the end portion only needs sufficient rigidity to hold its shape and will deform under a load. For example, the end portion may be configured with a similar rigidity to a guidewire, or slightly stiffer.

The anchor **15**n may comprise a planar spiral band **17.** The anchor **15**n may comprise a spiral band **17** having a free end **22**n. The other end of the spiral band **17** may be coupled to the top (proximal end) or bottom (distal end) of the frame structure **12**n as described herein. Alternatively, or in combination, the other end of the spiral band **17** may not be attached to the frame structure **12**n as described herein. The free end **22**n of the spiral band **17** may facilitate capturing of the valve leaflets and/or chordae tendineae within the sweep of the free end during rotation as described herein. During rotation of the spiral band **17,** the leaflets and/or chordae tendineae may be captured by the free end **22**n and trapped between the valve frame structure 12n and an interior surface of the spiral band **17.**

The free end **22**n of the spiral band **17** may be sized and dimensioned for insertion through the native valve, for example through tissue at or near a commissure of the native valve or through the valve opening itself. In some embodiments, the free end **22**n may comprise an atraumatic tip to avoid reduce risk of injury to the native valve tissue and leaflets. For example, the free end **22**n may comprise a blunt end, a ball tip, a curved tip (e.g. J-tip or pigtail), or other atraumatic shapes. Alternatively, the free end **22**n may be configured for piercing tissue. In various embodiments, the free end **22**n is separate from and extends outward from the main coils of the anchor **15**n. In various embodiments, the main body coils of the anchor **15**n circumscribe an area (in the case of a spiral coil) or a volume (in the case of a helical coil) having a diameter, and the free end **22**n extends to a radius greater than the diameter of the anchor **15**n. In various embodiments, the free end **22**n extends to a radius substantially greater than the diameter of the anchor **15**n. In various embodiments, the free end **22**n is configured to circumscribe a larger diameter than the anchor **15**n. In various embodiments, the free end **22**n is configured to circumscribe all of the chordae tendineae of the native valve to be treated.

The free end **22**n of the spiral band **17** may optionally rotated around one or more structures on the second side of the native valve such that the one or more structures (e.g., chordae, leaflets, or annulus) are pulled radially inwards towards the longitudinal axis of the anchor **15**n and/or towards the longitudinal axis of the delivery device **30**n. The spiral band **17** and/or free end **22**n may be configured such that minimal torque is applied to the one or more structures. Alternatively, or in combination, the spiral band **17** and/or free end **22**n may be configured such that the one or more structures are not rotated, or are minimally rotated, during rotation of the spiral band **17.** For example, the spiral band **17** may comprise one or more spaces between loops of the spiral band (for example, spaces **18** shown in FIG. 57) which facilitate movement of the captured tissue (e.g. chordae and/or leaflets) from the free end **22**n to the center of the spiral structure with little or no torque and/or rotation of the structures during rotation of the spiral band **17** as described herein. Alternatively or in combination, the spiral band **17** may be configured such that, when fully deployed (for example, as shown in FIG. 59), none of the structures reside between the loops of the spiral. Instead, the one or more structures may sit radially inward of the loops in order to facilitate capture of the one or more structures between the spiral band **17** and the expanded frame structure **12**n. The one or more structures may retain or nearly retain their normal anatomical position when the spiral band **17** is fully deployed.

The spiral band **17** may comprise a delivery (e.g. an elongated) configuration and a deployed configuration. The spiral band **17** may be configured to be actuated from the delivery configuration to the deployed configuration adjacent a native valve in a patient. In various embodiments, the band **17** may have a generally spiral shape in the deployed configuration. In various embodiments, the band **17** may be elongated-rather than spiral-shaped-in the delivery configuration. For example, the band **17** may be elongated into a straight shape within the delivery device. In various embodiments, a portion of the band **17** may have a spiral shape. In various embodiments, a substantial portion of the band **17** may have a spiral shape. In embodiments of the invention, the spiral band **17** is formed as a flat spiral (in the deployed configuration) whereby the loops generally are positioned within the same plane (the plane being perpendicular to a longitudinal axis).

Spiral band **17** may be formed of a material having sufficient rigidity to hold a predetermined shape. The wire may, for example, be formed of a shape memory material (e.g. NiTi). It may be desirable for at least an end portion (e.g. free end **22**n) to be relatively rigid such that it can exert a force to move chordae tendineae, while still retaining flexibility to be collapsed within a delivery device. In various embodiments, the end portion only needs sufficient rigidity to hold its shape and will deform under a load. For example, the end portion may be configured with a similar rigidity to a guidewire, or slightly stiffer.

FIGS. 55-57 show several views of the flat spiral anchor **15**n of FIGS. 52-54 loaded on a delivery device **30**n. The delivery device **30**n may be substantially similar to any of the delivery devices described herein. For example, the delivery device **30**n may comprise an outer sheath in which the anchor **15**n may be disposed, for example in an elongated or undeployed delivery configuration as described herein. Alternatively, or in combination, the delivery device **30**n may comprise an inner shaft, for example disposed within the lumen of the outer sheath. The anchor **15**n and/or frame structure **12**n may be coupled to the inner shaft as described herein. The delivery device **30**n may comprise a guidewire as described herein. The delivery device **30**n may be configured to deliver the anchor **15**n to the native valve as described herein. The manner of implanting valve device **10**n may be substantially similar to any of the methods described herein.

The flat spiral anchor **15**n (e.g., spiral band **17**) may comprise one or more loops. For example, the anchor **15**n may comprise a plurality of loops, which may increase the radial strength of the anchor by increasing friction and addition structural support. The one or more loops of the anchor **15**n may be spiral radially outward from a central point or central axis of the spiral, for example along an axis which is coaxial with a longitudinal axis of a delivery device **30**n such that the spiral anchor **15**n lies approximately along a plane perpendicular to the longitudinal axis of the delivery device **30**n.

The one or more loops (also referred to herein as coils or turns) of the flat spiral anchor **15**n (e.g., spiral band **17**) may comprise a shape that bends around back towards its origin (for example, an arc, ellipsoid, circle, or the like). In some embodiments, a loop may comprise a shape that bends back towards its origin but does not cross itself, making a rotation within a range of about 180 degrees to about 360 degrees. For example, a loop may comprise an arc having a central angle within a range of about 180 degrees to about 360 degrees. In at least some embodiments, the one or more loops may comprise an arc. In some embodiments, a loop may comprise a shape that bends back towards and crosses itself, making at least a 360 degree rotation. In at least some embodiments, the one or more loops may comprise a 360 degree rotation (for example, a circle). In some embodiments, the one or more loops may comprise a 360 degree to 720 degree rotation (for example, a loop crossing itself once and rotating further towards a second crossing and formation of a second 360 loop).

The one or more loops may comprise any number of loops desired, for example, one, two, three, four, five, six, seven, eight, nine, or ten loops. The one or more loops may comprise a rotation within a range of about 180 degrees to about 3600 degrees. The one or more loops may comprise a rotation within a range bounded by any of the following values: 180 degrees, 270 degrees, 360 degrees, 450 degrees, 540 degrees, 630 degrees, 720 degrees, 810 degrees, 900 degrees, 990 degrees, 1080 degrees, 1170 degrees, 1260 degrees, 1350 degrees, 1440 degrees, 1530 degrees, 1620 degrees, 1710 degrees, 1800 degrees, 1890 degrees, 1980 degrees, 2070 degrees, 2160 degrees, 2250 degrees, 2340 degrees, 2430 degrees, 2520 degrees, 2610 degrees, 2700 degrees, 2790 degrees, 2880 degrees, 2970 degrees, 3060 degrees, 3150 degrees, 3240 degrees, 3330 degrees, 3420 degrees, 3510 degrees, or 3600 degrees.

**In** some embodiments, for example when the free end 22 is disposed at an angle (upward, downward, or radially outward) relative to the rest of the anchor body, the main body of the anchor comprises the one or more loops and the rotation of the one or more loops may be determined without taking into account the angled free end 22.

Interaction of the frame structure **12**n with the one or more loops of the anchor **15**n may create opposing forces therebetween that provide mechanical leverage for anchoring the frame structure to the one or more anatomical structures. In some embodiments, the one or more loops may comprise at least 360 degrees of rotation (e.g., 540 degrees) when deployed such that the loops wrap around one another and provide additional mechanical leverage against the frame structure in order to facilitate anchoring of the frame structure as described herein. Additional loops or partial loops may provide additional mechanical strength and/or leverage.

In some embodiments, the one or more loops may comprise a rotation of about 540 degrees. A first loop may comprise a rotation of 360 degrees and a second loop may comprise a rotation of about 180 degrees. The second loop may wrap around the first loop for about 180 degrees in order to provide additional mechanical strength and/or leverage against the frame structure in order to facilitate anchor of the frame structure. In some instances, the one or more loops comprising a rotation of about 540 degrees may facilitate easier and safer capture of the chordae tendineae and/or valve leaflets than an anchor having additional loops by providing sufficient mechanical strength and/or leverage and/or radial stability while reducing or minimizing the amount of overlap between loops in which to potentially ensnare or entangle the native valve structures during rotation.

In some embodiments, the one or more loops of the anchor **15**n may comprise one or more spaces **18** therebetween. The spaces **18** may facilitate movement of the captured tissue (e.g. chordae and/or leaflets) from the free end **22**n to the center of the spiral structure during rotation of the anchor **15**n as described herein.

The anchor **15**n (e.g., spiral band **17**) may comprise a spiral wire. The anchor **15**n may comprise a plurality of spiral wires as described herein.

In some embodiments, the delivery device **30**n may be configured to carry the anchor **15**n in an undeployed configuration and deploy the anchor **15**n into a deployed configuration as the desired location as described herein.

In some embodiments, the anchor **15**n may be configured to wrap at least partially around a distal portion of the delivery device **30**n, for example around the inner shaft, in the deployed configuration.

In some embodiments, the anchor **15**n may comprise one or more locking mechanisms configured to maintain the anchor **15**n in the deployed configuration. The one or more locking mechanisms may be any of the locking mechanisms described herein or understood by one of ordinary skill in the art from the description herein. In various embodiments, one or more loops may be nested with each other when the anchor is in the deployed configuration.

FIGS. 58-59 show deployment of the anchor of FIGS. 52-54 from an undeployed configuration (FIG. 58) to a deployed configuration (FIG. 59). The anchor **15**n may comprise an undeployed configuration (shown in FIG. 58) and a deployed configuration (shown in FIG. 59). The anchor **15**n may be configured to be actuated from the undeployed configuration to the deployed configuration adjacent the native valve. The anchor **15**n may comprise a spiral band **17.** The anchor **15**n may comprise a flat spiral or planar spiral shape in the deployed configuration. The free end **22**n of the anchor **15**n may extend radially outward from the frame **12**n, and in particular from the remainder of the anchor **15**n. In some embodiments, expansion of the frame structure **12**n inside the anchor **15**n may cause the anchor **15**n to expand from the undeployed configuration to the deployed configuration. Alternatively, or in combination, rotation of the anchor **15**n around one or more structures as described herein may cause the anchor **15**n to expand from the undeployed configuration to the deployed configuration.

In some embodiments, the anchor **15**n may comprise a delivery (e.g., elongated) configuration when disposed within a delivery device as described herein. The delivery device may be configured to deploy the anchor **15**n from the elongated configuration to a first intermediate deployed configuration (substantially similar to the undeployed configuration shown in FIG. 58) as described herein. The anchor **15**n may then be actuated into the second fully deployed configuration (substantially similar to the deployed configuration shown in FIG. 59), for example by expanding the frame structure within the anchor **15**n or by rotating the anchor **15**n to capture the one or more structures as described herein.

The anchor **15**n may comprise one or more loops of a spiral as described herein. The anchor 15n may comprise a more compact spiral, with more loops, in the undeployed configuration compared to the deployed configuration. As the anchor **15**n expands the spiral loops may unwind.

In some embodiments, the outer perimeter of the anchor **15**n (e.g., spiral band **17**) may be substantially similar in the undeployed and deployed configurations. As the spiral loops unwind, the spiral may expand from the center, for example simultaneously with expansion of the frame structure in the center of spiral anchor **15**n, such that the spiral loops are expanded against one another as shown in FIG. 59. As will be understood by one of ordinary skill in the art, the shape, size, and number of loops may determine the limits of expansion of the spiral anchor **15**n and alteration of any or all of these parameters may be done to generate a final deployed configuration desired from an initial undeployed configuration.

FIGS. 60-61 show an optional locking mechanism for a spiral band anchor. Anchor **15**n may comprise a spiral band **17.** The anchor **15**n may comprise one or more optional locking features disposed along the spiral loops such that they interlock when expanded against one another. For example, the locking features may comprise one or more "key-in-hole" (e.g., key and key hole) interlocking features **19** as shown which lock the spiral anchor **15**n in a deployed configuration when the spiral loops are expanded such that the two corresponding features meet and connect. In some instances, it may be beneficial to provide the spiral anchor **15**n with a plurality of locking features, for example to ensure secure locking and/or to provide for a plurality of intermediate expanded/deployed configurations between the fully undeployed and the fully deployed configuration. It will be understood by one of ordinary skill in that any number or style of locking features may be used to lock the spiral anchor **15**n into a deployed (and/or intermediately deployed) configuration.

FIG. 62 shows another optional locking mechanism for a spiral band anchor. Anchor **15**n may comprise a spiral band **17.** The anchor **15**n may comprise an optional frictional band **21** disposed upon a length of the anchor **15**n which may facilitate locking the anchor **15**n in a deployed configuration as the spiral loops expand. By adding a frictional texture or band **21** to at least a portion of the anchor **15**n, the loops may be encouraged to engage one another as they are expanded against each other.

The frictional band **21** may, for example comprise a strip of frictional material that may be bonded to the surface of the anchor **15**n by any method understood by one of ordinary skill in the art from the description herein.

Alternatively, or in combination, at least a portion of the anchor **15**n may be coated with a polymer and the outer surface of the polymer may be made rough using any technique understood by one of ordinary skill in the art from the description herein.

Alternatively, or in combination, the surface of the anchor **15**n may be directly altered for increased friction to form the frictional band **21** using any technique understood by one of ordinary skill in the art from the description herein.

FIGS. 63-64 show an optional tip orientation for a spiral anchor. In some embodiments, the spiral anchor 15n, which may comprise a spiral band **17,** may comprise a free end **22**n which is angled or bent such that it points upwards towards the proximal direction of the delivery device 30n. FIGS. 65-66 show another optional tip orientation for a spiral anchor, which may comprise a spiral band **17.** In some embodiments, the spiral anchor **15**n may comprise a free end **22**n which is angled or bent such that it points downwards towards the distal direction of the delivery device **30**n. Angling the free end **22**n distally or proximally, or otherwise disposing the free end **22**n away from the rest of the anchor **15**n (for example radially outward therefrom), may aid in deployment of the anchor **15**n from the delivery device and/or capture of the one or more structures (e.g., chordae tendineae, leaflets, etc.).

FIGS. 67-69 show various optional configurations for a spiral anchor. FIG. 67 shows an anchor **15**n comprising a spiral band **17**a having a hollow, tubular cross-section. The spiral anchor **15**n may, for example, comprise a hypotube. The anchor **15**n may be configured to pass another component (e.g. a wire, guidewire, etc.) therethrough. FIG. 68 shows a spiral band 17b which may comprise a solid, circular cross-section. The spiral band 17b may, for example, comprise a wire. FIG. 69 shows a spiral band 17c with a non-round, elongated cross-section comprising a plurality of channels disposed therein. Four channels are shown but it will be understood to one of ordinary skill in the art that any number of channels may be utilized as desired. The channels may for example be left as open lumens. Alternatively, or in combination, the channels may be filled, for example with one or more stiffening members. For example, at least one channel may be open and at least one channel may be filled.

The spiral anchor **15**n may have a cross-section of any shape desired, for example a circular, tubular (e.g. hollow), square, elongated, triangular, or any other shaped cross-section. The spiral anchor **15**n may comprise a solid anchor, or the spiral anchor may comprise one, two, three, four, five, or more lumens or channels disposed therein. The lumens or channels **71** may be open lumens and/or filled channels, for example with one or more stiffening members, guidewires, or the like. It will be understood by one of ordinary skill in the art that the spiral anchor may have any configuration, or combination of configurations, described herein or understood by one of ordinary skill in the art from the description herein.

For example, the one or more lumens or channels **71** may be configured to hold one or more stiffening members in order to provide structural support to the anchor **15**n. Alternatively, or in combination, the anchor 15n may be delivered to the heart over a guidewire(s) translatably and removably disposed within one or more of the lumens of channels **71.**

The spiral band **17** may have a cross-section of any shape desired, for example a circular, tubular (e.g. hollow), square, elongated, triangular, or any other shaped cross-section. The spiral band may comprise a solid band, or the spiral band may comprise one, two, three, four, five, or more lumens or channels disposed therein. The lumens or channels **71** may be open lumens and/or filled channels, for example with one or more stiffening members, guidewires, or the like. It will be understood by one of ordinary skill in the art that the spiral band may have any configuration, or combination of configurations, described herein or understood by one of ordinary skill in the art from the description herein.

FIGS. 70-72 show several views of an anchor **15**o comprising a spiral band **17.** The spiral band **17** may be a tapered spiral band **17**d. Tapered spiral band **17**d may be deployed and configured to anchor a frame structure of a valve prosthesis adjacent a native valve as described herein. The anchor **15**o may, for example, be configured to be fully advanced from a first side of a native valve in a patient (e.g. an atrial side) to a second side of the native valve (e.g. into a ventricle of the heart) and anchor a frame structure to the native valve when the frame structure is in an expanded configuration adjacent the native valve as described herein. The tapered spiral band **17**d may comprise a delivery (e.g., elongated) configuration and a deployed configuration (and optional intermediate configurations) substantially similar to those shown in FIGS. 52-69. The tapered spiral band **17**d may be configured to be actuated from the elongated configuration to the deployed configuration adjacent a native valve in a patient. The tapered spiral band **17**d may be delivered to the native valve by a delivery device as described herein. The tapered spiral band **17**d may be deployed adjacent the native valve substantially similarly to other anchor embodiments as described herein. In some embodiments, the anchor **15**o may comprise one or more locking mechanisms configured to maintain the anchor **15**o in the deployed configuration. The one or more locking mechanisms may be any of the locking mechanisms described herein or understood by one of ordinary skill in the art from the description herein.

The anchor **15**o may comprise a tapered spiral band **17**d having a free end **22**o. The other end of the tapered spiral band **17**d may be coupled to the top (proximal end) or bottom (distal end) of a frame structure as described herein. Alternatively, or in combination, the other end of the tapered spiral band **17**d may not be attached to a frame structure as described herein. The free end **22**o of the tapered spiral band **17**d may facilitate capturing of the valve leaflets and/or chordal tendineae within the sweep of the free end **22**o during rotation as described herein. During rotation of the tapered spiral band **17**d, the leaflets and/or chordae tendineae may be captured by the free end **22**o and trapped between the valve frame structure and an interior surface of the spiral band **17**d.

The free end **22**o of the spiral band **17**d may be sized and dimensioned for insertion through the native valve, for example through tissue at or near a commissure of the native valve or through the valve opening itself. In some embodiments, the free end **22**o may comprise an atraumatic tip to avoid reduce risk of injury to the native valve tissue and leaflets. For example, the free end may comprise a blunt end, a ball tip, a curved tip (e.g. J-tip or pigtail), or other atraumatic shapes. Alternatively, the free end **22**o may be configured for piercing tissue. In various embodiments, the free end **22**o is separate from and extends outward from the main coils of the anchor **15**o. In various embodiments, the main body coils of the anchor **15**o circumscribe an area (in the case of a spiral coil) or a volume (in the case of a helical coil) having a diameter, and the free end **22**o extends to a radius greater than the diameter of the anchor **15**o. In various embodiments, the free end **22**o extends to a radius substantially greater than the diameter of the anchor **15**o. In various embodiments, the free end **22**o is configured to circumscribe a larger diameter than the anchor **15**o. In various embodiments, the free end **22**o is configured to circumscribe all of the chordae tendineae of the native valve to be treated.

The free end **22**o of the tapered spiral band **17**d may optionally be rotated around one or more structures on the second side of the native valve such that the one or more structures (e.g., chordae, leaflets, or annulus) are pulled radially inwards towards the longitudinal axis of the anchor **15**o and/or towards the longitudinal axis of the delivery device **30.** The spiral band **17**d and/or free end **22**o may be configured such that minimal torque is applied to the one or more structures. Alternatively, or in combination, the tapered spiral band **17**d and/or free end **22**o may be configured such that the one or more structures are not rotated, or are minimally rotated, during rotation of the spiral band **17**d. For example, the spiral band **17**d may comprise one or more spaces between loops of the spiral band (for example, spaces **18** shown in FIG. 57) which facilitate movement of the captured tissue (e.g. chordae and/or leaflets) from the free end **22**o to the center of the spiral structure with little or no torque and/or rotation of the structures during rotation of the tapered spiral band **17**d as described herein. Alternatively or in combination, the spiral band **17**d may be configured such that, when fully deployed (for example, as shown in FIG. 72), none of the structures reside between the loops of the spiral. Instead, the one or more structures may sit radially inward of the loops in order to facilitate capture of the one or more structures between the spiral band **17**d and the expanded frame structure as described herein. The one or more structures may retain or nearly retain their normal anatomical position when the spiral band **17**d is fully deployed.

Spiral band **17**d may be configured to taper in height axially. For example, spiral band **17**d may be configured to taper in height from a first end of the spiral band **17**d, which may be coupled to a delivery device and/or frame structure as described herein, to a free end **22**o of the spiral band **17**d. In some embodiments, the spiral band **17**d may taper in height from a proximal end to a distal end. Alternatively, the spiral band **17**d may taper in height from a distal end to a proximal end.

The spiral band **17**d may be tapered such that subsequent turns of the tapered spiral band **17**d nest into each other so as to reduce a radial footprint of the tapered spiral band.

The spiral band **17**d may, for example, comprise a solid wire-like spiral band similar to that shown in FIG. 64 but with a taper as described herein. In some embodiments, the spiral band **17**d may comprise a plurality of spiral support wires.

Alternatively, or in combination, at least a portion of the tapered spiral band may comprise a support structure **70** and a band material **72** disposed therein (or thereon as desired by one of ordinary skill in the art).

The support structure **70** may, for example, comprise a wire. The support structure **70** may be formed of a material having sufficient rigidity to hold a predetermined shape. The support structure **70** may, for example, be formed of a shape memory material (e.g. NiTi). It may be desirable for at least an end portion (e.g. free end **22**o) to be relatively rigid such that it can exert a force to move chordal tendineae, while still retaining flexibility to be collapsed within a delivery device. In various embodiments, the end portion only needs sufficient rigidity to hold its shape and will deform under a load. For example, the end portion may be configured with a similar rigidity to a guidewire, or slightly stiffer.

The band material **72** may comprise a permeable, semi-permeable, or impermeable material. The band material **72** may be flexible, semi-flexible, or rigid. In some embodiments, the band material **72** may be relatively soft so as to reduce the risk of injury to the one or more structures during rotation of the tapered spiral band **17**d. The band material **72** may, for example, comprise a webbing material, a fabric, a polymeric material, an elastomeric material, or the like. The band material **72** may span the structural support **70** so as to reduce leakage therethrough. Alternatively, or in combination, the band material **72** may be configured to improve alignment of the support structure **70** wire.

It will be understood by one of ordinary skill in the art from the description herein that any of the anchors **15** described herein may comprise a support structure and a material disposed therein or thereon as described with referenced to tapered spiral band **17**d.

The tapered spiral band **17**d may comprise one or more loops. For example, the spiral band **17**d may comprise a plurality of loops, which may increase the radial strength of the anchor by increasing friction and addition structural support. The one or more loops of the spiral band **17**d may be spiral radially outward from a central point or central axis of the spiral, for example along an axis which is coaxial with a longitudinal axis of a delivery device 30n such that the spiral band **17**d lies approximately along a plane perpendicular to the longitudinal axis of a delivery device.

In some embodiments, the one or more loops of the spiral band **17**d may comprise one or more spaces therebetween, which may be substantially similar to spaces **18** shown in FIG. 57. The spaces may facilitate movement of the captured tissue (e.g. chordae and/or leaflets) from the free end **22**o to the center of the spiral structure during rotation of the anchor **15**o as described herein.

In some embodiments, the support structure 70 may comprise one or more channels or lumens 71 disposed therein. The support structure 70 may comprise a hollow, tubular cross-section. The support structure 70 may, for example, comprise a hypotube. The lumen of the support structure 70 may be configured to pass another component (e.g. a wire, guidewire, etc.) therethrough.

FIGS. 73-76 show several views of another anchor **15**o comprising a tapered spiral band **17**e. FIGS. 73, 75, and 76 show the anchor **15**o in a deployed configuration. FIG. 74 shows the anchor **15**o in an elongated delivery configuration. The spiral band **17**e may be substantially similar to tapered spiral band **17**d shown in FIGS. 70-72 except that the support structure **70** may comprise more than one wire. The support structure **70** may, for example, comprise an upper wire **70**a and a lower wire **70**b. The wires **70**a, **70**b may be coupled to one another at a proximal attachment point **73**a and/or a distal attachment point **73**b. The wires **70**a, **70**b may be round wires or have other cross-sectional shapes. In various embodiments, the support structure **70** may comprise a scaffold such as a laser-etched Nitinol scaffold or a mesh. The spiral band **17**e may be tapered such that subsequent turns of the tapered spiral band **17**e nest into each other so as to reduce a radial footprint of the tapered spiral band. Wires **70**a, **70**b may be configured to nest with one another when the in the deployed configuration.

The tapered spiral band **17**e may comprise a delivery (e.g., elongated) configuration and a deployed configuration (and optional intermediate configurations) as described herein. The tapered spiral band **17**e may be configured to be actuated from the elongated configuration to the deployed configuration adjacent a native valve in a patient. The tapered spiral band **17**e may be delivered to the native valve by a delivery device in the elongated configuration as described herein. The tapered spiral band **17**e may be coupled to the delivery device and/or a frame structure of a valve prosthesis as described herein, for example at a proximal portion (*e.g.* adjacent proximal attachment point **73**a on an interior of the spiral) or distal portion (*e.g*. adjacent distal attachment point **73**b on an exterior of the spiral) thereof. The tapered spiral band **17**e may be deployed adjacent the native valve substantially similarly to other anchor embodiments as described herein.

In some embodiments, the upper wire **70**a and lower wire **70**b may be bundled together during deployment from an undeployed configuration to an intermediate deployed configuration. The intermediate configuration may be configured to reduce the size the size of the lumen and/or aperture in or through which, respectively, the spiral anchor **15**o may travel (for example, a lumen or aperture of the delivery device) during deployment. The anchor **15**o may be maintained in the intermediate configuration during rotation around the one or more structures as described herein. In at least some instances, coupling the wires **70**a, **70**b together into an intermediate configuration may facilitate alignment of the wires making up the support structure **70.** After delivery from the delivery device and/or rotation around one or more structures, the anchor **15**o may be fully deployed into the deployed configuration by releasing the bundle and allowing the wires **70**a, **70**b to "spring out" into the deployed configuration.

The support structure **70** may have a band material **72** disposed therein or thereon as described herein. The band material **72** may span the distance between wires **70**a, **70**b in order to couple the wires **70**a, **70**b to one another. The band material **72** may span the structural support **70** so as to reduce leakage therethrough as described herein. Alternatively, or in combination, the band material **72** may be configured to improve alignment of the support structure **70** wires **70**a, **70**b such that they maintain a desired relative position to one another.

The anchor **15**o may comprise a free end **22**o. The free end **22**o may be substantially similar to any of free ends described herein. The other end of the tapered spiral band **17**e may be coupled to the top (proximal end) or bottom (distal end) of a frame structure as described herein. Alternatively, or in combination, the other end of the tapered spiral band **17**e may not be attached to a frame structure as described herein. The free end **22**o of the tapered spiral band **17**e may facilitate capturing of the valve leaflets and/or chordal tendineae within the sweep of the free end **22**o during rotation as described herein. During rotation of the tapered spiral band **17**e, the leaflets and/or chordae tendineae may be captured by the free end **22**o and trapped between the valve frame structure and an interior surface of the spiral band **17**e against the support structure **70** and/or band material **72.**

The free end **22**o of the spiral band **17**e may be sized and dimensioned for insertion through the native valve, for example through tissue at or near a commissure of the native valve or through the valve opening itself. In some embodiments, the free end **22**o may comprise an atraumatic tip to avoid reduce risk of injury to the native valve tissue and leaflets. For example, the free end may comprise a blunt end, a ball tip, a curved tip (e.g. J-tip or pigtail), or other atraumatic shapes. Alternatively, the free end **22**o may be configured for piercing tissue. In various embodiments, the free end **22**o is separate from and extends outward from the main coils of the anchor **15**o. In various embodiments, the main body coils of the anchor **15**o circumscribe an area (in the case of a spiral coil) or a volume (in the case of a helical coil) having a diameter, and the free end **22**o extends to a radius greater than the diameter of the anchor **15**o. In various embodiments, the free end **22**o extends to a radius substantially greater than the diameter of the anchor **15**o. In various embodiments, the free end **22**o is configured to circumscribe a larger diameter than the anchor **15**o. In various embodiments, the free end **22**o is configured to circumscribe all of the chordae tendineae of the native valve to be treated. In various embodiments, the free end **22**o may be shaped and configured to reduce the risk of counterrotation. For example, the tip **22**o may have a curled end to cause the free end **22**o to snag chordae if it is rotated in a direction opposite the anchoring rotation.

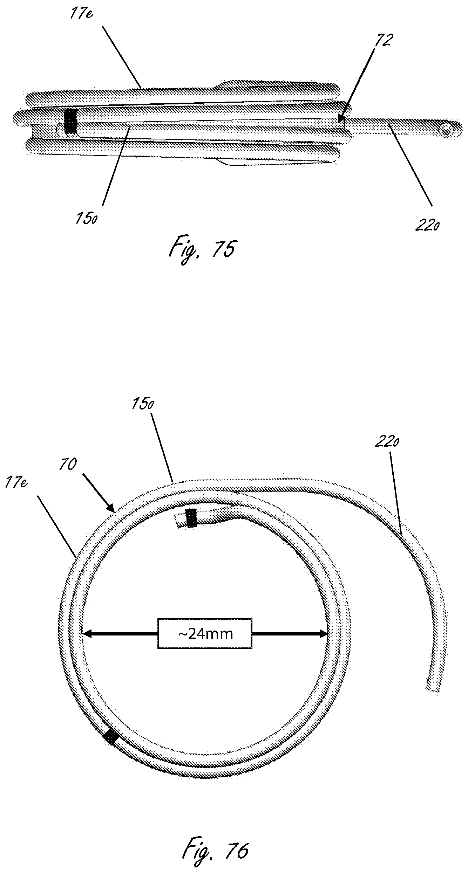

The free end **22**o of the tapered spiral band **17**e may optionally rotated around one or more structures on the second side of the native valve such that the one or more structures (e.g., chordae, leaflets, or annulus) are pulled radially inwards towards the longitudinal axis of the anchor **15**o and/or towards the longitudinal axis of the delivery device **30.** The spiral band **17**e and/or free end **22**o may be configured such that minimal torque is applied to the one or more structures. Alternatively, or in combination, the tapered spiral band **17**e and/or free end **22**o may be configured such that the one or more structures are not rotated, or are minimally rotated, during rotation of the spiral band **17**e. For example, the spiral band **17**e may comprise one or more spaces between loops of the spiral band (for example, spaces **18** shown in FIG. 57) which facilitate movement of the captured tissue (e.g. chordae and/or leaflets) from the free end **22**o to the center of the spiral structure with little or no torque and/or rotation of the structures during rotation of the tapered spiral band **17**e as described herein. Alternatively or in combination, the spiral band **17**e may be configured such that, when fully deployed (for example, as shown in FIG. 76), none of the structures reside between the loops of the spiral. Instead, the one or more structures may sit radially inward of the loops in order to facilitate capture of the one or more structures between the spiral band **17**e and the expanded frame structure as described herein. The one or more structures may retain or nearly retain their normal anatomical position when the spiral band **17e** is fully deployed.

The free end **22**o may be disposed radially outwards from the support structure **70.** Disposing the free end **22**o radially outward from the remainder of support structure **70** may, for example, aid in deployment of the anchor **15**o from the delivery device and/or capture of the one or more structures as described herein.

The free end **22**o may be angled proximally (e.g., towards a proximal portion of the anchor **15**o and a distal end of the delivery device **30**) or distally (e.g., away from a proximal portion of the anchor **15**o and towards a proximal portion of the delivery device **30**) from the support structure **70.** Angling the free end **22**o proximally or distally towards or away from the delivery device may, for example, aid in deployment of the anchor **15**o from the delivery device and/or capture of the one or more structures as described herein.

In some embodiments, the support structure **70** may comprise one or more channels or lumens 71 disposed therein. For example, one or more of the wires **70**a, **70**b may comprise one or more channels or lumens **71.** One or more of the wires 70a, 70b may comprise a hollow, tubular cross-section. The spiral band **17**e may, for example, comprise a hypotube. The lumen of the spiral band **17**e may be configured to pass another component (e.g. a wire, guidewire, etc.) therethrough. The channels or lumens **71** may for example be left as open lumens. Alternatively, or in combination, the channels or lumens **71** may be filled, for example with one or more stiffening members.

FIGS. 77-80 show several views of an optional locking mechanism for a flat spiral anchor. FIG. 77 shows a top view of a flat spiral anchor **15**p comprising a spiral band **17** having a plurality of interlocking features **24** disposed thereon in an intermediate deployed or undeployed configuration (which may be substantially similar to the configurations shown in FIGS. 58-59 and described herein). FIG. 78 shows a perspective view of the spiral band anchor **15**p in the undeployed configuration. FIG. 79 shows a top view of the spiral band anchor in a fully deployed configuration. FIG. 80 shows a perspective view of a valve prosthesis **10**p comprising the spiral band anchor **15**p in the deployed configuration disposed around a frame structure **12**p in an expanded configuration.

The valve prosthesis **10**p may be substantially similar to any of the valve prostheses described herein. The frame structure **12**p may have an unexpanded configuration (for example, a compressed configuration as described herein) and an expanded configuration as described herein. The frame structure **12**p may be substantially similar to any of the frame structures described herein or understood by one of ordinary skill in the art from the description herein. The anchor **15**p may comprise a spiral band **17.** The anchor **15**p may be configured to anchor the frame structure **12**p to the native valve when the frame structure **12**p is in the expanded configuration adjacent the native valve. The frame structure **12**p may be configured to be actuated from the unexpanded configuration to the expanded configuration adjacent a native valve in a patient. The spiral band anchor **15**p may be deployed and configured to anchor the frame structure **12**p of the valve prosthesis **10**p adjacent a native valve as described herein. The anchor **15**p may, for example, be configured to be fully advanced from a first side of a native valve in a patient (e.g. an atrial side) to a second side of the native valve (e.g. into a ventricle of the heart) and anchor the frame structure **12**p to the native valve when the frame structure **12**p is in an expanded configuration adjacent the native valve as described herein. The spiral band anchor **15**p may be delivered to the native valve by a delivery device as described herein.

The anchor **15**p may comprise a spiral band **17** having a free end **22**p. The spiral band **17** may be substantially similar to any of the spiral bands described herein. The free end **22**p may be substantially similar to the free end of other anchors described herein. The other end of the anchor **15**p (e.g., spiral band **17**) may be coupled to the top (proximal end), bottom (distal end), or an intermediate portion (e.g. middle) of the frame structure **12**p as described herein. Alternatively, or in combination, the other end of the anchor **15**p may not be attached to the frame structure **12**p as described herein. The free end **22**p of the spiral anchor **15**p may facilitate capturing of the valve leaflets and/or chordae tendineae within the sweep of the free end during rotation as described herein. During rotation of the spiral anchor **15**p, the leaflets and/or chordae tendineae may be captured by the free end **22**p and trapped between the valve frame structure **12**p and an interior surface of the spiral anchor **15**p. The anchor **15**p may comprise one or more spaces between loops of the anchor **15**p which facilitate movement of the captured tissue (e.g. chordae and/or leaflets) from the free end **22**p to the center of the spiral structure with little or no torque and/or rotation of the structures during rotation of the anchor **15**p as described herein. At least a portion of the spiral anchor **15**p may be formed of a material having sufficient rigidity to hold a predetermined shape as described herein.

In some embodiments, the anchor **15**p may comprise a delivery (e.g., elongated) configuration when disposed within a delivery device as described herein. The delivery device may be configured to deploy the anchor **15**p from the delivery configuration to an undeployed or first intermediate deployed configuration as described herein (as shown in FIGS. 77 and 78). The anchor **15**p may then be actuated into the deployed or second fully deployed configuration (as shown in FIGS. 79 and 80), for example by expanding the frame structure **12**p within the anchor **15**p or by rotating the anchor **15**p to capture the one or more structures as described herein. In some embodiments, the anchor **15**p may be configured to be actuated into more than one intermediate deployed configuration, for example, by partial expansion of the frame structure **12**p.

The anchor **15**p may comprise a plurality of loops of a spiral as described herein. The anchor **15**p may comprise a more compact spiral, with more loops, in the undeployed or first intermediate deployed configuration compared to the deployed or second fully deployed configuration. As the anchor **15**p expands the spiral loops may unwind. In some embodiments, the outer perimeter of the anchor **15**p may be substantially similar in the undeployed (or first intermediate deployed) and deployed (or second fully deployed) configurations. As the spiral loops unwind, the spiral may expand from the center, for example simultaneously with expansion of the frame structure in the center of anchor **15**p, such that the spiral loops are expanded against one another as shown in FIG. 79. As will be understood by one of ordinary skill in the art, the shape, size, and number of loops may determine the limits of expansion of the anchor **15**p and alteration of any or all of these parameters may be done to generate a final deployed configuration desired from an initial undeployed configuration.

The anchor **15**p may have a generally spiral shape in the deployed configuration. The anchor **15**p may be formed as a flat spiral (in the deployed configuration) whereby the loops generally are positioned within the same plane (the plane being perpendicular to a longitudinal axis). The plurality of loops of the anchor **15**p may be spiral radially outward from a central point or central axis of the spiral, for example along an axis which is coaxial with a longitudinal axis of a delivery device such that the anchor **15**p lies approximately along a plane perpendicular to the longitudinal axis of the delivery device as described herein. The plurality of loops may comprise at least 360 degrees of rotation when deployed such that the loops wrap around one another and provide additional mechanical leverage against the frame structure in order to facilitate anchoring of the frame structure as described herein. Additional loops or partial loops may provide additional mechanical strength and/or leverage.

The spiral band anchor **15**p may be substantially similar to any of the anchors described herein with the addition of interlocking features **24** disposed along the loops of the anchor **15**p (e.g., along the loops of spiral band **17)** such that they interlock when expanded against one another. The interlocking features **24** may, for example, comprise correspondingly-shaped waves, bends, humps, or the like disposed on adjacent loops of the spiral band anchor 15p such that the interlocking features **24** on adjacent loops may be nested with each other when the anchor **15**p is in the deployed configuration. The interlocking features can provide a locking mechanism in the general sense. In some embodiments, the interlocking features may act more like index features for the opening and/or closing of the spiral band (i.e., expansion and/or contraction of the diameter). In this manner, the interlocking features may index the opening and/or closing by discreet increments. In some embodiments, the interlocking features may positively control the opening and/or closing of the spiral band. One will appreciate from the description herein that the resistance provided by the interlocking features may be adjusted depending on the application. In some embodiments, the resistance may be relatively high to prevent or reduce the risk of the diameter of the spiral anchor or band changing diameter. One will appreciate that the interlocking features may be designed and configured in various other manner. Although shown as a somewhat repeating pattern in FIGS. 77-80, the interlocking features may have one or more shapes and sizes. The pattern may be repeating or non-repeating. The spiral anchor or band may include different sets of interlocking features. For example, it may be desirable to provide one or more sets of interlocking features to index self-assembly and/or anchoring of the spiral anchor or band to the chordae and another set(s) configured to prevent the spiral anchor or band from expanding beyond a certain predetermined diameter, such as to increase the opposing force against the expanding frame.

In some embodiments, the spiral anchor **15**p may comprise a more than one pair of interlockingly-shaped features **24** disposed on adjacent loops of the anchor **15**p (e.g., on adjacent loops of the spiral band **17**) in order to ensure secure locking and/or to provide for a plurality of intermediate expanded/deployed configurations between the fully undeployed and the fully deployed configuration. For example, the spiral band anchor **15**p may comprise a plurality of waves **24** in adjacent loops of the generally spiral-shaped anchor **15**p which can provide intermittent locking of the anchor **15**p as the anchor **15**p is deployed in multiple configurations. As the anchor **15**p is expanded, the waves **24** may move into and out of nesting with one another, locking when nested together and expanding when enough energy has been applied to disengage the nested waves from one another.

In some embodiments, the plurality of features **24** may be disposed along the generally spiral-shaped anchor **15**p at pre-determined distances. In some embodiments, the plurality of features **24** may be disposed along the generally spiral-shaped anchor **15**p at regular intervals. In some embodiments, the plurality of features **24** may be disposed along only a portion of the anchor **15**p. IN some embodiments, the plurality of features **24** may be disposed along the entire anchor **15**p.

In some embodiments, the plurality of interlocking features **24** may comprise two, four, six, eight, ten, twelve, fourteen, sixteen, eighteen, twenty, or more features **24** disposed on adjacent loops of the spiral band **17**. For example, each adjacent loop may comprise one, two, three, four, five, six, seven, eight, nine, ten, or more features **24** per loop.

In some embodiments, the plurality of features **24** may comprise a plurality of waves disposed along the generally spiral-shaped anchor **15**p such that the anchor resembles a flower-like shape (e.g., as shown in FIGS. 77-80).

It will be understood by one or ordinary skill in the art that any of the anchors described herein may comprise any of the locking mechanisms, or any combination of locking mechanisms, described herein or understood based on the teachings herein.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the present disclosure. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present disclosure.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

The foregoing descriptions of specific embodiments of the present disclosure have been presented for purposes of illustration and description. They are not intended to be exhaustive or to limit the invention to the precise forms disclosed, and obviously many modifications and variations are possible in light of the above teaching. The embodiments were chosen and described to best explain the principles of the invention and its practical application, to thereby enable others skilled in the art to best utilize the present disclosure and various embodiments with various modifications as are suited to the particular use contemplated. It is intended that the scope of the invention be defined by the Claims appended hereto.

While preferred embodiments of the present disclosure have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that structures within the scope of these claims be covered thereby

## Claims

1. A system for treating a diseased native valve in a patient, the system comprising:
a frame structure (12n, 12p) having an unexpanded configuration and an expanded configuration; and
an anchor (15n, 15p) comprising a free end (22n, 22p) and having a flat spiral shape, wherein the flat spiral shape comprises more than one loop positioned within a single plane that is perpendicular to a central longitudinal axis of the anchor,
wherein the frame structure is configured to be actuated from the unexpanded configuration to the expanded configuration adjacent a native valve in a patient, and
wherein the anchor is configured to anchor the frame structure to the native valve when the frame structure is in the expanded configuration adjacent the native valve.

2. The system of claim 1, wherein the anchor (15n, 15p) is configured to be fully advanced from an atrial side of the native valve into a ventricle of the heart.

3. The system of claim 2, wherein at least a portion of the anchor (15n, 15p) is configured to reside within a subvalvular plane of the heart when it anchors the frame structure to the native valve.

4. The system of claim 3, wherein the anchor (15n, 15p) is configured to reside entirely within the subvalvular plane of the heart when it anchors the frame structure to the native valve.

5. The system of claim 1, wherein the anchor (15n, 15p) comprises a delivery configuration and a deployed configuration.

6. The system of claim 5, wherein the anchor (15n, 15p) comprises the flat spiral shape in the deployed configuration.

7. The system of claim 5, wherein the anchor (15n, 15p) comprises one or more locking mechanisms (19) configured to maintain the anchor in the deployed configuration.

8. The system of claim 7, wherein the one or more locking mechanisms comprise a frictional band (21), a polymer coating, or one or more key and one or more key hole features (19).

9. The system of claim 1, wherein the more than one loop of the flat spiral shape comprises about 540 degrees of rotation.

10. The system of claim 1, wherein the flat spiral comprises a plurality of loops that spiral radially outward from a central point.

11. The system of claim 1, wherein the anchor (15n) comprises at least one channel or lumen (71) disposed therein.

## Patentansprüche

1. System zum Behandeln einer erkrankten natürlichen Herzklappe bei einem Patienten, wobei das System umfasst:
eine Rahmenstruktur (12n, 12p) mit einer nicht expandierten Konfiguration und einer expandierten Konfiguration; und
einen Anker (15n, 15p), der ein freies Ende (22n, 22p) umfasst und eine flache Spiralform aufweist, wobei die flache Spiralform mehr als eine innerhalb einer einzelnen Ebene, die senkrecht zu einer zentralen Längsachse des Ankers ist, positionierte Schleife umfasst,
wobei die Rahmenstruktur dazu konfiguriert ist, von der nicht expandierten Konfiguration in die expandierte Konfiguration neben einer natürlichen Herzklappe in einem Patienten betätigt zu werden, und
wobei der Anker dazu konfiguriert ist, die Rahmenstruktur an der natürlichen Herzklappe zu verankern, wenn sich die Rahmenstruktur in der expandierten Konfiguration neben der natürlichen Herzklappe befindet.

2. System nach Anspruch 1, wobei der Anker (15n, 15p) dazu konfiguriert ist, vollständig von einer atrialen Seite der natürlichen Herzklappe in einen Ventrikel des Herzens vorgeschoben zu werden.

3. System nach Anspruch 2, wobei wenigstens ein Abschnitt des Ankers (15n, 15p) dazu konfiguriert ist, sich innerhalb einer subvalvulären Ebene des Herzens zu befinden, wenn er die Rahmenstruktur an der natürlichen Klappe verankert.

4. System nach Anspruch 3, wobei der Anker (15n, 15p) dazu konfiguriert ist, sich vollständig innerhalb der subvalvulären Ebene des Herzens zu befinden, wenn er die Rahmenstruktur an der natürlichen Klappe verankert.

5. System nach Anspruch 1, wobei der Anker (15n, 15p) eine Abgabekonfiguration und eine entfaltete Konfiguration umfasst.

6. System nach Anspruch 5, wobei der Anker (15n, 15p) in der entfalteten Konfiguration eine flache Spiralform aufweist.

7. System nach Anspruch 5, wobei der Anker (15n, 15p) einen oder mehrere Verriegelungsmechanismen (19) umfasst, die dazu konfiguriert sind, den Anker in der entfalteten Konfiguration zu halten.

8. System nach Anspruch 7, wobei der eine oder die mehreren Verriegelungsmechanismen ein Reibungsband (21), eine Polymerbeschichtung oder einen oder mehrere Schlüssel und ein oder mehrere Schlüssellochmerkmale (19) umfassen.

9. System nach Anspruch 1, wobei die mehr als eine Schleife der flachen Spiralform etwa 540 Grad Drehung umfasst.

10. System nach Anspruch 1, wobei die flache Spirale eine Mehrzahl von Schleifen umfasst, die sich radial von einem Mittelpunkt nach außen spiralförmig erstrecken.

11. System nach Anspruch 1, wobei der Anker (15n) wenigstens einen Kanal oder ein Lumen (71) umfasst, der/das darin angeordnet ist.

## Revendications

1. Système de traitement d'une valve native malade chez un patient, le système comprenant :
une structure de cadre (12n, 12p) présentant une configuration non dilatée et une configuration dilatée ; et
un ancrage (15n, 15p) comprenant une extrémité libre (22n, 22p) et présentant une forme en spirale plate, dans lequel la forme de spirale plate comprend plus d'une boucle positionnée dans un seul plan qui est perpendiculaire à un axe longitudinal central de l'ancrage,
dans lequel la structure de cadre est configurée pour passer de la configuration non dilatée à la configuration dilatée à proximité d'une valve native d'un patient, et
dans lequel l'ancrage est configuré pour ancrer la structure de cadre à la valve native lorsque la structure de cadre est dans la configuration dilatée à proximité de la valve native.

2. Système selon la revendication 1, dans lequel l'ancrage (15n, 15p) est configuré pour être entièrement avancé à partir d'un côté auriculaire de la valve native jusque dans un ventricule du cœur.

3. Système selon la revendication 2, dans lequel au moins une portion de l'ancrage (15n, 15p) est configurée pour se loger dans un plan sous-valvulaire du cœur lorsqu'elle ancre la structure de cadre à la valve native.

4. Système selon la revendication 3, dans lequel l'ancrage (15n, 15p) est configuré pour se loger entièrement dans le plan sous-valvulaire du cœur lorsqu'il ancre la structure de cadre à la valve native.

5. Système selon la revendication 1, dans lequel l'ancrage (15n, 15p) comprend une configuration de mise en place et une configuration déployée.

6. Système selon la revendication 5, dans lequel l'ancrage (15n, 15p) comprend la forme en spirale plate dans la configuration déployée.

7. Système selon la revendication 5, dans lequel l'ancrage (15n, 15p) comprend un ou plusieurs mécanismes de verrouillage (19) configurés pour maintenir l'ancrage dans la configuration déployée.

8. Système selon la revendication 7, dans lequel le ou les mécanismes de verrouillage comprennent une bande de frottement (21), un revêtement en polymère, ou des éléments à une ou plusieurs clavettes et un ou plusieurs trous de clavette (19).

9. Système selon la revendication 1, dans lequel les plus d'une boucle de la forme en spirale plate comprennent environ 540 degrés de rotation.

10. Système selon la revendication 1, dans lequel la spirale plate comprend une pluralité de boucles qui s'enroulent en spirale radialement vers l'extérieur à partir d'un point central.

11. Système selon la revendication 1, dans lequel l'ancrage (15n) comprend au moins un canal ou au moins une lumière (71) disposé(e) à l'intérieur de celui-ci.
